# EUROPEAN PATENT SPECIFICATION

(11) **EP 2 344 611 B1**
(45) Date of publication and mention of the grant of the patent: **26.02.2014**
(21) Application number: 09820757.4
(22) Date of filing: 14.10.2009
(51) Int. Cl.: C09K 19/04, C09K 19/32, C09K 19/34

(54) **BENZIMIDAZOLE COMPOUNDS AND ORGANIC PHOTOELECTRIC DEVICE WITH THE SAME**
BENZIMIDAZOLVERBINDUNGEN UND ORGANISCHES PHOTOELEKTRISCHES BAUELEMENT DAMIT
COMPOSÉS DE BENZIMIDAZOLE ET DISPOSITIF PHOTOÉLECTRIQUE ORGANIQUE LES COMPRENANT

(30) Priority: 14.10.2008 KR 20080100726
(43) Date of publication of application: 20.07.2011
(73) Proprietor: CHEIL INDUSTRIES INC., Kumi-city Kyungsangbuk-do 730-030 (KR)
(72) Inventor: JUNG, Sung-Hyun, Uiwang-si Gyeonggi-do 437-711 (KR); KIM, Hyung-Sun, Uiwang-si Gyeonggi-do 437-711 (KR); KIM, Young-Hoon, Uiwang-si Gyeonggi-do 437-711 (KR); LEE, Ho-Jae, Uiwang-si Gyeonggi-do 437-711 (KR); LEE, Seung-Gyoung, Uiwang-si Gyeonggi-do 437-711 (KR); YU, Eun-Sun, Uiwang-si Gyeonggi-do 437-711 (KR); CHAE, Mi-Young, Uiwang-si Gyeonggi-do 437-711 (KR)
(74) Representative: Michalski Hüttermann & Partner Patentanwälte
(86) International application number: PCT/KR2009/005911
(87) International publication number: WO 2010/044607

(56) References cited:
- EP-A2- 2 100 941
- WO-A1-02/088274
- WO-A1-2005/023250
- WO-A1-2005/076669
- WO-A1-2006/080640
- WO-A1-2007/046658
- WO-A1-2008/069586
- JP-A- 2007 277 306
- US-A1- 2007 131 929
- US-A1- 2007 205 412
- US-A1- 2007 205 412
- US-A1- 2007 267 970
- US-A1- 2007 267 970
- US-A1- 2008 079 356
- US-A1- 2008 100 207
- US-A1- 2008 233 387
- US-A1- 2008 233 387
- US-A1- 2008 284 322
- KATRITZKY A R ET AL: "A 1,4-PHOTOCHEMICAL ARYL SHIFT", TETRAHEDRON LETTERS, ELSEVIER, AMSTERDAM, NL, vol. 23, no. 12, 1 January 1982 (1982-01-01), XP001062670, ISSN: 0040-4039, DOI: 10.1016/S0040-4039(00)87071-9
- DATABASE CAPLUS [Online] CHEMICAL ABSTRACTS SERVICE, COLUMBUS, OHIO, US; 8 April 2004 (2004-04-08), DUBEY P K KUMAR C R ET AL: "Syntheses of 1-alkyl-2-(substituted-2- pyridyl)benzimidazoles", XP008147249, retrieved from CAPLUS Database accession no. 2003-797541
- MEI-YI LAI ET AL: "Benzimidazole/Amine-Based Compounds Capable of Ambipolar Transport for Application in Single-Layer Blue-Emitting OLEDs and as Hosts for Phosphorescent Emitters", ANGEWANDTE CHEMIE INTERNATIONAL EDITION, vol. 47, no. 3, 4 January 2008 (2008-01-04), pages 581-585, XP55020057, ISSN: 1433-7851, DOI: 10.1002/anie.200704113
- CHEMICAL ABSTRACTS, 18 February 2010, Columbus, Ohio, US; abstract no. 147:477288, XP008147255 & JP 2007 277306 A 06 September 2007
- CHEMICAL ABSTRACTS, 18 February 2010, Columbus, Ohio, US; abstract no. 143:256780, XP008147256 & US 2005 186445 A1 25 August 2005
- CHEMICAL ABSTRACTS, 18 February 2010, Columbus, Ohio, US; abstract no. 142:363445, XP008147258 & JP 2005 089543 A (TOYO INK MFG. CO., LTD) 07 April 2005
- CHEMICAL ABSTRACTS, 18 February 2010, Columbus, Ohio, US; abstract no. 142:64983, XP008147251 & JP 2004 352655 A (LDEMITSU KOSAN CO., LTD.) 16 December 2004
- CHEMICAL ABSTRACTS, 18 February 2010, Columbus, Ohio, US; abstract no. 97:109833, KATRITZKY, ALAN R. ET AL.: 'A 1,4-photochemical aryl shift.' XP008147257 & TETRAHEDRON LETTERS vol. 23, no. 12, 1982, pages 1241 - 2
- CHEMICAL ABSTRACTS, 18 February 2010, Columbus, Ohio, US; abstract no. 126:232625, PETOUD, STEPHANE, ET AL.: 'Luminescent Properties of Lanthanide Nitrato Complexes with Substituted Bis(benzimidazolyl)pyridines' & INORGANIC CHEMISTRY vol. 36, no. 7, 1997, pages 1345 - 1353
- CHEMICAL ABSTRACTS, 18 February 2010, Columbus, Ohio, US; abstract no. 140:245412, STIBRANY, ROBERT T. ET AL.: 'A Geometrically Constraining Bis(benzimidazole) Ligand and Its Nearly Tetrahedral Complexes with Fe(II) and Mn(II).' XP008147247 & INORGANIC CHEMISTRY vol. 43, no. 4, 2004, pages 1472 - 1480
- CHEMICAL ABSTRACTS, 18 February 2010, Columbus, Ohio, US; abstract no. 147:406819, XP008147244 & WO 2007 111262 A1 (LDEMITSU KOSAN CO., LTD.) 04 October 2007
- CHEMICAL ABSTRACTS, 18 February 2010, Columbus, Ohio, US; abstract no. 140:253493, DUBEY, P. K. ET AL.: 'Syntheses of 1-alkyl-2-(substituted-2- pyridyl)benzimidazoles.' XP008147249 & INDIAN JOURNAL OF CHEMISTRY, SECTION B: ORGANIC CHEMISTRY INCLUDING MEDICINAL CHEMISTRY vol. 42B, no. 9, 2003, pages 2115 - 2118
- CHEMICAL ABSTRACTS, 18 February 2010, Columbus, Ohio, US; abstract no. 141:140588, HUANG, WEI-SHENG, ET AL.: 'Highly Phosphorescent Bis-Cyclometalated Iridium Complexes Containing Benzoimidazole-Based Ligands.' XP008147245 & CHEMISTRY OF MATERIALS vol. 16, no. 12, 2004, pages 2480 - 2488
- CHEMICAL ABSTRACTS, 18 February 2010, Columbus, Ohio, US; abstract no. 149:67936, XP008147243 & WO 2008 069586 A1 (LG CHEM, LTD.) 12 June 2008
- CHEMICAL ABSTRACTS, 18 February 2010, Columbus, Ohio, US; abstract no. 148:526319, XP008147242 & US 2008 100207 A1 (PARK, SANG-HOON ET AL.) 01 May 2008
- CHEMICAL ABSTRACTS, 18 February 2010, Columbus, Ohio, US; abstract no. 148:437023, XP008147241 & US 2008 079356 A1 (PARK, SANG-HOON ET AL.) 03 April 2008
- CHEMICAL ABSTRACTS, 18 February 2010, Columbus, Ohio, US; abstract no. 148:341332, LAI, MEI-YI, ET AL.: 'Benzimidazole/amine-based compounds capable of ambipolar transport for application in single-layer blue-emitting OLEDs and as hosts for phosphorescent emitters' XP008147236 & ANGEWANDTE CHEMIE vol. 47, no. 3, 2008, pages 581 - 585
- CHEMICAL ABSTRACTS, 08 February 2010, Columbus, Ohio, US; abstract no. 147:82368, XP008147235 & US 2007 131929 A1 (BAE, JAE-SOON: ET AL.) 14 June 2007
- CHEMICAL ABSTRACTS, 18 February 2010, Columbus, Ohio, US; abstract no. 146:461976, XP008147237 & WO 2007 046658 A1 (LG CHEM, LTD.) 26 April 2007
- CHEMICAL ABSTRACTS, 18 February 2010, Columbus, Ohio, US; abstract no. 146:229351, XP008147261 & WO 2007 018007 A1 (IDEMITSU KOSAN:CO.) 15 February 2007
- CHEMICAL ABSTRACTS, 18 February 2010, Columbus, Ohio, US; abstract no. 145:198545, XP008147262 & WO 2006 080640 A1 (LG CHEM, LTD.) 03 August 2006
- CHEMICAL ABSTRACTS, Columbus, Ohio, US; abstract no. 137:360139, XP008147268 & WO 02 088274 A1 (LG CHEM, LTD.) 07 November 2002
- CHEMICAL ABSTRACTS, 18 February 2010, Columbus, Ohio, US; abstract no. 151:19855, XP008147264 & US 2009 134783 A1 (LIN, JIANN TSUEN ET AL.) 28 May 2009
- CHEMICAL ABSTRACTS, 18 February 2010, Columbus, Ohio, US; abstract no. 151:159848, XP008147270 & JP 2009 158848 A (IDEMITSU KOSAN CO LTD) 16 July 2009
- CHEMICAL ABSTRACTS, 18 February 2010, Columbus, Ohio, US; abstract no. 151:208154, XP008147266 & KR 2009 0 073 850 A (CHEIL INDUSTRIES, INC.) 03 July 2009
- CHEMICAL ABSTRACTS, 18 February 2010, Columbus, Ohio, US; abstract no. 151:324987, KE, TUNG-HUEI, ET AL.: 'Single molecule color controllable light emitting organic field effect transistors for white light emission with high color stability' XP008147267 & APPLIED PHYSICS LETTERS vol. 95, no. 6, 2009, pages 063303/1 - 063303/3
- CHEMICAL ABSTRACTS, 18 February 2010, Columbus, Ohio, US; abstract no. 151:300930, XP008147265 & KR 10 0 910 150 B1 (GRACEL CO., LTD.) 03 August 2009
- CHEMICAL ABSTRACTS, 18 February 2010, Columbus, Ohio, US; abstract no. 150:551296, KE, TUNG-HUEI, ET AL.: 'High efficiency blue light emitting unipolar transistor incorporating multifunctional electrodes' XP008147219 & APPLIED PHYSICS LETTERS vol. 94, no. 15, 2009, pages 153307/1 - 153307/3
- CHEMICAL ABSTRACTS, 18 February 2010, Columbus, Ohio, US; abstract no. 149:521041, XP008147269 & WO 2008 133483 A2 (LG CHEM, LTD.) 06 November 2008
- CHEMICAL ABSTRACTS, 18 February 2010, Columbus, Ohio, US; abstract no. 151:135263, XP008147271 & WO 2009 084544 A1 (IDEMITSU KOSAN CO., LTD.) 09 July 2009

## Description

### [Technical Field]

The present invention relates to benzimidazole compounds and an organic photoelectric device including the same. More particularly, the present invention relates to novel benzimidazole compounds that have high solubility in an organic solvent, and that are applicable as a host material of an emission layer, an electron transporting material, or a hole blocking material of an organic photoelectric device since they emit fluorescence and phosphorescence at a red wavelength through a blue wavelength, and an organic photoelectric device including the same.

### [Background Art]

An organic photoelectric device has been highlighted as the next generation display device. The organic photoelectric device can be driven at a low voltage, and can solve various problems of a thin film transistor-liquid crystal display (TFT-LCD), such as is difficult to make it thinner and have a wide viewing angle and rapid response speed. The organic photoelectric device of a middle size or less also has equivalent or better image quality to a TFT-LCD compared to other displays, and its manufacturing process is very simple. Therefore, it is considered that it will be advantageous in terms of cost in the future.

An organic photoelectric device includes an organic light emitting material between a rear plate including ITO transparent electrode patterns as an anode on a transparent glass substrate and an upper plate including a metal electrode as a cathode on a substrate. When a predetermined voltage is applied between the transparent electrode and the metal electrode, current flows through the organic light emitting material to emit light.

Such an organic light emitting material for an organic photoelectric device was firstly developed by Eastman Kodak, Inc., in 1987. The material is a low molecular aromatic diamine and aluminum complex as an emission-layer-forming material (Applied Physics Letters. 51, 913, 1987). C. W. Tang et al. firstly disclosed a practicable device as an organic photoelectric device in 1987 (Applied Physics Letters, 51 12, 913-915, 1987).

According to the reference, the organic layer has a structure in which a thin film (hole transport layer (HTL)) of a diamine derivative and a thin film of tris(8-hydroxy-quinolate)aluminum (Alq₃) are laminated. The Alq₃ thin film functions as an electron transporting emission layer.

Generally, an organic photoelectric device is composed of an anode of a transparent electrode, an organic thin layer of a light emitting region, and a metal electrode (cathode) formed on a glass substrate, in that order. The organic thin layer may include an emission layer, a hole injection layer (HIL), a hole transport layer (HTL), an electron transport layer (ETL), or an electron injection layer (EIL). It may further include an electron inhibition layer or a hole inhibition layer due to the emission characteristics of the emission layer.

When the organic light emitting diode is applied with an electric field, the holes and electrons are injected from the anode and the cathode, respectively. The injected holes and electrons are recombined on the emission layer though the hole transport layer (HTL) and the electron transport layer (ETL) to provide light emitting excitons. The provided light emitting excitons emit light by transiting to the ground state.

The light emission may be classified as a fluorescent material including singlet excitons and a phosphorescent material including triplet excitons according to the light emitting mechanism.

Recently, it is has become known that a phosphorescent light emitting material can be used for a light emitting material of an organic photoelectric device in addition to the fluorescent light emitting material (D. F. O'Brien et al., Applied Physics Letters, 74 3, 442-444, 1999; M. A. Baldo et al., Applied Physics letters, 75 1, 4-6, 1999). Such a phosphorescent material emits light by transiting the electrons from a ground state to an excited state, non-radiance transiting of a singlet exciton to a triplet exciton through intersystem crossing, and transiting a triplet exciton to a ground state to emit light.

When the triplet exciton is transited, it cannot directly transit to the ground state. Therefore, the electron spin is flipped, and then it is transited to the ground state so that it provides a characteristic of extending the lifetime (emission duration) to more than that of fluorescent emission.

In other words, the duration of fluorescent emission is extremely short at several nanoseconds, but the duration of phosphorescent emission is relatively long such as at several microseconds.

In addition, evaluating quantum mechanically, when holes injected from the anode are recombined with electrons injected from the cathode to provide light emitting excitons, the singlet and the triplet are produced in a ratio of 1:3, in which the triplet light emitting excitons are produced at three times the amount of the singlet light emitting excitons in the organic photoelectric device.

Accordingly, the percentage of the singlet exited state is 25% (the triplet is 75%) in the case of a fluorescent material, so it has limits in luminous efficiency. On the other hand, in the case of a phosphorescent material, it can utilize 75% of the triplet exited state and 25% of the singlet exited state, so theoretically the internal quantum efficiency can reach 100%. When a phosphorescent light emitting material is used, it has advantages in an increase in luminous efficiency of around four times that of the fluorescent light emitting material.

In the above-mentioned organic photoelectric device, a light emitting colorant (dopant) may be added to an emission layer (host) in order to increase the efficiency and stability in the emission state.

In this structure, the efficiency and properties of the light emission diodes are dependent on the host material in the emission layer. According to studies regarding the emission layer (host), the organic host material can be exemplified by a material including naphthalene, anthracene, phenanthrene, tetracene, pyrene, benzopyrene, chrysene, pycene, carbazole, fluorene, biphenyl, terphenyl, triphenylene oxide, dihalobi phenyl, trans-stilbene, and 1,4-diphenylbutadiene.

Generally, the host material includes 4,4-N,N-dicarbazole biphenyl (CBP) having a glass transition temperature of 110°C or less and excessively high symmetry. Thereby, it tends to crystallize and cause problems such as a short circuit and a pixel defect according to results of thermal resistance tests of the devices.
One solution is for instance disclosed by JP2007277306 A, wherein a material having electron and positive hole transporting properties for organic luminescent elements is provided. The material is obtained by copolymerizing conjugated carbazole with imidazole structure-containing units and is able to form thin membranes. The material can suitably be used as a light emission material for the organic electroluminescent elements, and the organic electroluminescent element excellent in light emission characteristics and durability.
In another approach described by US2008/0079356 A1 a cyclopentaphenanthrene-based compound is described, which is said to exhibit an excellent solubility, color purity, and color stability and can be useful as a material for forming an organic layer, in particular, an emitting layer, in an organoelectroluminescent device, and as an organic dye or an electronic material such as a nonlinear optical material.

In addition, most host materials including CBP are materials in which the hole transporting property is greater than the electron transporting property. In other words, as the injected hole transportation is faster than the injected electron transportation, the excitons are ineffectively formed in the emission layer. Therefore, the resultant device has deteriorated luminous efficiency.

Accordingly, host materials, or charge transporting materials such as electron transporting materials or hole blocking materials that have high thermal stability and triplet T1 energy, have been required to be developed.

### [DISCLOSURE]

### [Technical Problem]

One embodiment of the present invention provides a novel benzimidazole compound having high charge transporting properties, good film stability, and high triplet T1 energy and thus is applicable to host materials, or charge transporting materials such as electron transporting materials or hole blocking materials.

### [Technical Solution]

Another embodiment of the present invention provides an organic photoelectric device including the benzimidazole compound.

According to one embodiment of the present invention, a benzimidazole compound represented by the following Chemical Formula 35 to 131 is provided.

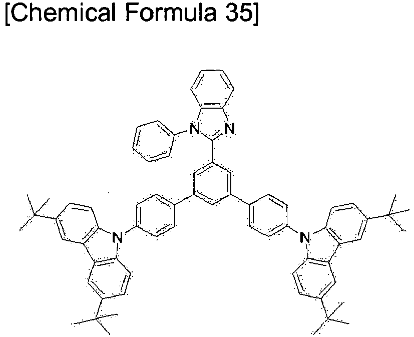

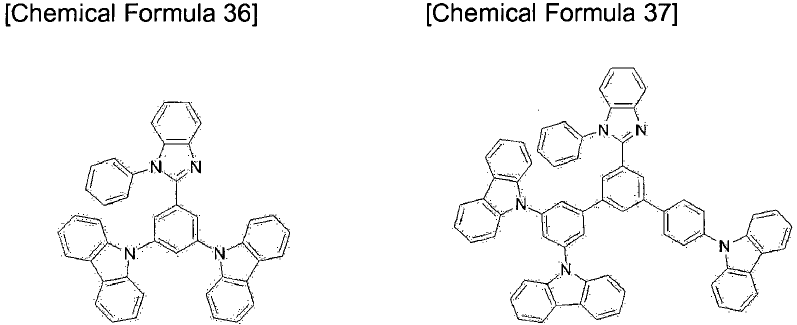

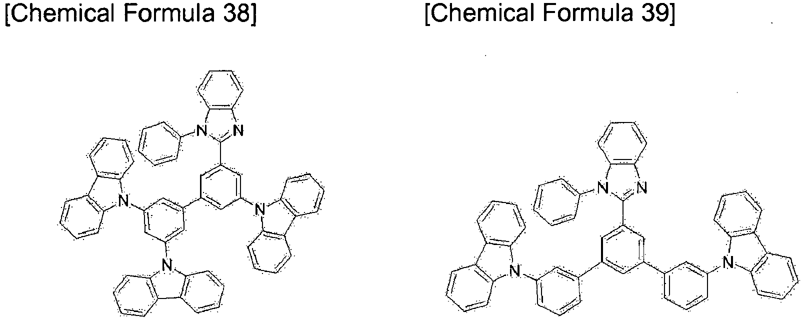

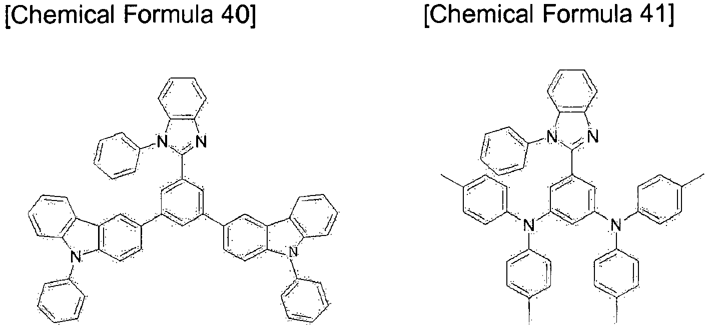

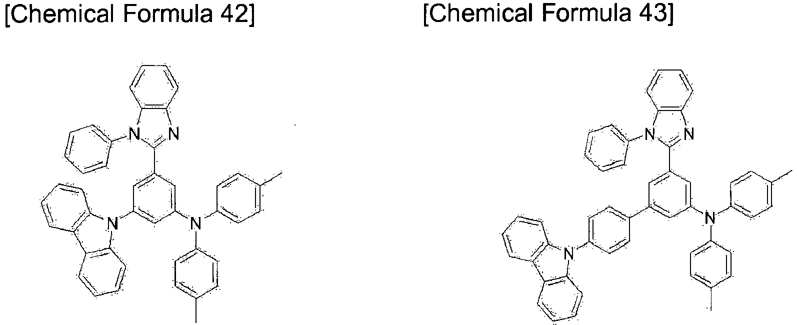

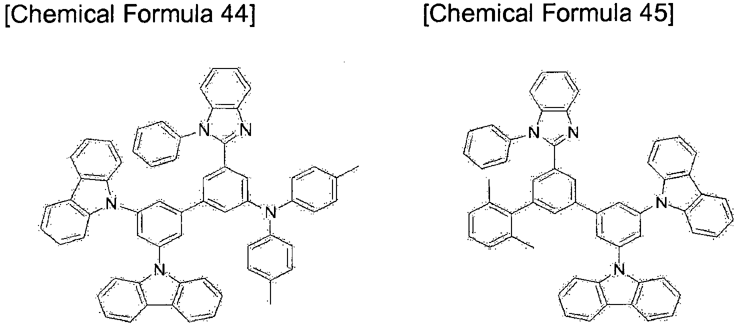

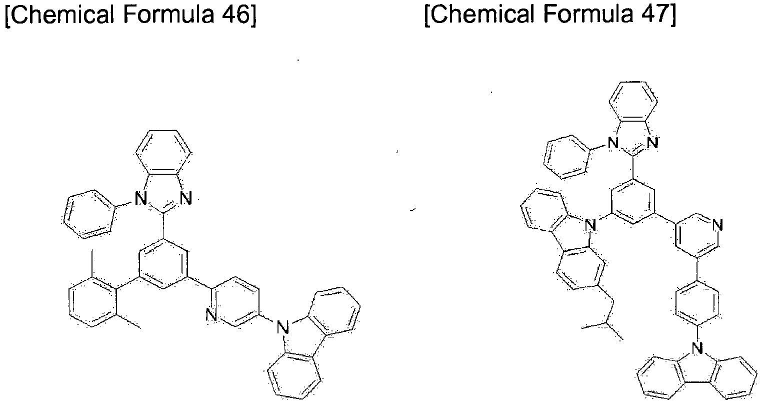

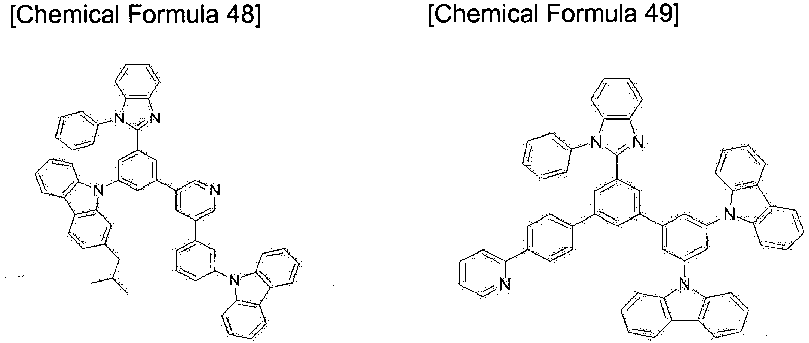

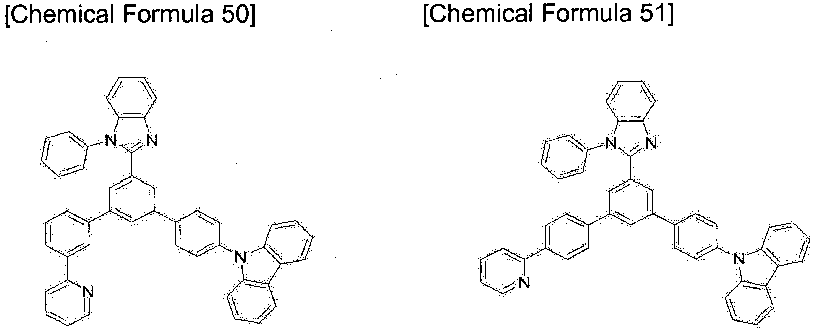

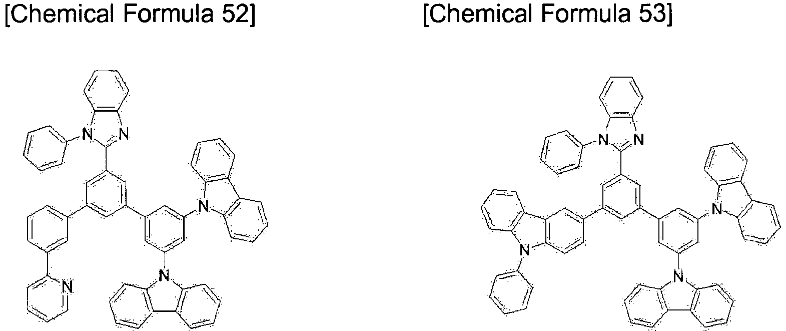

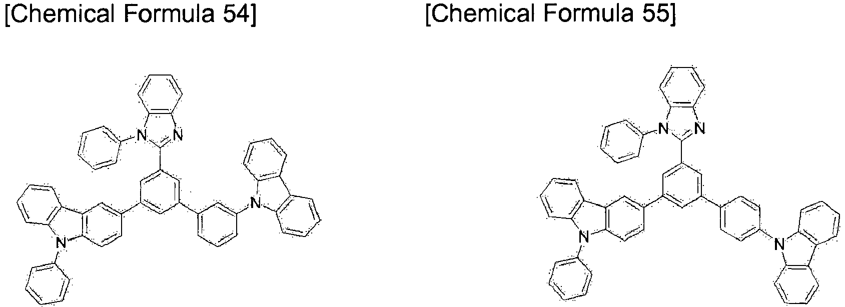

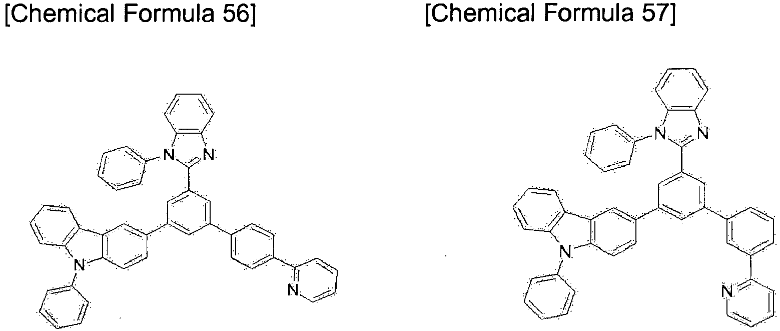

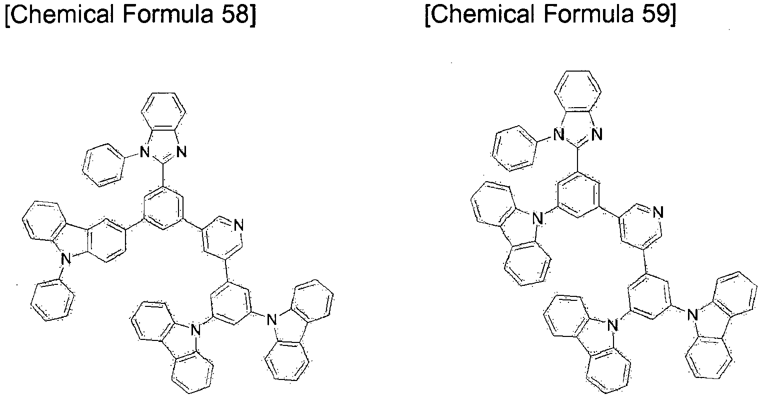

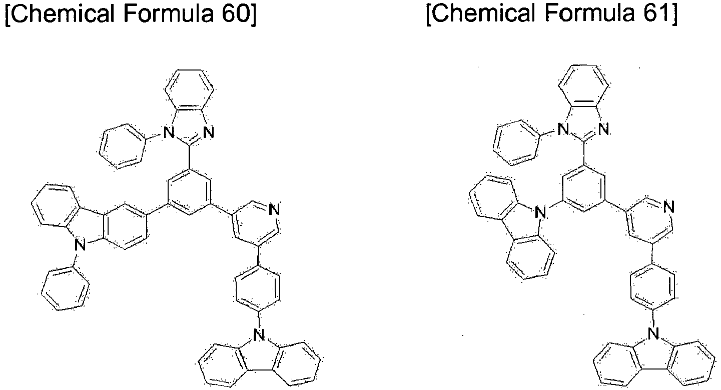

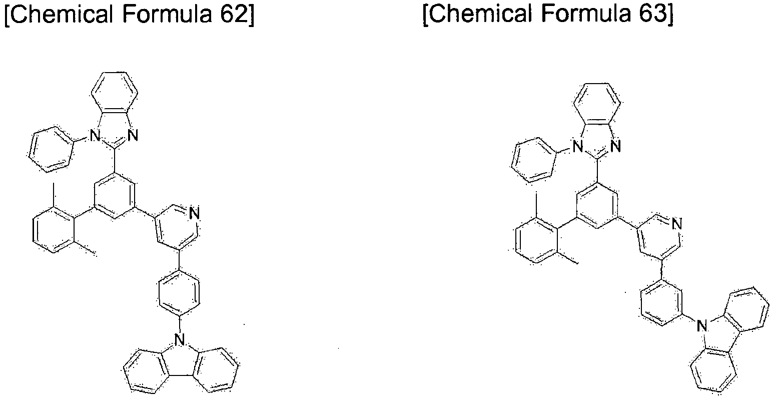

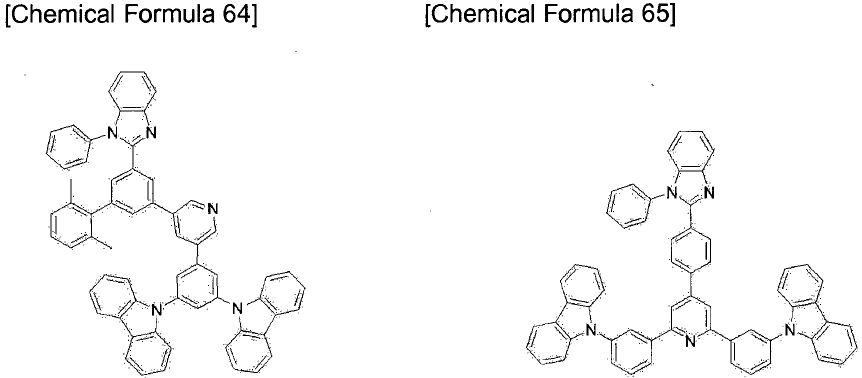

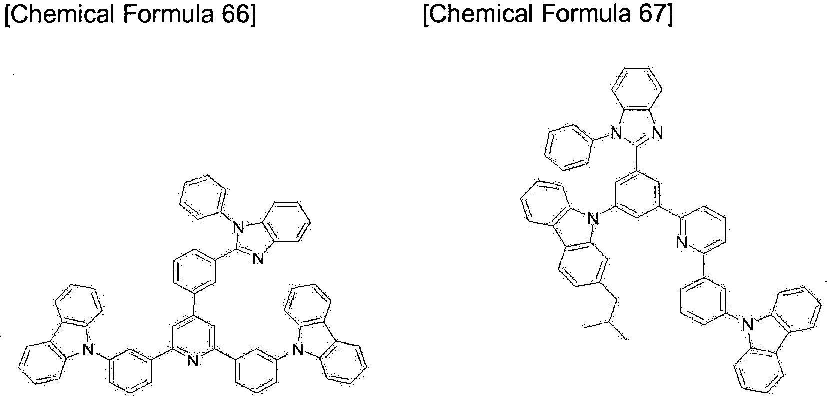

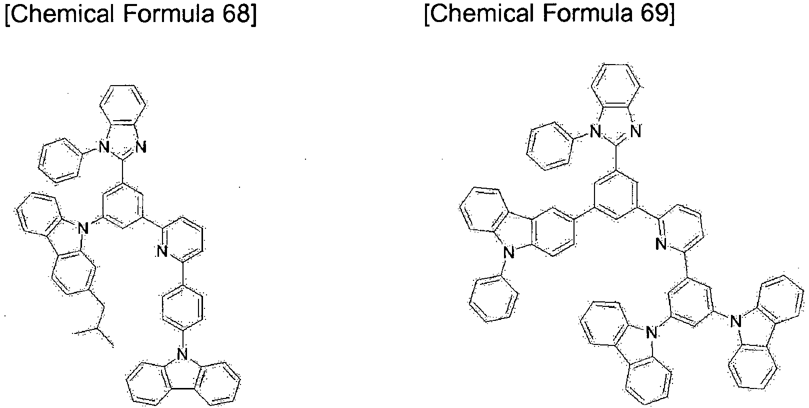

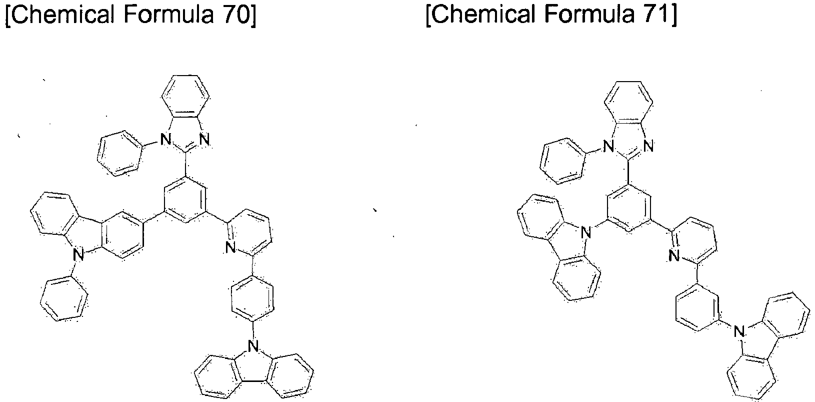

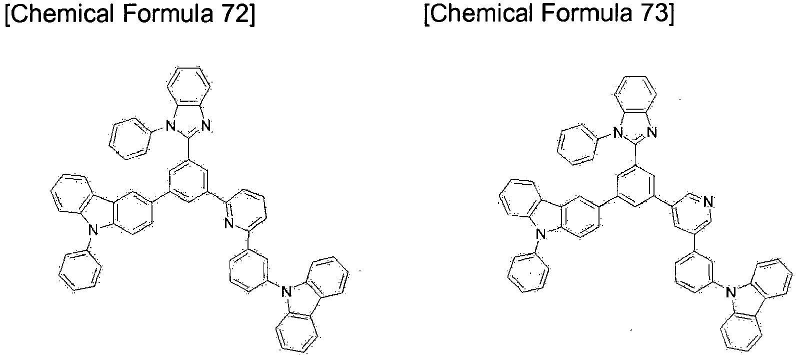

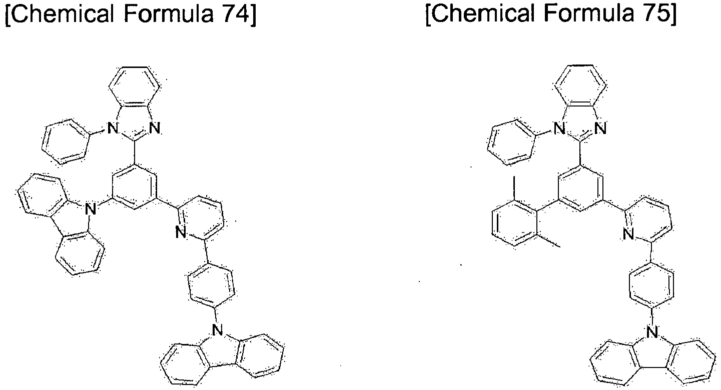

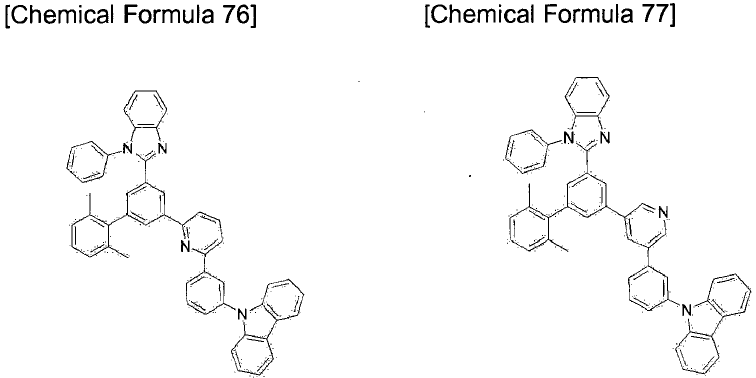

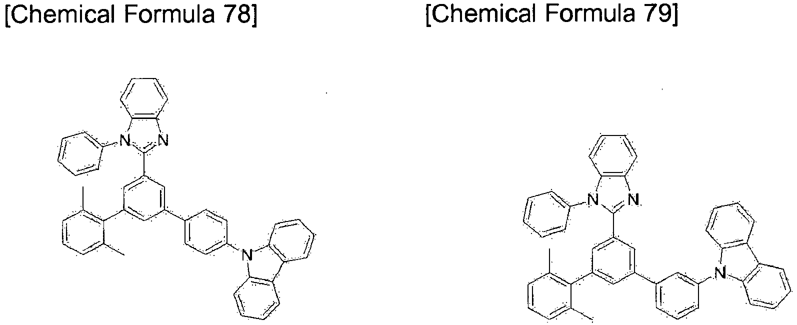

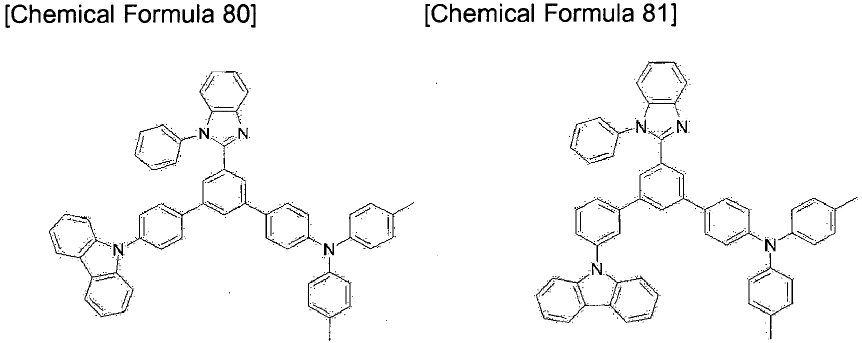

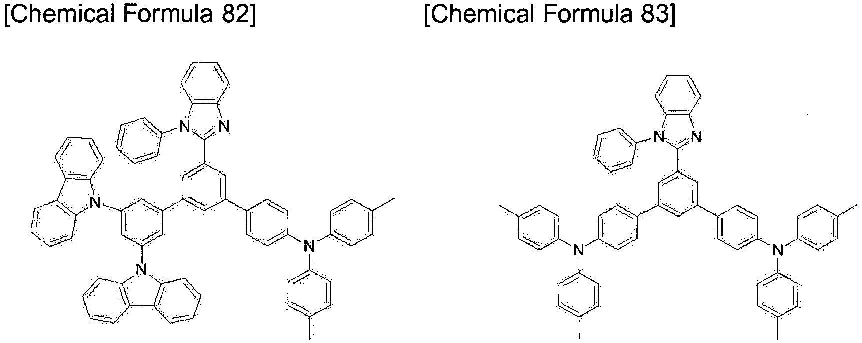

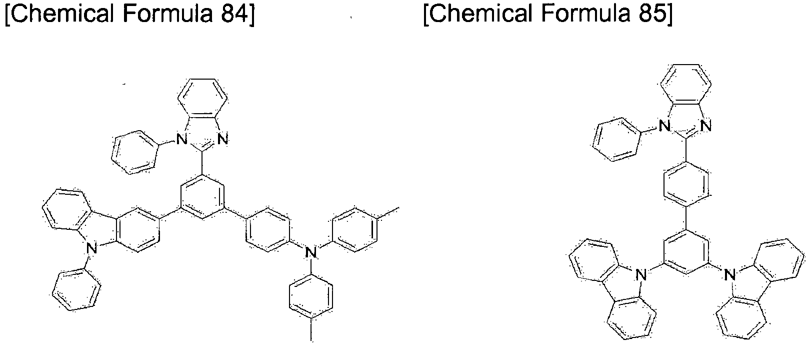

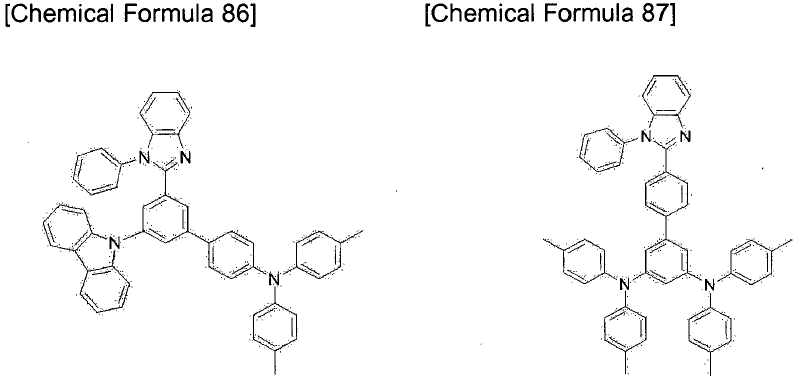

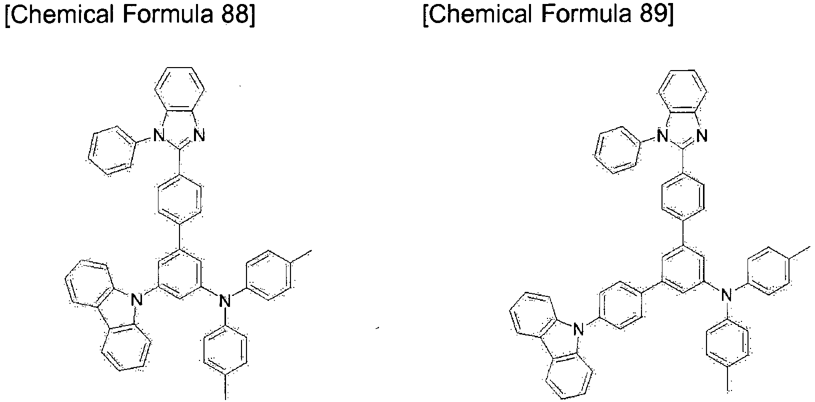

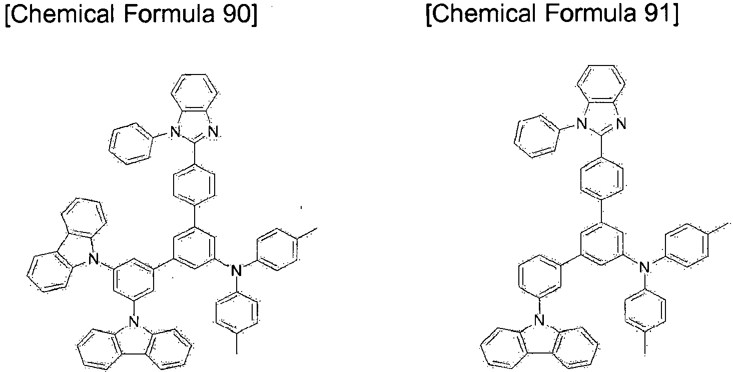

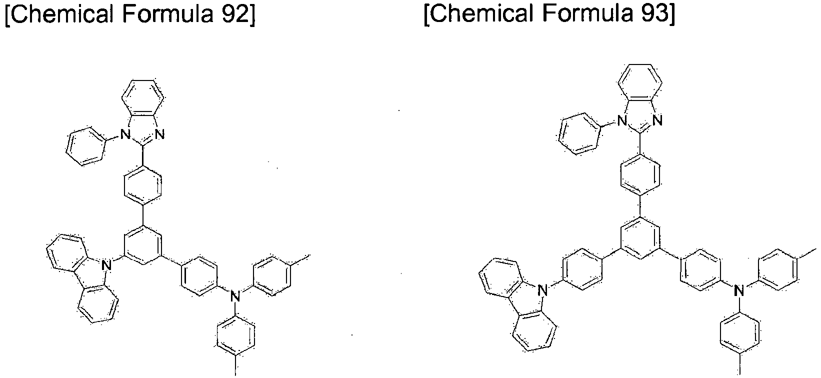

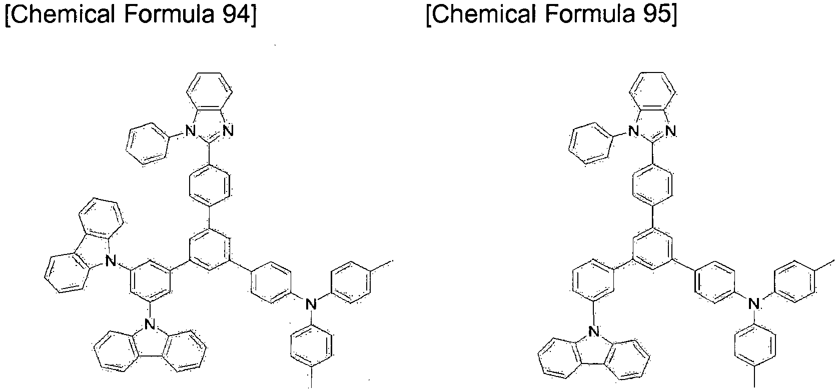

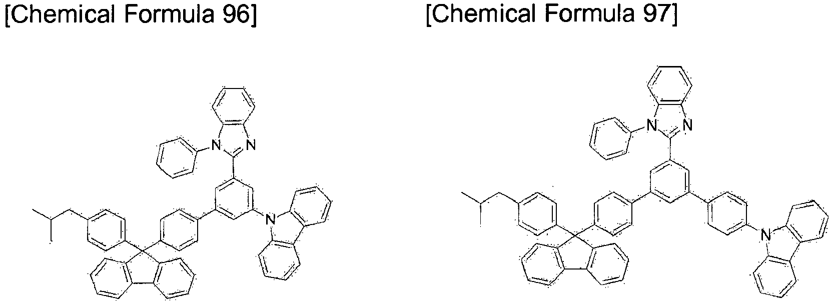

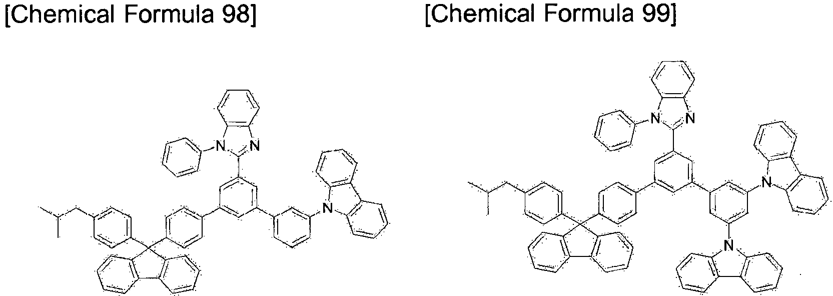

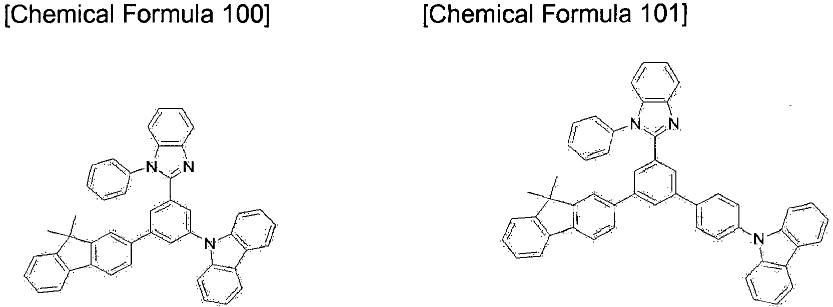

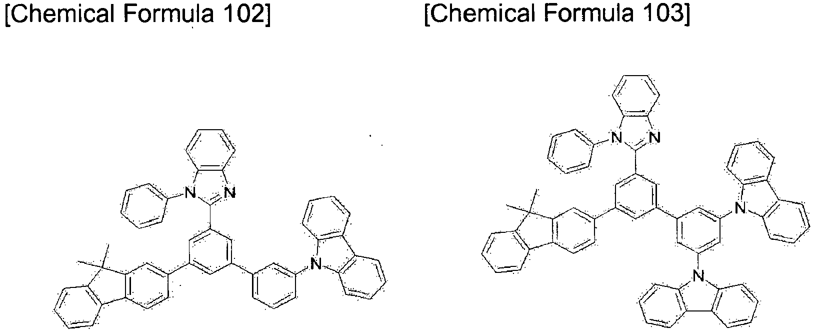

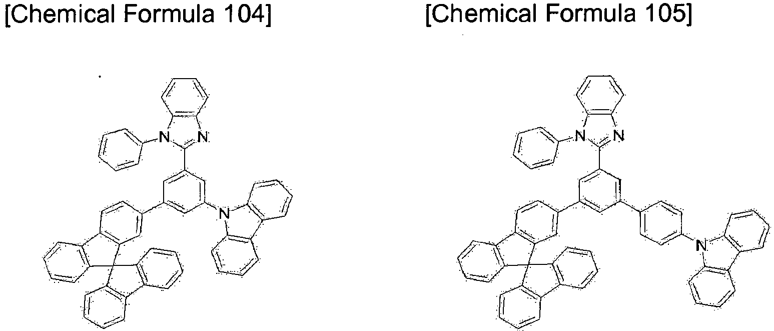

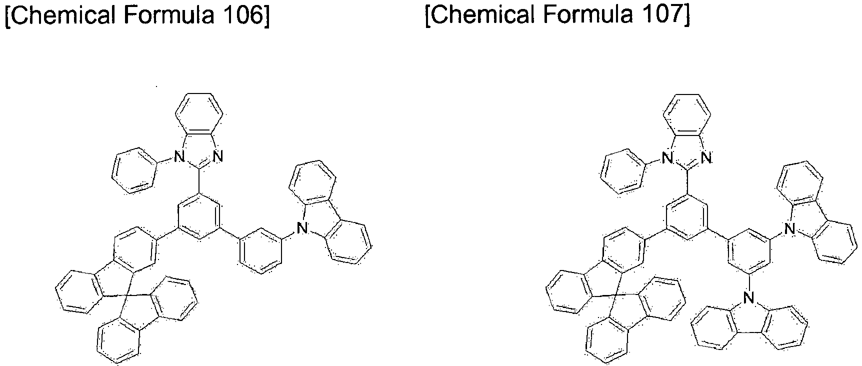

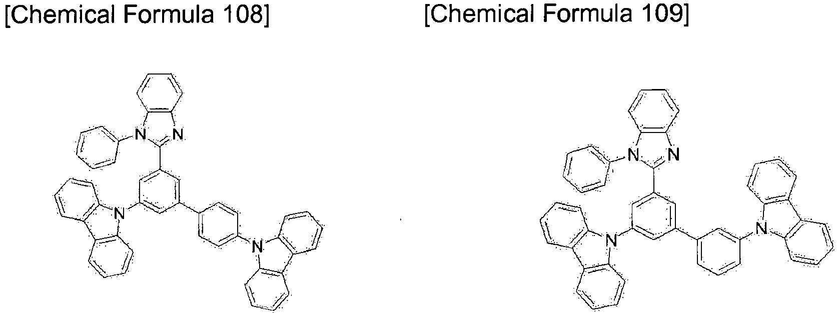

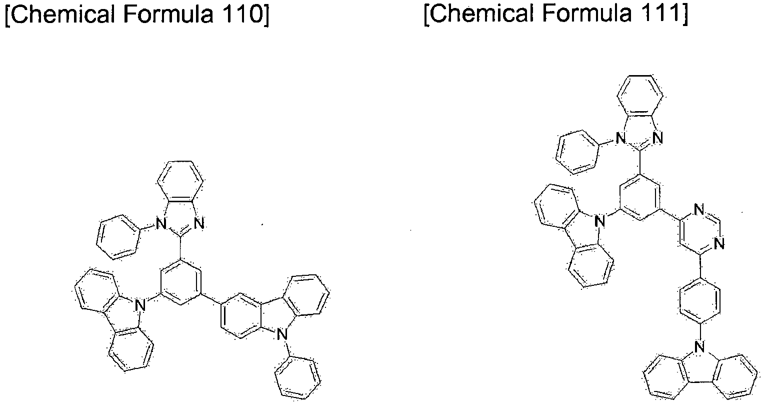

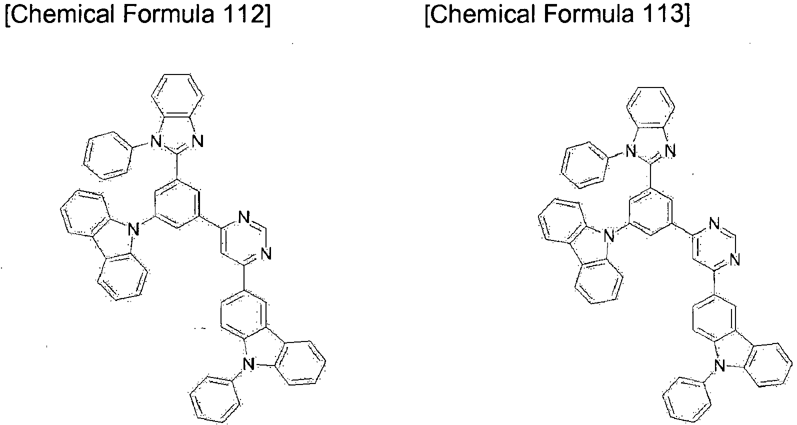

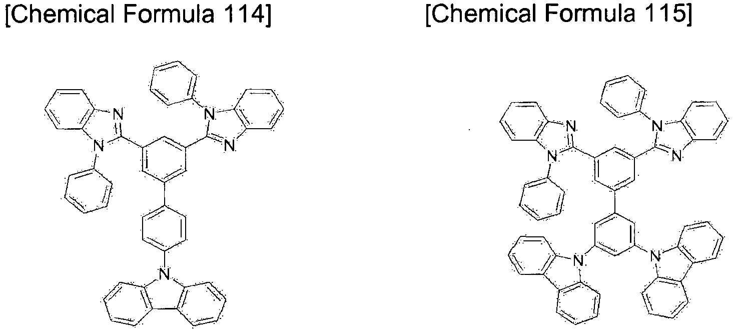

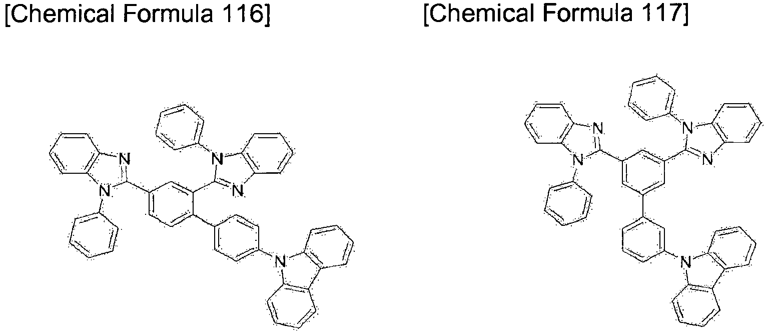

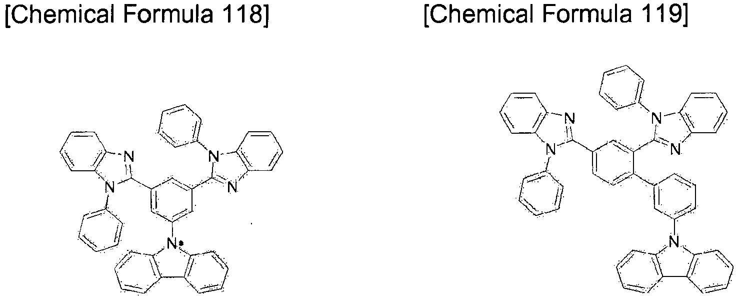

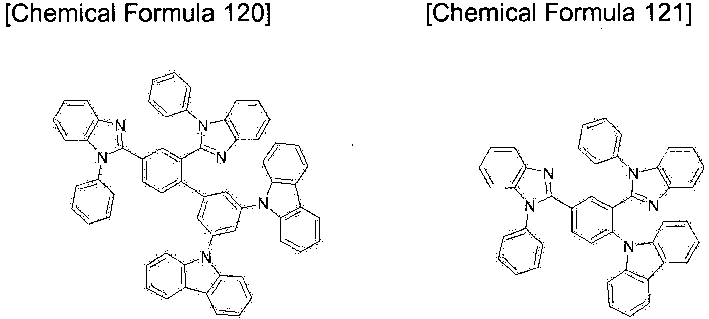

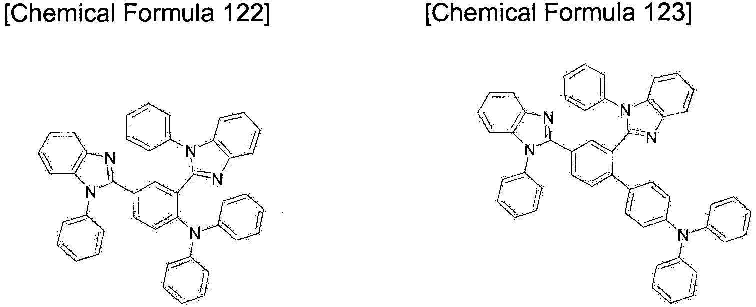

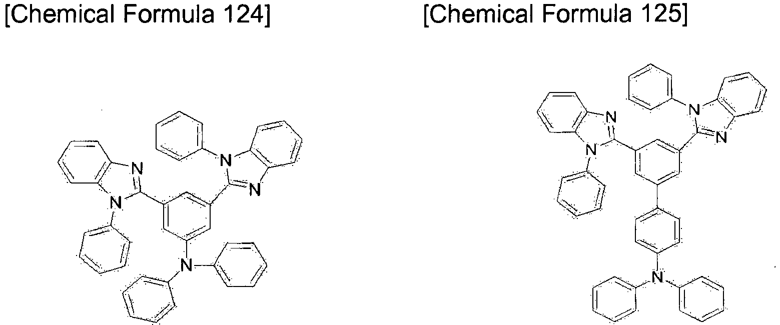

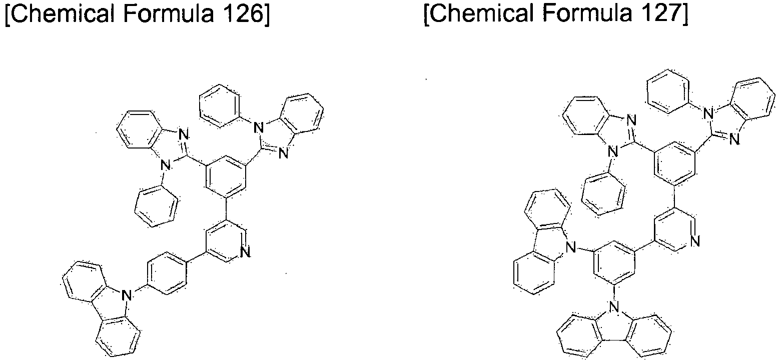

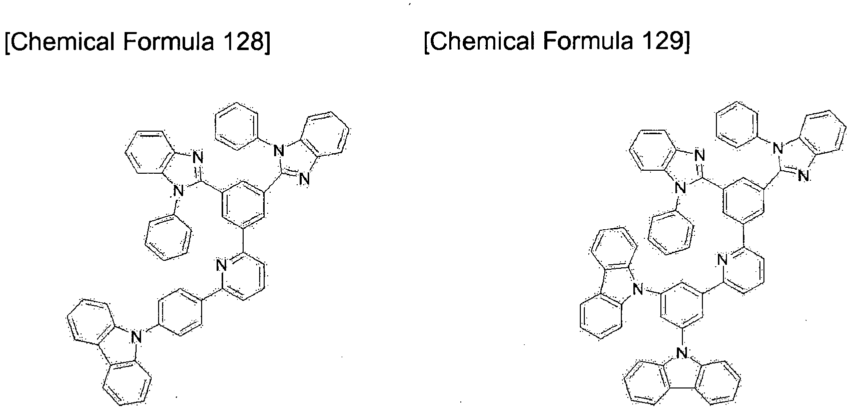

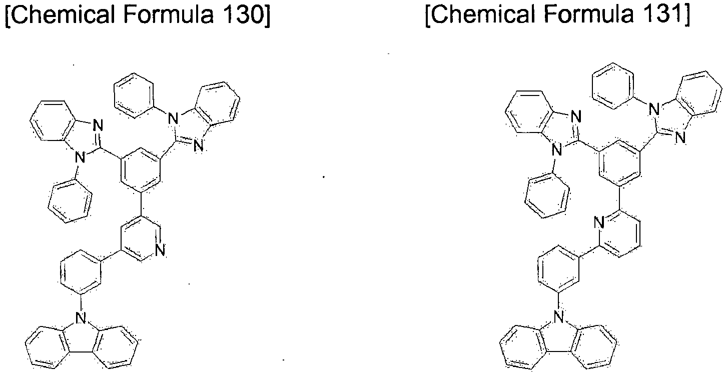

According to another embodiment of the present invention, an organic photoelectric device is provided that includes an anode, a cathode, and organic thin layers disposed between the anode and cathode. The organic thin layer includes the above benzimidazole compound.

According to a further embodiment of the present invention, a display element is provided that includes the organic photoelectric device.

Hereinafter, further embodiments of the present invention will be described in detail.

### [Advantageous Effects]

The benzimidazole compound is applicable as host materials, electron transporting materials, or hole blocking materials, and thus is used for an organic thin layer of an organic photoelectric device such as an organic emission layer, an electron transport layer (ETL) or a hole blocking layer.

### [Description of the Drawings]

FIG. 1 is a cross-sectional view showing an organic photoelectric device according to one embodiment of the present invention.
FIG. 2 shows LC-MS data of the compound M-6 prepared in Example 4 of the present invention.
FIG. 3 is a graph showing a photoluminescence (PL) wavelength of the compound M-6 prepared in Example 4.
FIG. 4 is a graph showing current density versus voltage of organic photoelectric devices fabricated using a solution process according to Example 8 of the present invention and Comparative Example 1.
FIG. 5 is a graph showing current density versus voltage of organic photoelectric devices fabricated using a solution process according to Example 9 of the present invention and Comparative Example 2.
FIG. 6 is a graph showing luminance versus voltage of organic photoelectric devices fabricated using a solution process according to Example 8 of the present invention and Comparative Example 1.
FIG. 7 is a graph showing luminance versus voltage of organic photoelectric devices fabricated using a solution process according to Example 9 of the present invention and Comparative Example 2.
FIG. 8 is a graph showing luminous efficiency versus luminance of organic photoelectric devices fabricated using a solution process according to Example 8 of the present invention and Comparative Example 1.
FIG. 9 is a graph showing luminous efficiency versus luminance of organic photoelectric devices fabricated using a solution process according to Example 9 of the present invention and Comparative Example 2.

### <Description of Reference Numerals Indicating Primary Elements in the Drawings>

| | |
|---|---|
| 11: substrate | 12: anode |
| 13: hole transport layer (HTL) | 14: organic emission layer |
| 15: electron transport layer (ETL) | 16: cathode |

### [Best Mode]

Exemplary embodiments of the present invention will hereinafter be described in detail. However, these embodiments are only exemplary, and the present invention is not limited thereto.

As used herein, when specific definition is not provided, the term "substituted" refers to one substituted with a substituent selected from the group consisting of a halogen, a cyano, a hydroxy, an amino, a nitro, a carboxyl, an azo, a ferro, a substituted or unsubstituted C1 to C30 alkyl, a substituted or unsubstituted C1 to C20 alkenyl, a substituted or unsubstituted C6 to C30 aryl, a substituted or unsubstituted C2 to C30 heteroaryl, a substituted or unsubstituted C1 to C20 alkoxy, a substituted or unsubstituted C6 to C20 aryloxy, a substituted or unsubstituted C3 to C40 silyloxy, a substituted or unsubstituted C1 to C20 acyl, a substituted or unsubstituted C2 to C20 alkoxycarbonyl, a substituted or unsubstituted C2 to C20 acyloxy, a substituted or unsubstituted C2 to C20 heteroaryloxy, a substituted or unsubstituted C7 to C20 aryloxycarbonyl amino, a substituted or unsubstituted C1 to C20 sulfamoyl amino, a substituted or unsubstituted C1 to C20 sulfonyl, a substituted or unsubstituted C1 to C20 alkylthiol, a substituted or unsubstituted C6 to C20 arylthiol, a substituted or unsubstituted C1 to C20 heterocyclothiol, a substituted or unsubstituted C1 to C20 ureide, a substituted or unsubstituted C1 to C20 phosphoric acid amide, and a substituted or unsubstituted C3 to C40 silyl.

As used herein, when specific definition is not provided, the term "hetero" refers to one including 1 to 3 heteroatoms selected from the group consisting of N, O, S, and P in one ring.

The benzimidazole compound according to the present invention include the compounds represented by the following Chemical Formulae 35 to 131.

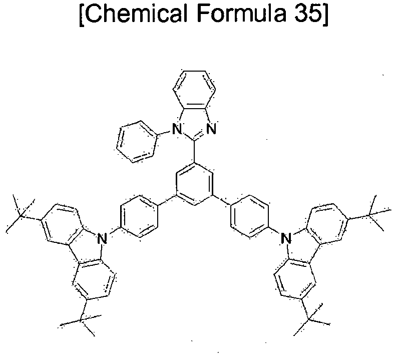

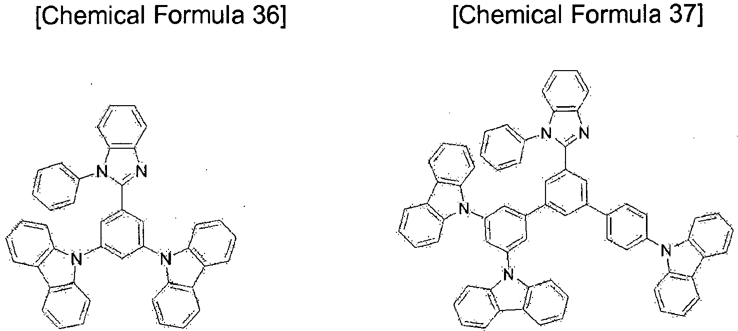

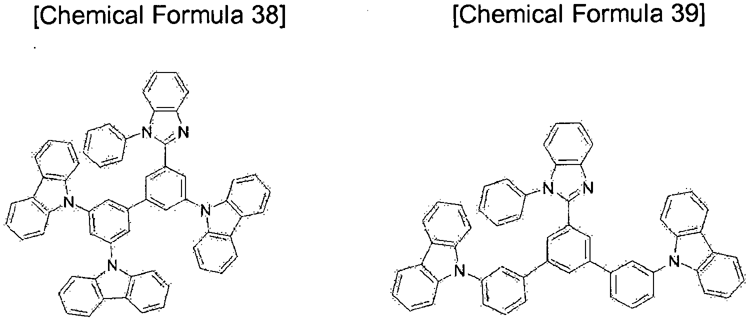

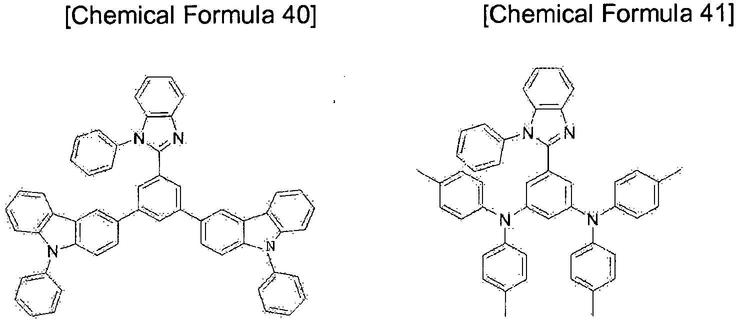

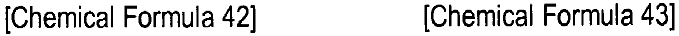

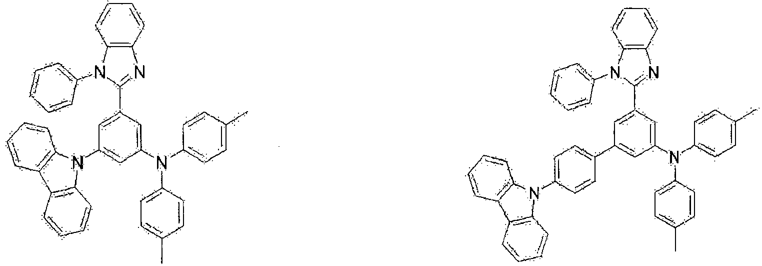

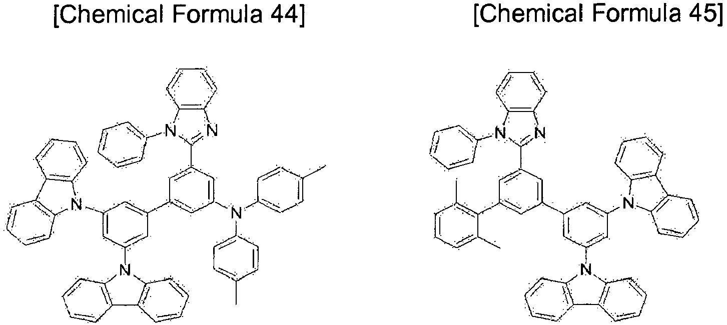

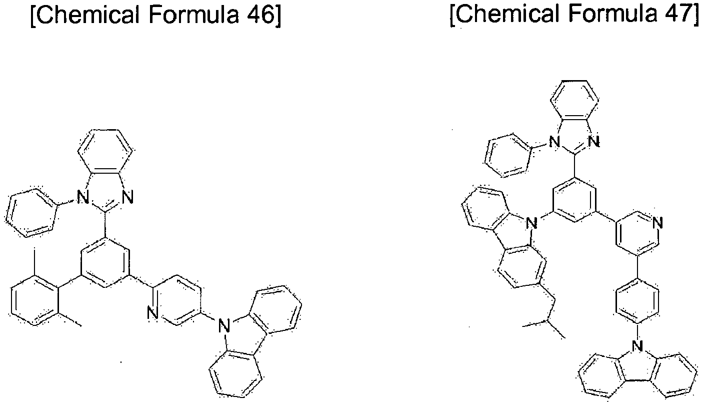

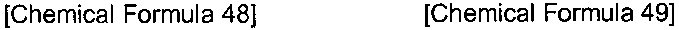

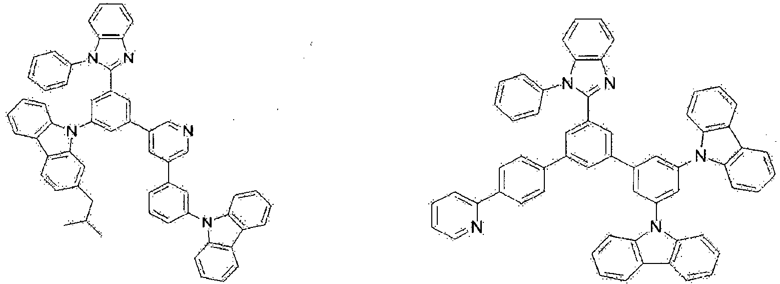

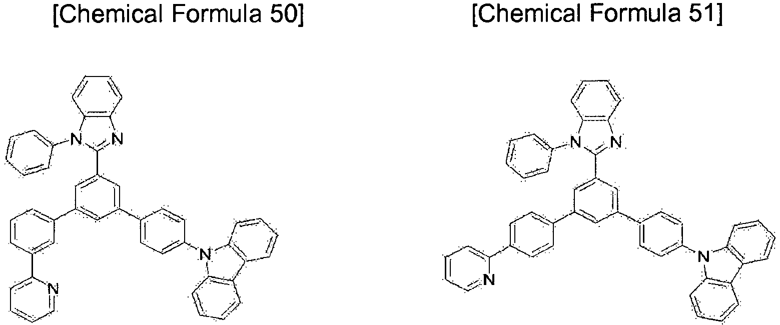

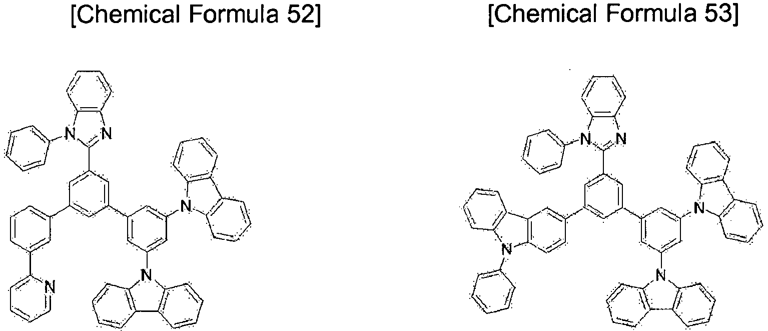

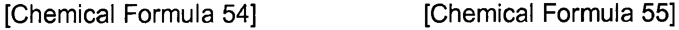

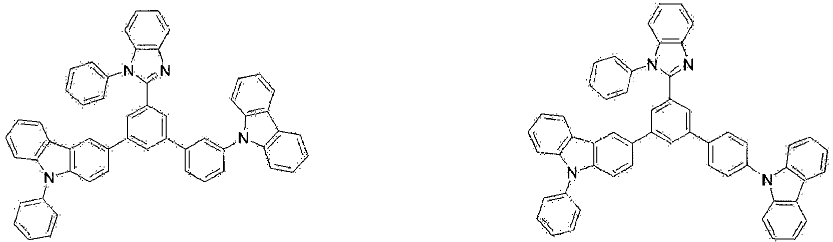

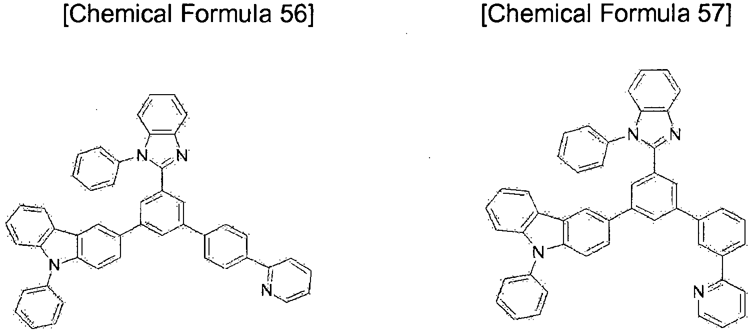

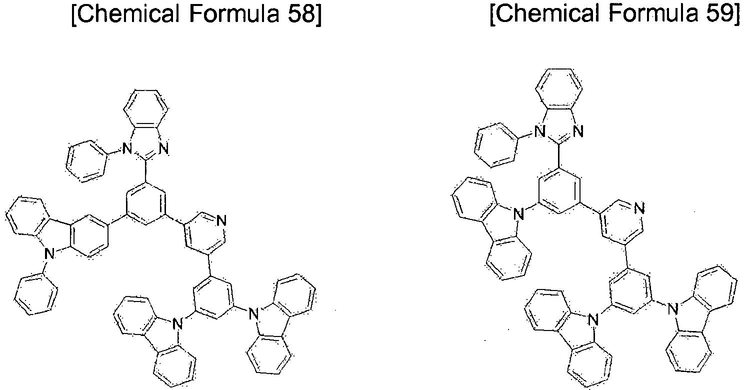

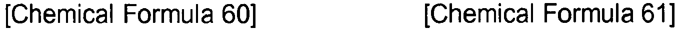

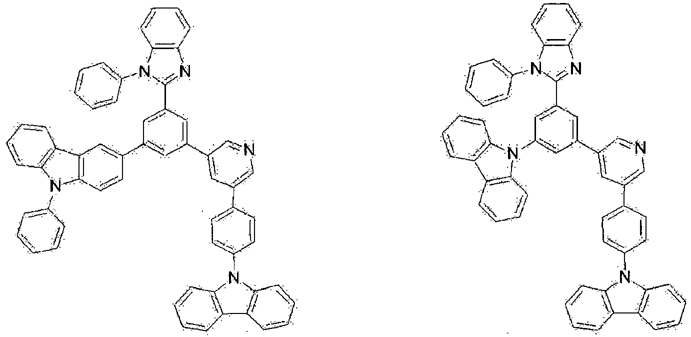

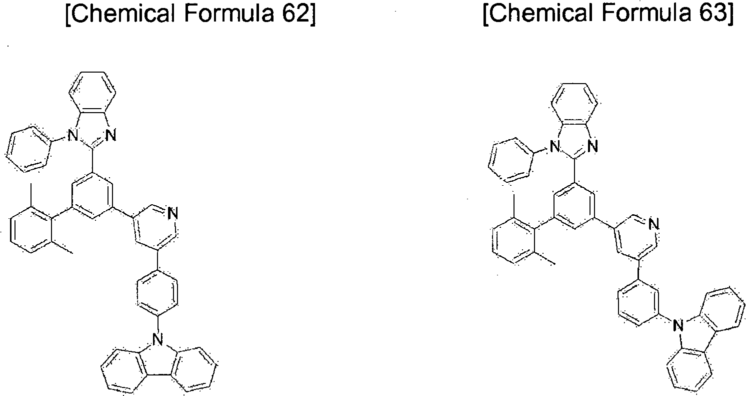

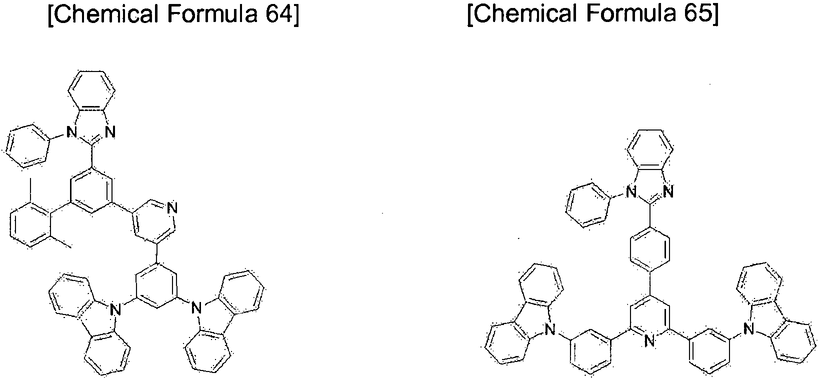

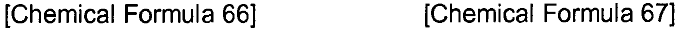

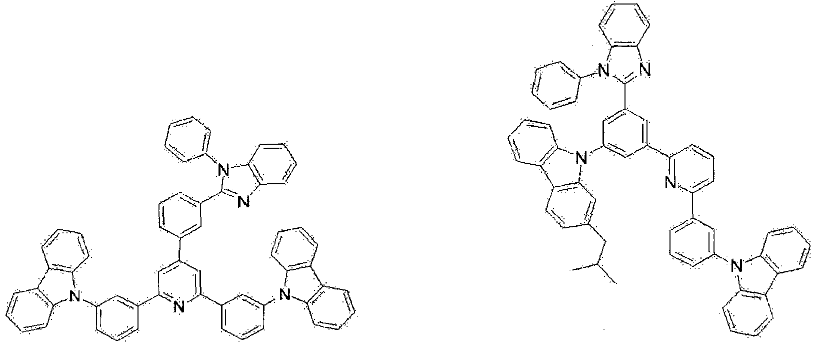

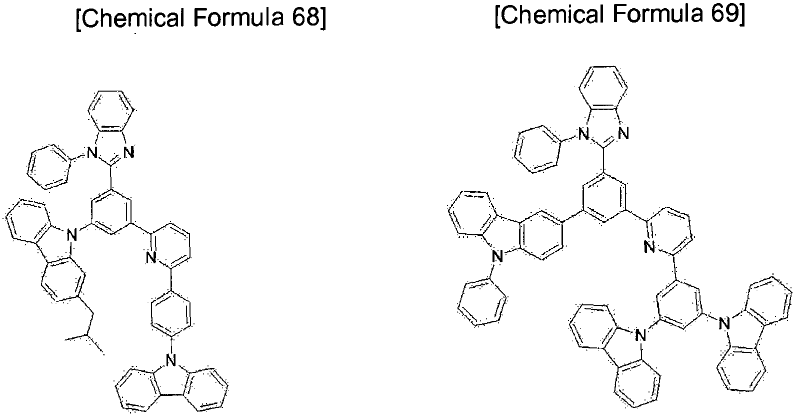

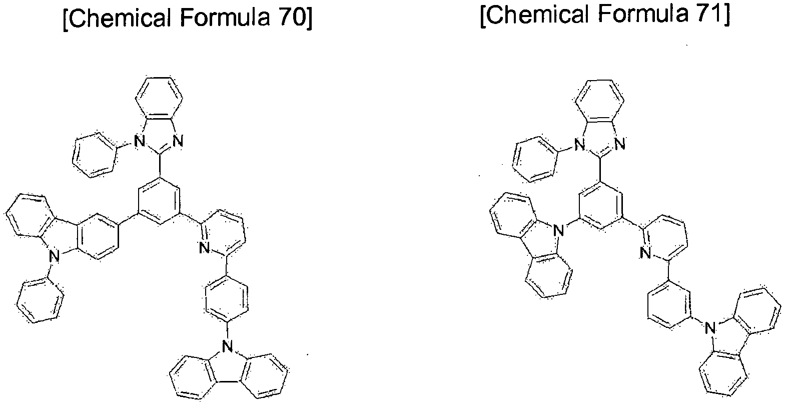

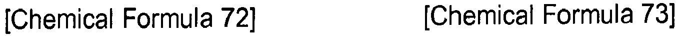

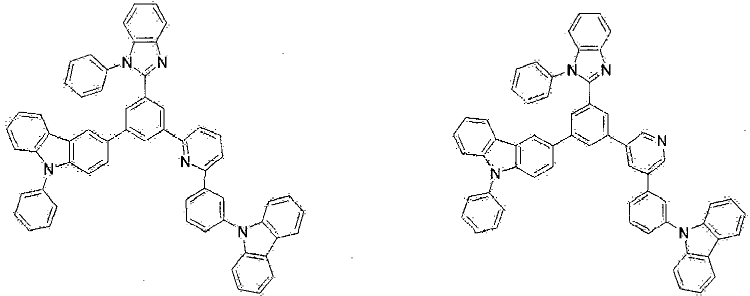

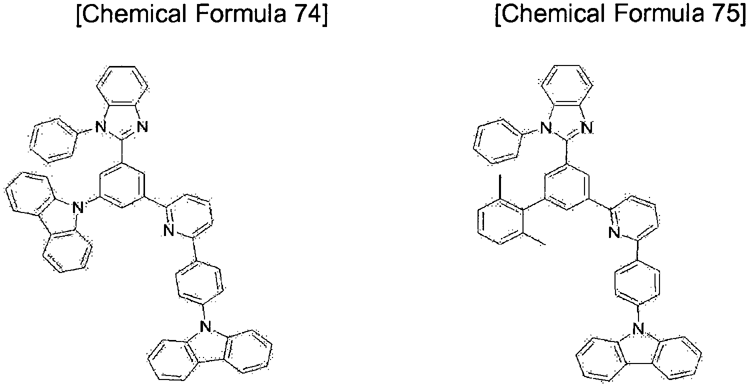

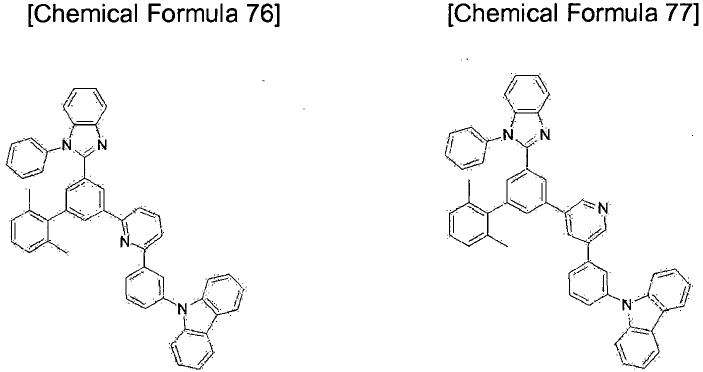

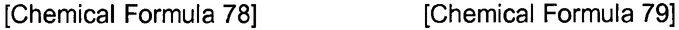

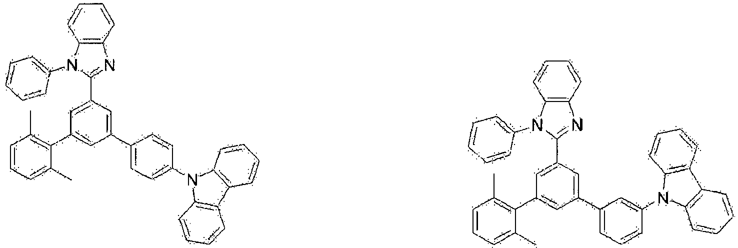

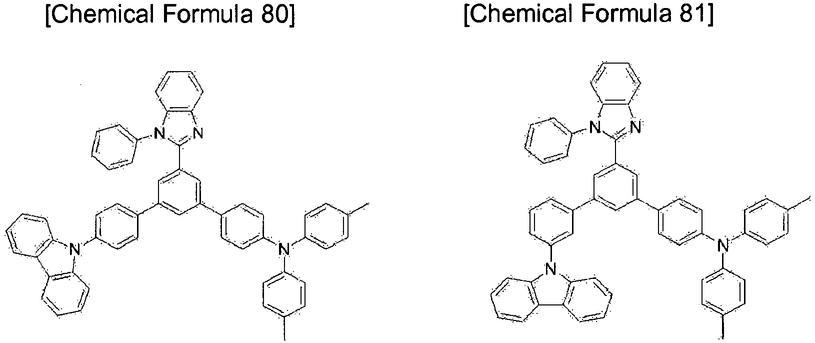

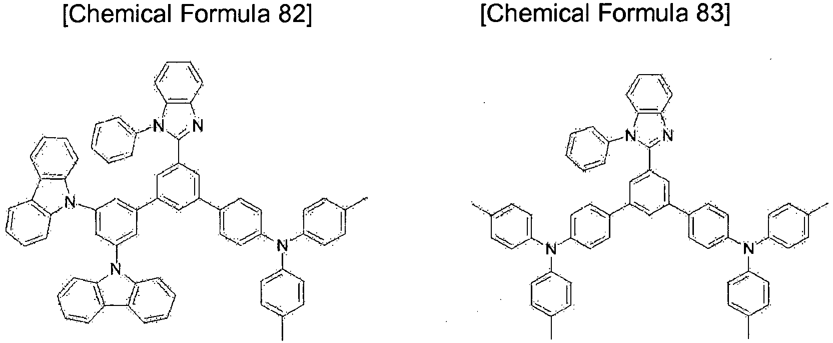

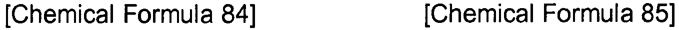

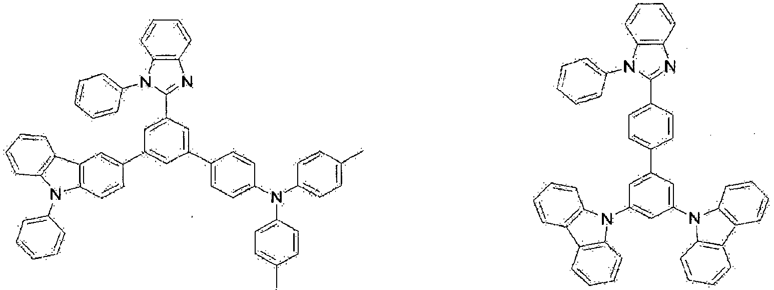

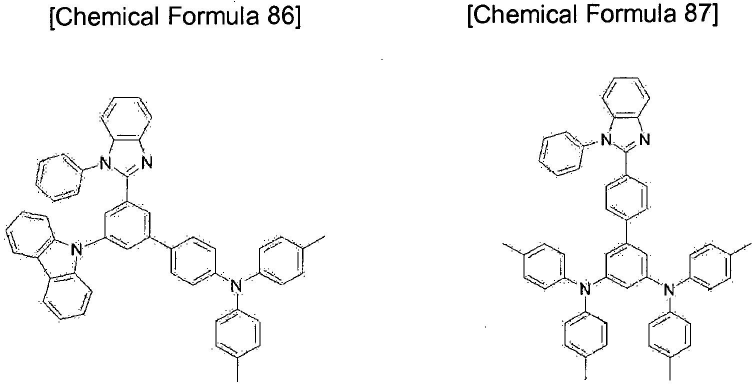

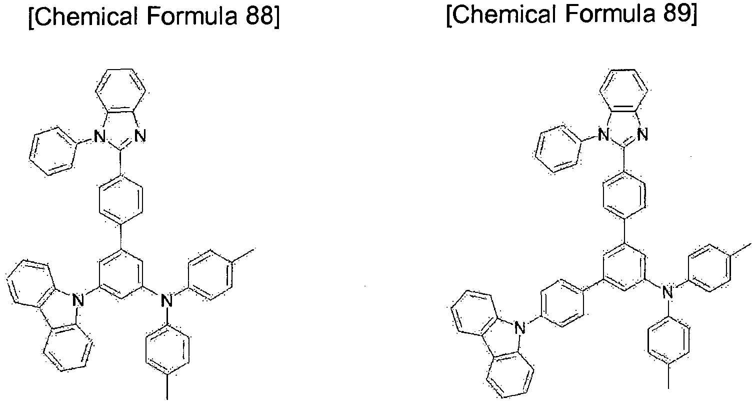

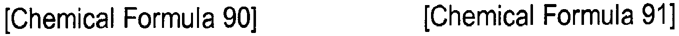

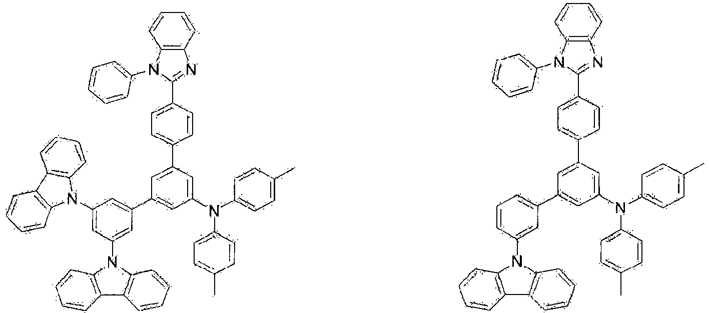

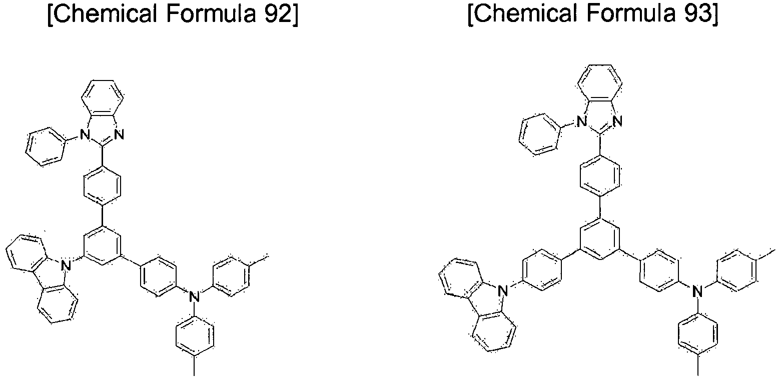

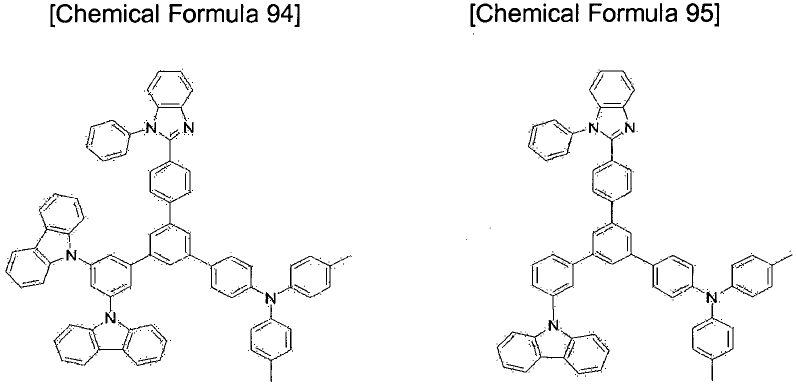

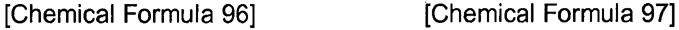

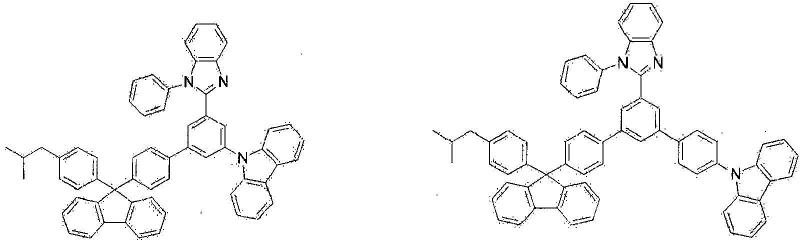

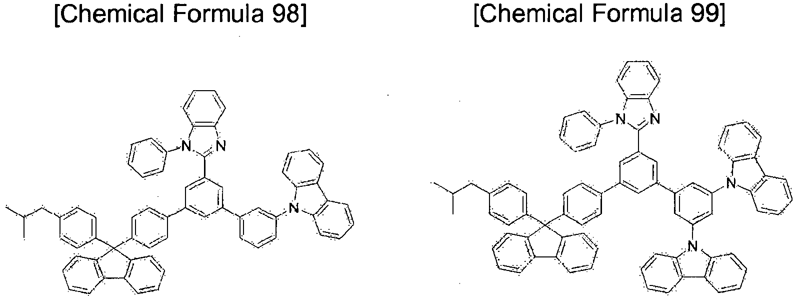

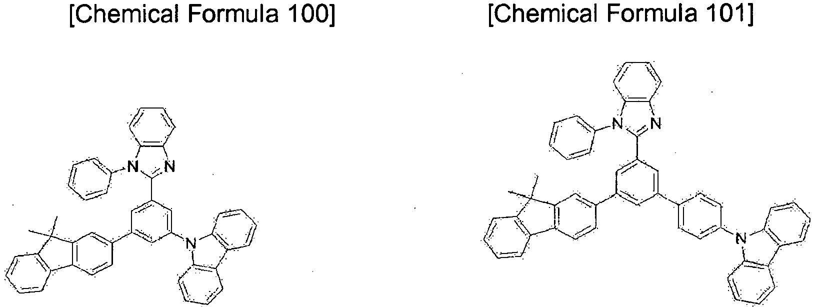

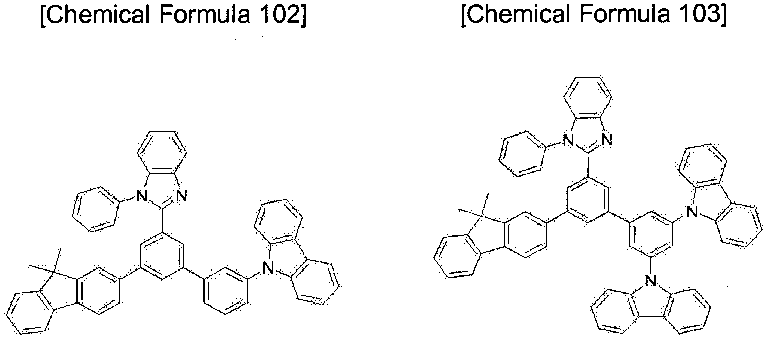

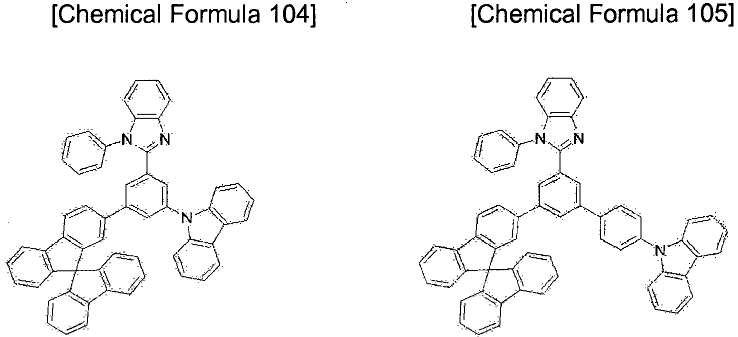

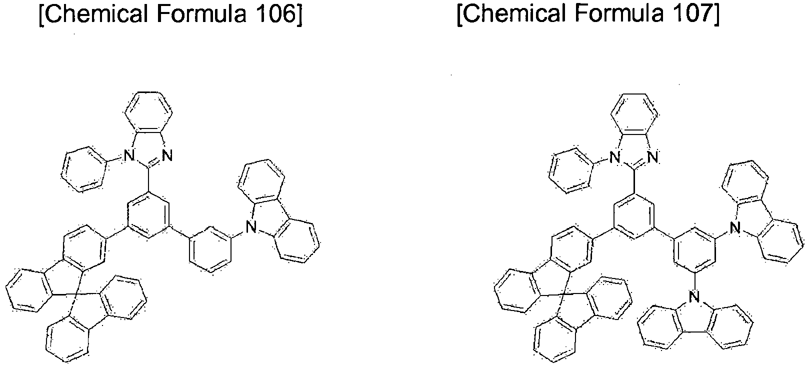

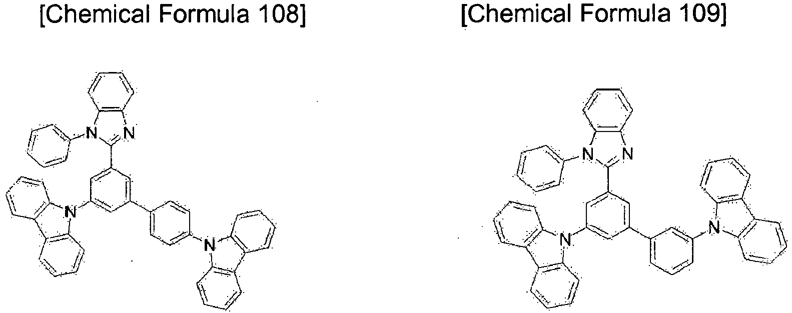

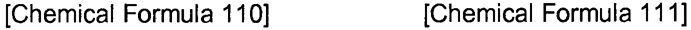

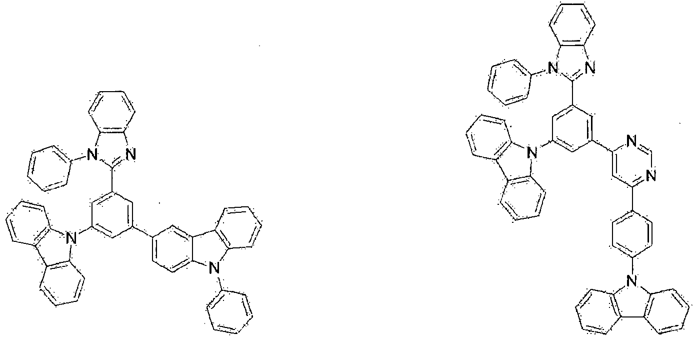

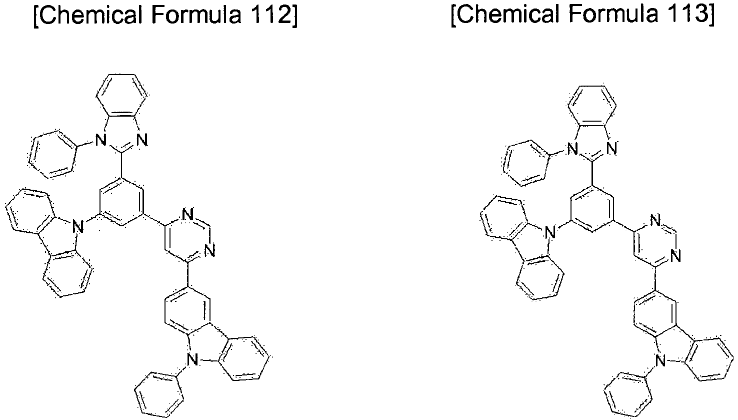

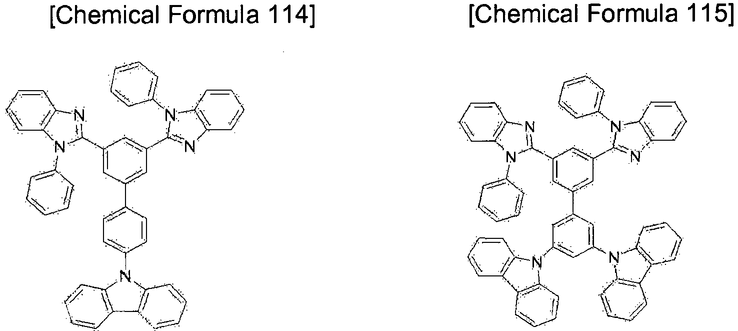

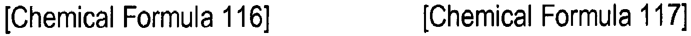

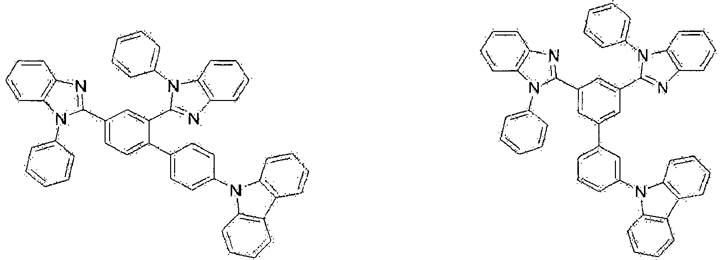

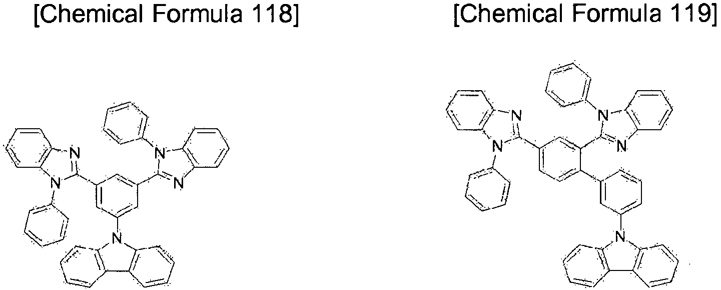

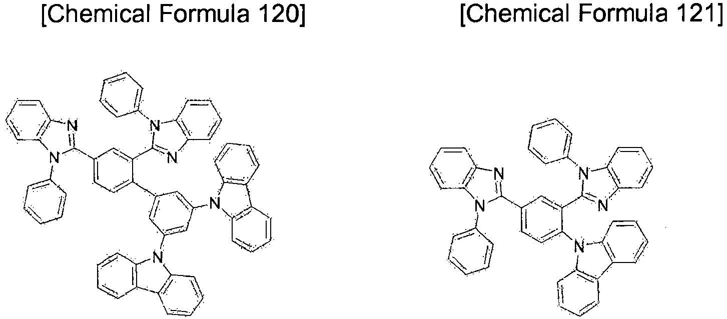

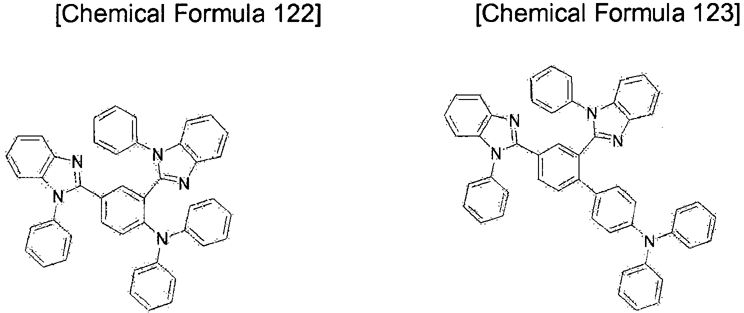

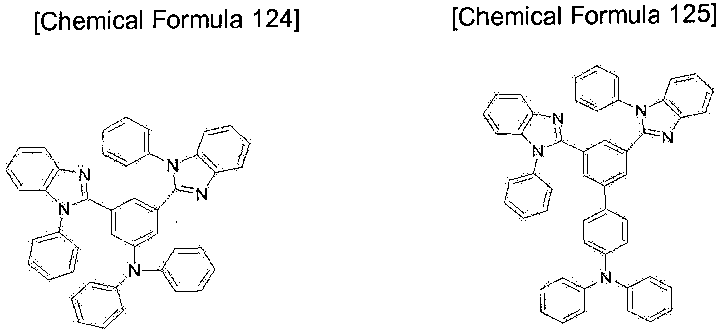

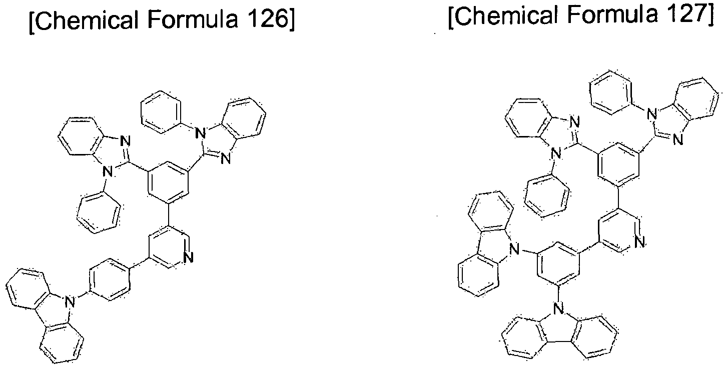

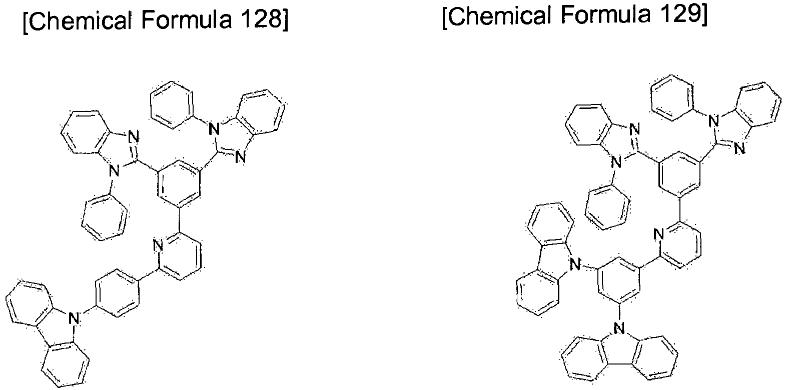

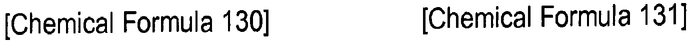

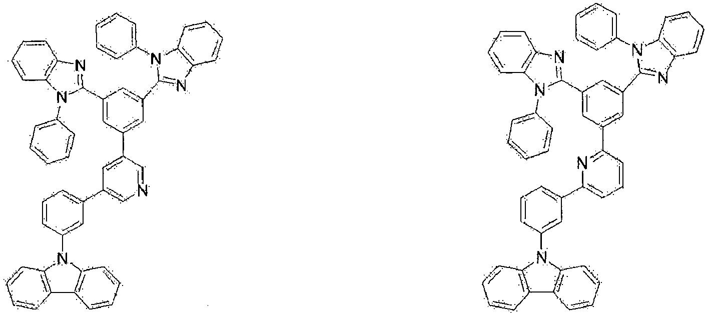

The benzimidazole compound according to one embodiment can be applicable to a host material or a charge transporting material for an organic photoelectric device. The benzimidazole compound may be also used as a nonlinear optical material, an electrode material, a chromic material, and as materials applicable to an optical switch, a sensor, a module, a waveguide, an organic transistor, a laser, an optical absorber, a dielectric material, and a membrane due to its optical and electrical properties.

When the benzimidazole compound is applied to a hole blocking layer as well as an electron transport layer (ETL) of a light emitting diode, its hole blocking properties may be reduced due to a hole transport backbone. Therefore, when the compound is applied to a hole blocking layer, it is preferable that it does not include a hole transport backbone. Such a hole transport backbone includes carbazoles, arylamines or penoxazines. However, when the compound is required to have electron transport and hole transport properties, the hole transport backbone introduction may improve lifespan and reduce the driving voltage of a light emitting diode.

In an organic photoelectric device according to another embodiment of the present invention including an anode, a cathode, and at least one organic thin layer interposed between the anode and cathode, at least one of the organic thin layers includes the benzimidazole compound. The organic photoelectric device includes an organic light emitting diode, an organic solar cell, an organic transistor, an organic photo-conductor drum or an organic memory device. In particular, the organic photoelectric device may preferably be an organic light emitting diode.

The benzimidazole compound may be applied to an emission layer of an organic thin layer. It may also be applied to an organic thin layer selected from the group consisting of an electron injection layer (EIL), an electron transport layer (ETL), a hole blocking layer, and combinations thereof.

An organic photoelectric device including an anode, a cathode, and an organic thin layer between the anode and cathode may include a general device structure including an anode, an emission layer, and a cathode. The organic thin layer of the organic photoelectric device may further include an inter-layer, a hole transport layer (HTL), and an electron transport layer (ETL). The inter-layer refers to a buffer layer such as a hole injection layer (HIL), a hole blocking layer, an electron injection layer (EIL), or an electron blocking layer.

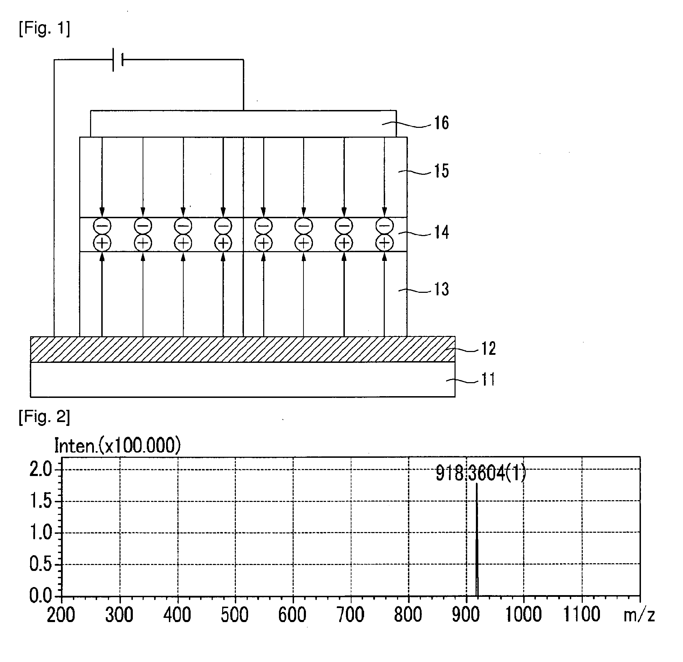

FIG. 1 is a cross-sectional schematic view of the organic photoelectric device 1 according to one embodiment. FIG. 1 shows an organic photoelectric device including a substrate 11, an anode 12, a hole transport layer (HTL) 13, an emission layer 14, an electron transport layer (ETL) 15, and a cathode 16.

Referring to FIG. 1, the organic photoelectric device may be fabricated using the compound.

First, an anode 12 material is coated on an upper side of the substrate 11.

The substrate 11 is a glass substrate or a transparent plastic substrate having excellent general transparence, face smoothness, handling ease, and water repellency.

The anode 12 material may include transparent and highly conductive indium tin oxide (ITO), tin oxide (SnO₂), zinc oxide (ZnO), or so on.

Then, a hole transport layer (HTL) 13 is disposed on the anode 12 using vacuum deposition, sputtering, or spin coating, and an emission layer 14 is disposed on the hole transport layer (HTL) 13 using vacuum deposition or a solution coating method such as spin coating, Inkjet printing, and so on.

An electron transport layer (ETL) 15 is disposed between the emission layer 14 and a cathode 16.

The emission layer 14, the hole transport layer (HTL) 13, and the electron transport layer (ETL) 15 may have a predetermined thickness but are not specifically limited. The emission layer 14 have a thickness of 5 nm to 1 µm, and preferably 10 to 500 nm, and the hole transport layer (HTL) 13 and electron transport layer (ETL) 15 respectively have a thickness of 10 to 10,000 A.

The electron transport layer (ETL) 15 is formed using vacuum deposition, sputtering, or spin coating of generally-used electron transport layer (ETL) 15 materials.

The hole transport layer (HTL) 13 and the electron transport layer (ETL) 15 play roles of efficiently transporting a carrier to the emission layer 14 to heighten light emitting recombination in the emission layer 14.

The hole transport layer (HTL) 13 material includes, but is not limited to, poly(3,4-ethylenedioxy-thiophene) (PEDOT) doped with poly(styrenesulfonic acid) (PSS), and N,N'-bis(3-methylphenyl)-N,N-diphenyl-[1,1'-biphenyl]-4,4'-diamine (TPD).

The electron transport layer (ETL) 15 material includes, but is not limited to, aluminum trihydroxyquinoline (Alq₃), a 1,3,4-oxadiazole derivative such as 2-(4-biphenylyl-5-phenyl-1,3,4-oxadiazole (PBD), a quinoxaline derivative such as 1,3,4-tris[(3-phenyl-6-trifluoromethyl)quinoxalin-2-yl] benzene (TPQ), and a triazole derivative.

The polymer may be mixed with a phosphorescent light emitting organic compound. The phosphorescent organic compound may be a phosphorescent light emitting organic metal complex from its triplet state, and is preferably a metal complex of at least one group VIII metal ion according to the periodic table of Gregor Johann Mendel. The group VIII metal ion includes a metal ion selected from the group consisting of Fe, Co, Ni, Ru, Rh, Pd, Os, Ir, and Pt, and is preferably Ir or Pt.

Examples of the metal complex may be represented by the following Chemical Formulae 132 to 134, but are not limited thereto.

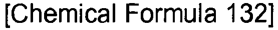

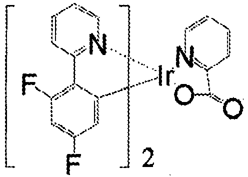

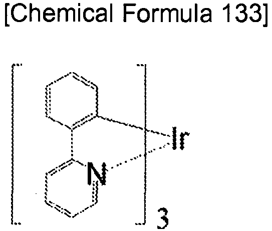

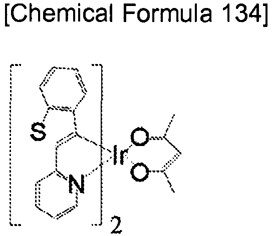

When the organic layer including the organic compound is formed using a solution coating, another low molecular host material can be included along with the organic compound. Examples of the low molecular host material include the compounds of the following Chemical Formulae 135 to 138, but are not limited thereto.

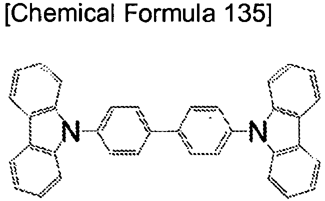

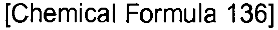

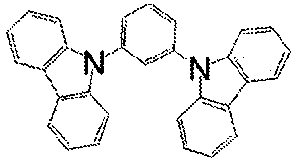

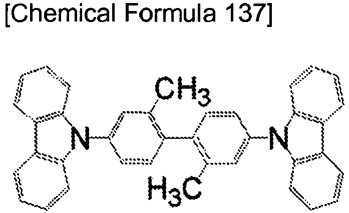

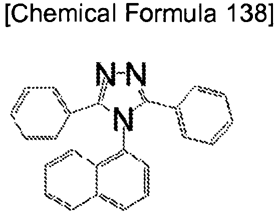

The benzimidazole compound may be used by mixing with polymers having conjugated double bonds such as fluorine-based polymers, polyphenylenevinylene-based polymers, and polyparaphenylene-based polymers, and also by mixing with binder resins.

The binder resins may include polyvinylcarbazole (PVK), polycarbonate, polyester, polyan arylate, polystyrene, acryl polymers, methacryl polymers, polybutyral, polyvinylacetal, diallylphthalate polymers, phenol resins, epoxy resins, silicone resins, polysulfone resins, or urea resins, and these resins can be used singularly and in combinations.

Selectively, a hole blocking layer may be disposed using vacuum deposition to limit transport speed of holes into the emission layer 14 and thus to increase the recombination opportunity of electrons and holes.

A cathode 16 material is coated on the electron transport layer (ETL).

The cathode material may be lithium (Li), magnesium (Mg), calcium (Ca), aluminum (Al), Al:Li, Ba:Li, or Ca:Li having a small work function.

### [Mode for Invention]

The following examples illustrate the present invention in more detail. However, it is understood that the present invention is not limited by these examples.

A person having ordinary skill in this art can sufficiently understand parts of the present invention that are not specifically described.

In the following Examples 1 to 6, monomers M-1 to M-9 for preparing a polymer according to one embodiment of the present invention were respectively prepared as shown in Reaction Schemes 1 to 9.

### Synthesis Example 1: Synthesis of M-1

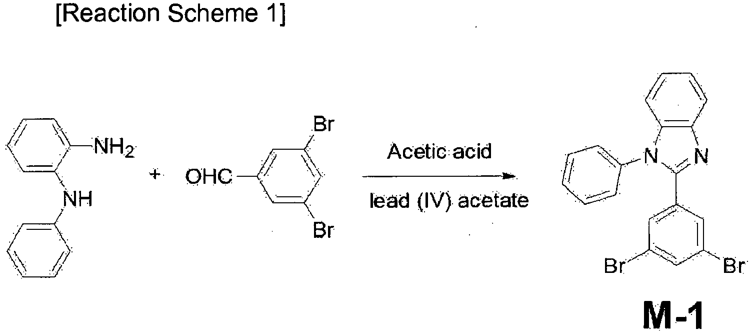

50 g (189 mmol) of 3,5-dibromobenzaldehyde and 38.3 g (208 mmol) of N-phenyl-o-phenylenediamine were put in a 500 Mℓ round bottom flask, and 200 Mℓ of acetic acid was added thereto. The resulting mixture was agitated for 30 minutes at room temperature, and 100 g (227 mmol) of lead acetate (lead, IV) was added thereto. The resulting product was agitated for 12 hours at room temperature. When the reaction was complete, the acetic acid was removed under reduced pressure. The reactant was dissolved in methylene chloride and washed five times with water. The organic solution was dried with anhydrous magnesium sulfate to remove the solvent. Then, the acquired solid was purified with a silica gel column in a methylene chloride solvent. The resulting product was recrystallized in a mixed solvent of methylene chloride/hexane in a ratio of 1:6, acquiring 34 g of a solid indicated as M-1 in the reaction scheme 1 (yield: 42%).

### Example 1: Synthesis of M-2

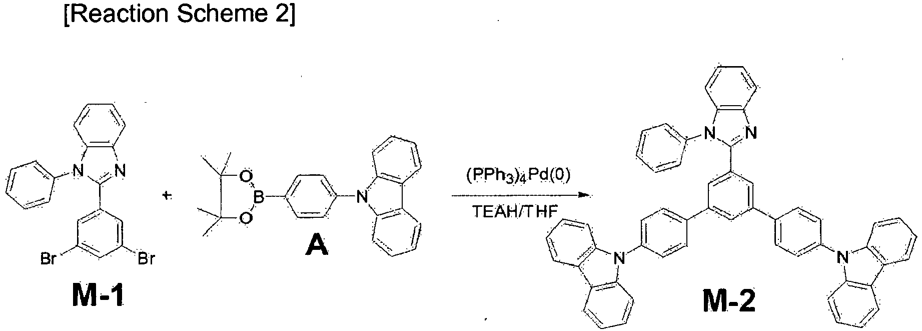

1.0g of M-1 (2.3 mmol), 1.89g of a material A (5.1 mmol), and 0.23 g of tetrakistriphenyl phosphine palladium (0.19 mmol) were dissolved in 30 mL of tetrahydrofuran (THF) under an argon atmosphere in a 100 Mℓ round bottom flask having a thermometer, a reflux condenser, and an agitator. Then, 15 mL of 20% tetratriethylammonium hydroxide was added thereto. The resulting product was agitated at 75 °C for 24 hours. When the reaction was complete, the reactant was cooled to room temperature and then extracted several times with methylene chloride and washed with water. The washed reactant was treated with anhydrous magnesium sulfate to remove moisture therefrom. The resulting reactant was filtrated to remove the solvent. When the solvent was removed, the acquired solid was recrystallized with a mixed solvent of methylene chloride/hexane in a ratio of 1:6, preparing 1.2 g of a white M-2 (yield: 68.4%). This was sublimated and purified to prepare 0.79 g of a white crystal. This crystal had a maximum light emitting wavelength at 383 nm when it was fabricated into a thin film. It had an LC-Mass theoretical value of C₅₅H₃₆N₄ [MH]⁺ 753.2940 and a measurement value of 753.2978.

### Example 2: Synthesis of M-3

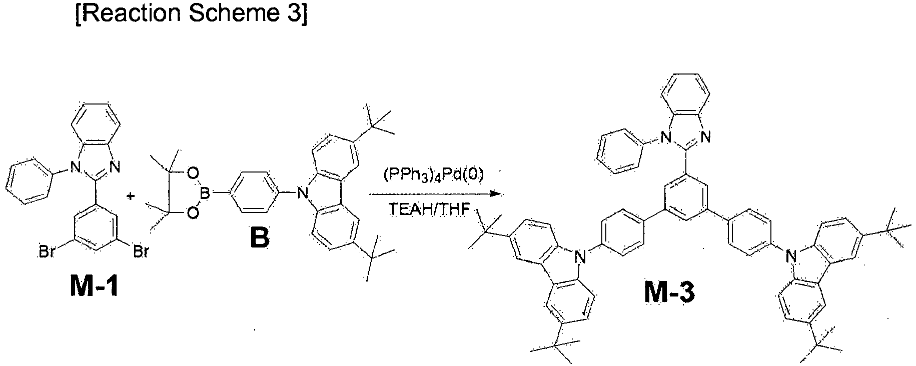

1.0 g (2.3 mmol) of M-1, 2.37 g (4.9 mmol) of a material B, and 0.23 g (0.19 mmol) of tetrakistriphenyl phosphine palladium were dissolved in 30 mL of THF under an argon atmosphere in a 100 Mℓ round bottom flask having a thermometer, a reflux condenser, and an agitator, and 15 mL of 20% tetratriethylammonium hydroxide was added thereto. The resulting mixture was agitated at 75 °C for 24 hours. When the reaction was complete, the reactant was cooled to room temperature and then extracted several times with methylene chloride and washed with water. Then, the washed reactant was treated with anhydrous magnesium sulfate to remove moisture and filtrated to remove the solvent. The resulting product was purified through a silica gel column with a methylene chloride solvent, preparing 1.78 g of a white M-3 (yield: 78.1%). When it was fabricated into a thin film, it had a maximum light emitting wavelength at 388 nm. It had an LC-Mass theoretical value of C₇₁H₆₈N₄ [MH]⁺ 977.5444 and measurement value of 977.5442.

### Example 3: Synthesis of M-4

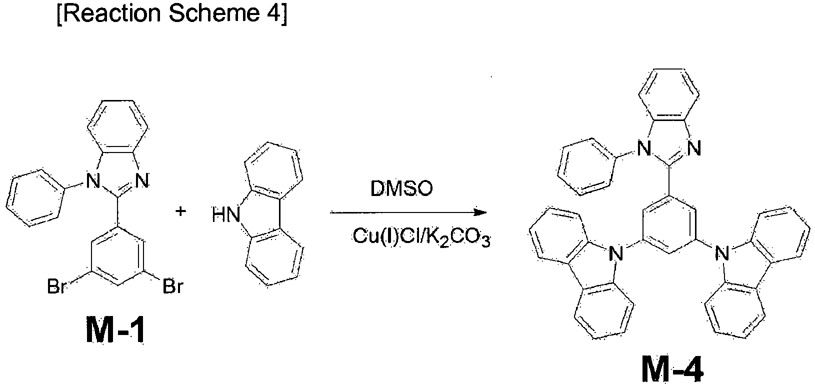

6 g (14 mmol) of M-1, 5.8 g (35 mmol) of carbazole, 1.5 g (15.1 mmol) of copper chloride, and 6g (43 mmol) of potassium carbonate were dissolved in 100 Mℓ of N,N-dimethylsulfoxide (DMSO) under an argon atmosphere in a 250 Mℓ round bottom flask having a thermometer, a reflux condenser, and an agitator. The resulting solution was agitated at 150 °C for 48 hours and cooled to room temperature. Then, DMSO was removed therefrom under reduced pressure. The remaining solid was dissolved in methylene chloride. The solution was washed several times with water to remove moisture with anhydrous magnesium sulfate. The resulting product was filtrated to remove the solvent. The acquired solid was purified through a silica gel column with a methylene chloride solvent, preparing 5.5 g of a white M-4 (yield: 65.4%). When it was prepared into a thin film, it had a maximum light emitting wavelength at 394 nm. It had an LC-Mass theoretical value of C₄₃H₂₈N₄ [MH]⁺ 601.2314 and a measurement value of 601.2384.

### Synthesis Example 2: Synthesis of M-5

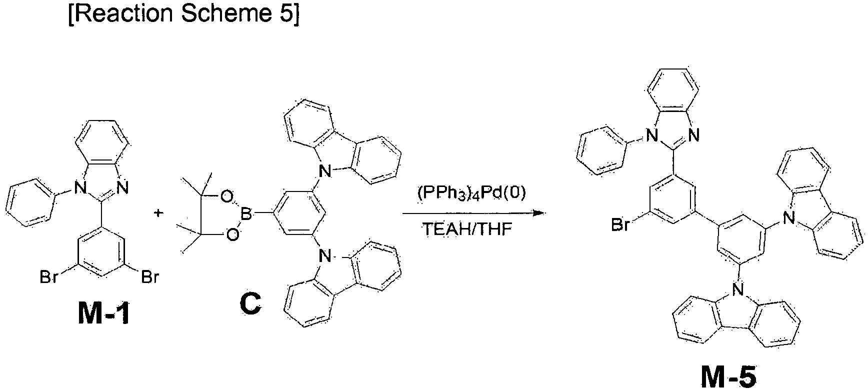

3.0 g (7.0 mmol) of M-1, 3.74 g (7.0 mmol) of a material C, and 0.16 g (0.14 mmol) of tetrakistriphenyl phosphine palladium were dissolved in 50 mL of tetrahydrofuran (THF) under an argon atmosphere in a 100 Mℓ round bottom flask having a thermometer, a reflux condenser, and an agitator, and 20 mL of 20% tetratriethylammonium hydroxide was added thereto. The resulting mixture was agitated at 75 °C for 24 hours.

When the reaction was complete, the reactant was cooled to room temperature and extracted several times with methylene chloride and washed with water. The washed reactant was treated with anhydrous magnesium sulfate to remove moisture. The remaining solid was filtrated to remove the solvent. The resulting product was purified through a silica gel column with a methylene chloride solvent, acquiring 3.0 g of a white M-5 (yield: 56.8%).

### Example 4: Synthesis of M-6

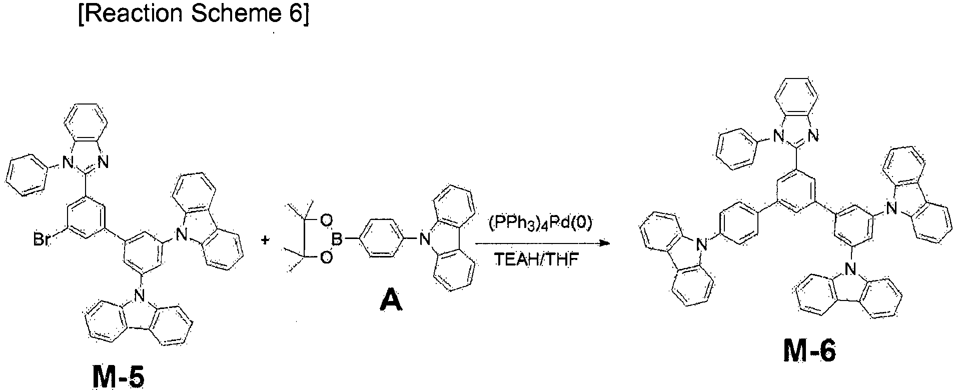

1.0 g (1.3 mmol) of M-5, 0.53 g (1.4 mmol) of a material A, and 0.15 g (0.16 mmol) of tetrakistriphenyl phosphine palladium were dissolved in 30 mL of THF under an argon atmosphere in a 100 Mℓ round bottom flask having a thermometer, a reflux condenser, and an agitator, and 15 mL of 20% tetratriethylammonium hydroxide was added thereto. The resulting mixture was agitated at 75 °C for 24 hours. When the reaction was complete, the reactant was cooled to room temperature and then extracted several times with methylene chloride and washed with water. The washed reactant was treated with anhydrous magnesium sulfate to remove moisture and then filtrated to remove the solvent. The resulting product was purified through a silica gel column with a methylene chloride solvent, acquiring 1.0 g of a white M-6 (yield: 82.6%). When it was fabricated into a thin film, it had a maximum light emitting wavelength at 390 nm. It had an LC-Mass theoretical value of C₆₇H₄₃N₅ [MH]⁺ 918.3518 and a measurement value of 918.3604.

### Example 5: Synthesis of M-7

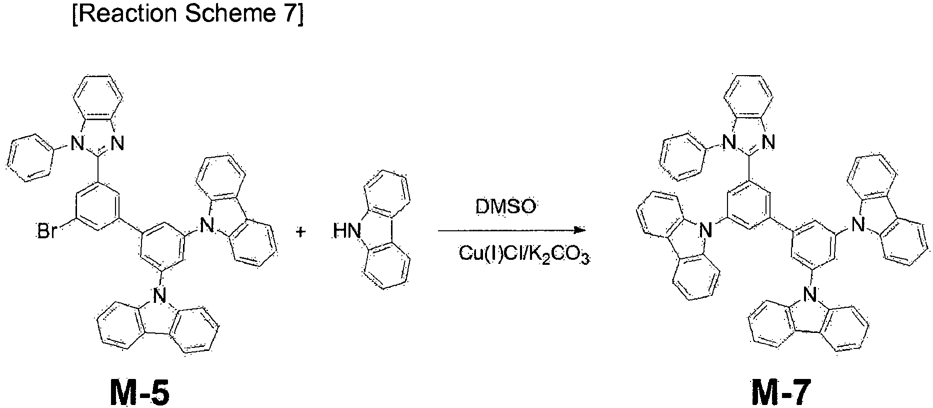

1.3 g (1.7 mmol) of M-5, 0.57 g (3.4 mmol) of carbazole, 0.08 g (0.86 mmol) of copper chloride, and 5.46 g (39 mmol) of potassium carbonate were dissolved in 30 Mℓ of DMSO under an argon atmosphere in a 250 Mℓ round bottom flask having a thermometer, a reflux condenser, and an agitator. The solution was agitated at 150 °C for 48 hours, and then cooled to room temperature and treated under reduced pressure to remove DMSO. The remaining solid was dissolved in methylene chloride, washed several times with water, treated with anhydrous magnesium sulfate to remove moisture, and then filtrated to remove the solvent. The resulting product was purified through a silica gel column with a methylene chloride solvent, acquiring 0.66 g of a white M-7 (yield: 45.5%). When it was fabricated into a thin film, it had a maximum light emitting wavelength at 404 nm. Its LC-Mass theoretical value was C₆₁H₃₉N₅ [MH]⁺ 842.3205, and the measurement value was 842.3331.

### Example 6: Synthesis of M-8

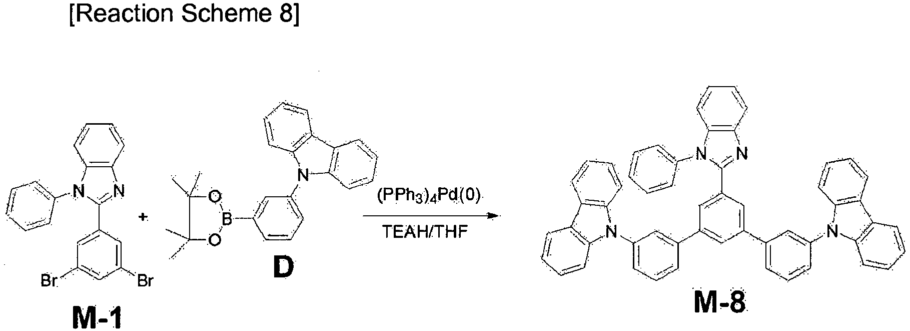

1.9 g (4.4 mmol) of M-1, 3.44 g (9.3 mmol) of a material D, and 0.4 g (0.34 mmol) of tetrakistriphenyl phosphine palladium were dissolved in 30 mL of THF under an argon atmosphere in a 100 Mℓ round bottom flask having a thermometer, a reflux condenser, and an agitator, and 15 mL of 20% tetratriethylammonium hydroxide was added thereto. The solution was agitated at 75 °C for 24 hours. When the reaction was complete, the reactant was cooled to room temperature and then extracted several times with methylene chloride and washed with water. The washed reactant was treated with anhydrous magnesium sulfate to remove moisture and filtrated to remove the solvent. The resulting product was treated with a methylene chloride solvent through a silica gel column, acquiring 1.5 g of a white M-8 (yield: 44.9%). It had an LC-Mass theoretical value of C₅₅H₃₆N₄ [MH]⁺ 753.2940 and a measurement value of 753.2949.

### Example 7: Synthesis of M-9

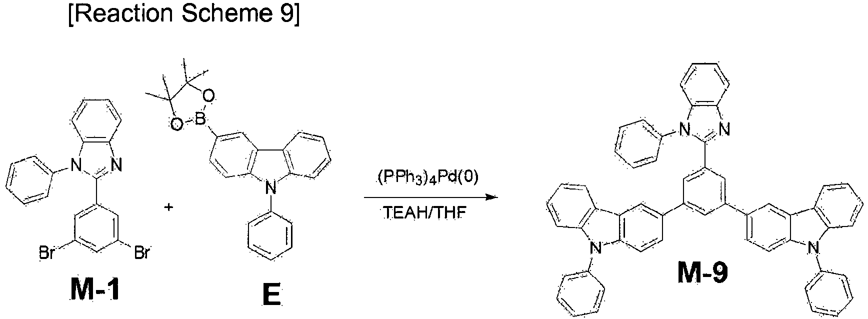

2.0 g (4.6 mmol) of M-1, 3.62 g (9.8 mmol) of a material E, and 0.4g (0.34 mmol) of tetrakistriphenyl phosphine palladium were dissolved in 30 mL of THF under an argon atmosphere in a 100 Mℓ round bottom flask having a thermometer, a reflux condenser, and an agitator, and 15 mL of 20% tetratriethylammonium hydroxide was added thereto. The solution was agitated at 75 °C for 24 hours. When the reaction was complete, the reactant was cooled to room temperature and then extracted several times with methylene chloride and washed with water. The washed reactant was treated with anhydrous magnesium sulfate to remove moisture and filtrated to remove the solvent. The remaining solid was purified with a methylene chloride solvent through a silica gel column, acquiring 1.6 g of a white M-8 (yield: 47.9%). It had a theoretical value of LC-Mass C₅₅H₃₆N₄ [MH]⁺ 753.2940 and a measurement value of 753.2980.

### Analysis and Characteristic Measurement of Compounds

The compounds (M-2 to M-4 and M-6 to M-9) of Examples 1 to 7 were measured regarding molecular weight to analyze the structure by using a liquid chromatography-mass analyzer (LC-MS, liquid chromatograph-mass spectrometry). LC-MS data of the compound M-6 prepared in Example 4 is shown in FIG. 2.

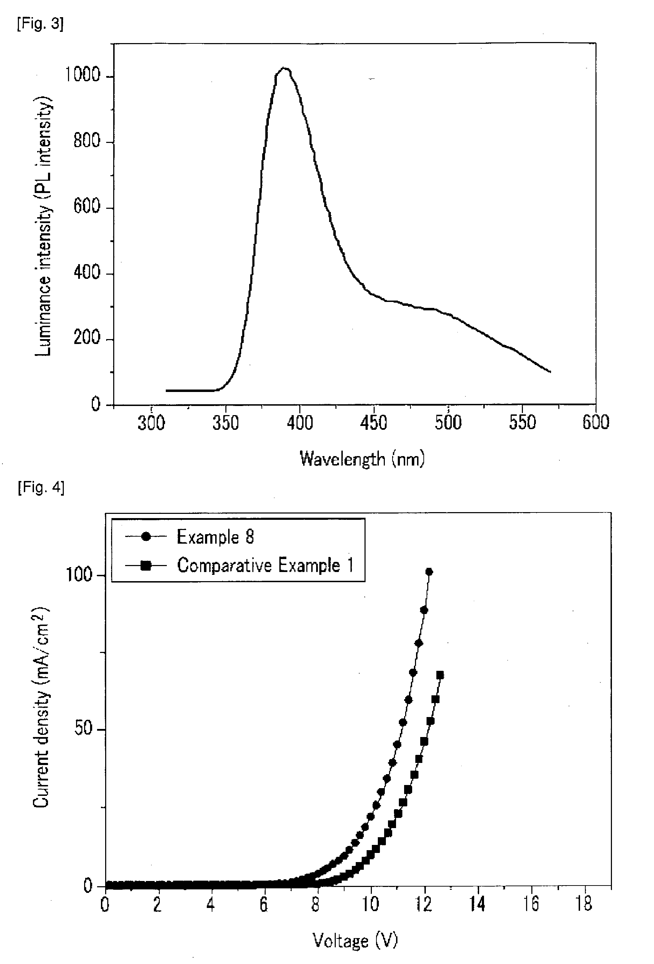

The compounds (M-2 to M-4 and M-6 to M-9) of Examples 1 to 7 were measured regarding photoluminescence (PL) wavelength by forming a thin film on a glass substrate by using a HITACHI F-4500 instrument to measure fluorescence characteristics. FIG. 3 shows the PL wavelength result of the M-6 according to Example 4. Referring to FIG. 3, when it was fabricated into a thin film, the M-6 had a maximum light emitting wavelength at 390 nm.

### Fabrication of an Organic Photoelectric Device

### Example 8: Devicefabrication using a solution process

An ITO substrate was used as an anode. The anode was spin-coated to form poly(3,4-ethylenedioxy-thiophene) (PEDOT) on the top thereof. Next, an emission layer 400 Å was spin-coated on the surface of the PEDOT by doping M-6 of Example 4 as a host and about 13 wt% of Ir(mppy)₃ as a dopant. On the emission layer, BAlq was vacuum-deposited to be 50 Å thick to form a hole blocking layer. Then, Alq₃ was vacuum-deposited to be 200 Å thick on top of the emission layer to form an electron transport layer (ETL). On the electron transport layer (ETL), 10 Å LiF and 1000 Å Al were sequentially vacuum-deposited to fabricate a cathode. Then, the anode and the cathode were used to fabricate an organic photoelectric device.

The organic photoelectric device included a 5-layered organic thin layer, and in particular, it was ITO 1500Å/ PEDOT 600Å/ EML (M-6:Ir(mppy)₃) 400Å/ BAlq 50Å/ Alq₃ 200Å/ LiF 10Å/ Al 1000Å.

### Comparative Example 1: Devicefabrication using a solution process

According to Comparative Example, a device included ITO 1500Å/ PEDOT 600Å/ EML (TCTA:TPBI 1:1, Ir(mppy)₃) 400Å/ BAlq 50Å/ Alq₃ 200Å/ LiF 10Å/ Al 1000Å.

Herein, the emission layer was spin-coated to be 400 Å thick by doping a mixture of 4,4',4"-tris(N-carbazolyl)triphenylamine (TCTA) and 2,2',2"-(1,3,5-benzenetriyl)tris(1-phenyl-1H-benzimidazole) (TPBI) prepared in a weight ratio of 1:1 as a host and about 13 wt% of Ir(mppy)₃ as a dopant.

A device using a solution process was fabricated according to the same method as in Example 8, except for the above.

### Example 9: Devicefabrication using a deposition process

An ITO substrate was used as an anode, and a device was fabricated by vacuum-depositing a layer thereon.

ITO/ DNTPD 600Å/ NPB 200Å/ M-4:Ir(ppy)₃,7 wt% 300Å/ BCP 50Å/ Alq₃ 250Å/LiF 10Å/ Al 1000A

The device of Example 9 included a hole transport layer (HTL) formed by vacuum-depositing 4,4'-bis[N-[4-(N,N-di-m-tolylamino)phenyl]-N-phenylamino]biphenyl (DNTPD) and N-(1-naphthyl)-N-phenyl-amino]biphenyl (NPB) to be respectively 600 Å and 200 Å thick.

In addition, a 50 Å-thick 2,9-dimethyl-4,7-diphenyl-1,10-phenanthroline (BCP) was used to form a hole blocking layer. It also included an emission layer formed to be 300 Å thick by vacuum-depositing M-4 of Example 3 as a host and about 7 wt% of Ir(ppy)₃ as a dopant.

The device using a deposit process was fabricated in the same method as in Example 8.

### Comparative Example 2: Devicefabrication using deposition process

ITO/ DNTPD 600Å/ NPB 200Å/CBP:Ir(ppy)₃, 7wt% 300Å/ CBP 50Å/ Alq₃ 250Å/ LiF 10Å/ Al 1000Å

According to Comparative Example 2, a device including an emission layer was formed by vacuum-depositing 4,4'-N,N'-dicarbazole-bipheyl (CBP) as a host and 7 wt% of Ir(ppy)₃ as a dopant to be 300 Å thick.

The device using a deposition process device was fabricated according the same method as in Example 9 except for the above.

### Performance Measurement of Organic Light Emitting Diodes

The organic light emitting diodes according to Examples 8 to 9 and Comparative Examples 1 and 2 were measured regarding current density and luminance change depending on voltage change and luminous efficiency change depending on luminance change. Specifically, they were measured as follows.

### 1) Current density change depending on voltage change

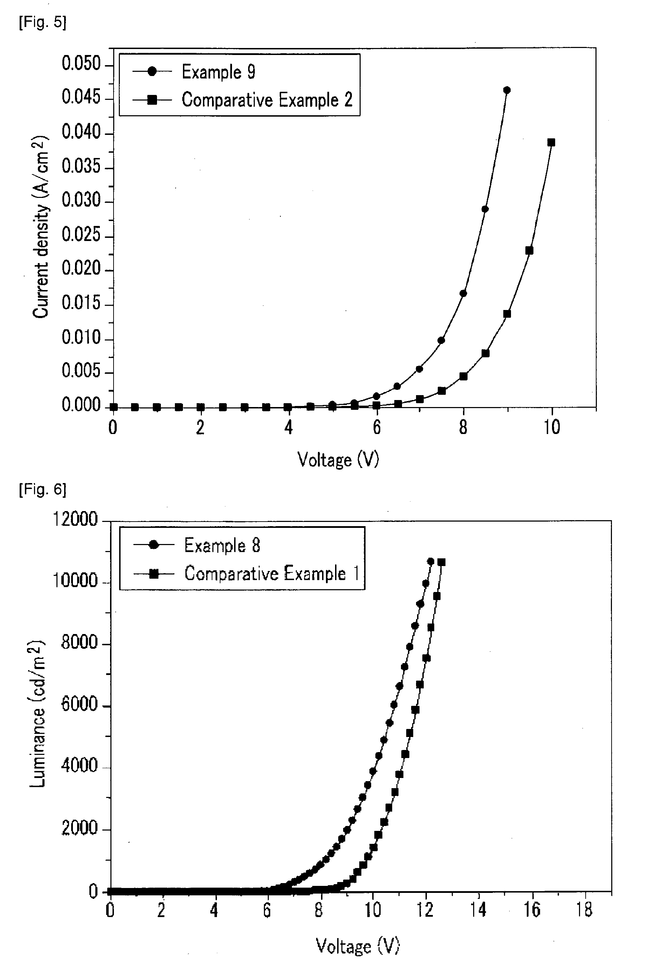

Each organic light emitting diode was measured regarding current value by using a current-voltage device (Keithley 2400) while its voltage was increased from 0. The current value was divided by area to calculate current density. The results are provided in FIGs. 4 and 5.

### 2) Luminance change depending on voltage change

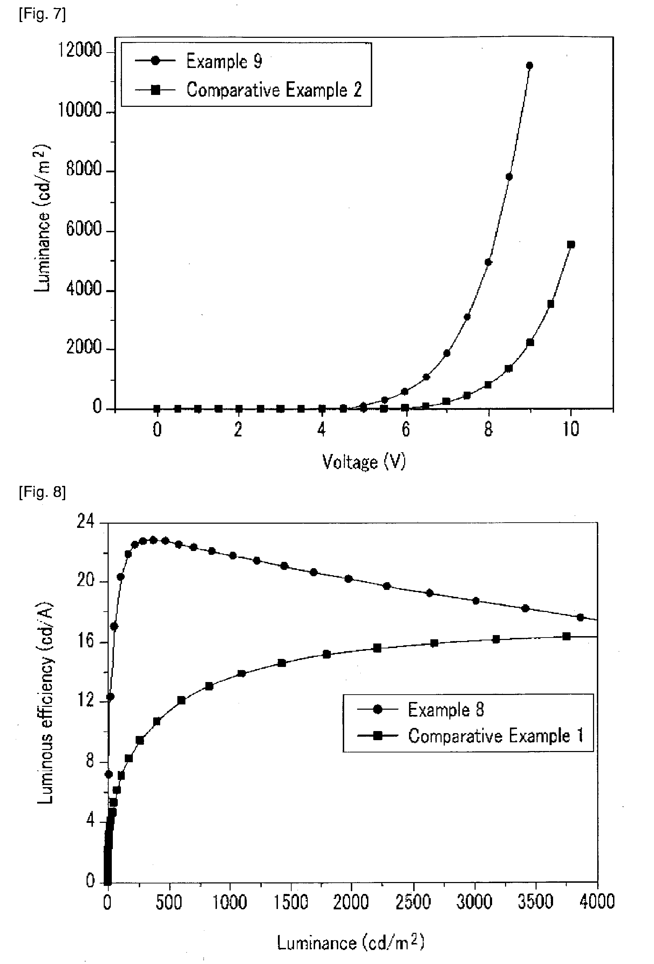

The organic light emitting diodes were measured regarding luminance by using a luminance meter (Minolta Cs-1000A) while its voltage was increased from 0. The results are provided in FIGs. 6 and 7.

### 3) Luminous efficiency measurement depending on luminance change

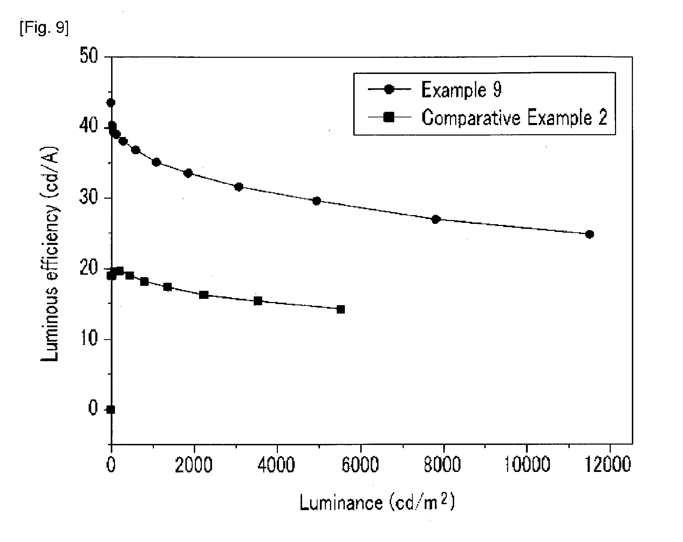

The organic light emitting diodes were measured regarding luminous efficiency change depending on luminance change. The results are provided in FIGs. 8 and 9.

Tables 1 and 2 comprehensively show all the results. In particular, Table 1 shows performance evaluation results of the solution process devices according to Comparative Example 1 and Example 8.

**[Table 1]**

| | Emission layer material | at 1000 cd/m² | | | Threshold voltage Vₜᵤᵣₙ on | Max. luminous efficiency | |
|---|---|---|---|---|---|---|---|
| | | Driving voltage | Luminous efficiency | | | | |
| | | (V) | (cd/A) | (Im/w) | (V) | (cd/A) | (Im/W) |
| Comp. Ex. 1 | TCTA:TPBI (1:1) | 9.80 | 13.90 | 4.45 | 4.80 | 16.50 | 4.71 |
| Ex. 8 | M-6 | 8.2 | 21.7 | 8.3 | 4.0 | 22.8 | 10.4 |

Referring to Table 1 and FIGs. 4, 6, and 8, a benzimidazole compound according to one embodiment of the present invention turned out to decrease the driving voltage of an organic light emitting diode as a host material and improve luminance and efficiency.

Table 2 shows performance evaluation results of the deposition process devices according to Comparative Example 2 and Example 9.

**Table 2**

| | Emission layer material | at 1000 cd/m² | | | Threshold voltage Vₜᵤᵣₙ on | Max. luminous efficiency | |
|---|---|---|---|---|---|---|---|
| | | Driving voltage | Luminous efficiency | | | | |
| | | (V) | (cd/A) | (Im/w) | (V) | (cd/A) | (Im/W) |
| Comp. Ex. 2 | CBP | 8.8 | 17.8 | 7.6 | 5 | 19.7 | 11.3 |
| Ex. 9 | M-4 | 7.0 | 35.4 | 19.3 | 3.5 | 43.5 | 43.2 |

Referring to Table 2 and FIGS. 5, 7, and 9, a benzimidazole compound according to one embodiment of the present invention turned out to decrease the driving voltage of an organic light emitting diode as a host material and improve luminance and efficiency.

The present invention is not limited to the embodiments, but can be fabricated with various modifications and equivalent arrangements included within the spirit and scope of the appended claims by a person who is ordinarily skilled in this field. Therefore, the aforementioned embodiments should be understood to be exemplary but not limiting the present invention in any way.

## Claims

1. A benzimidazole compound represented by the following Chemical Formula 35 to 131:

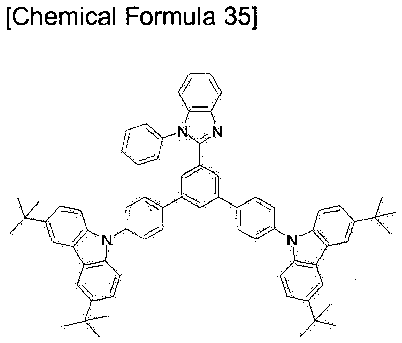

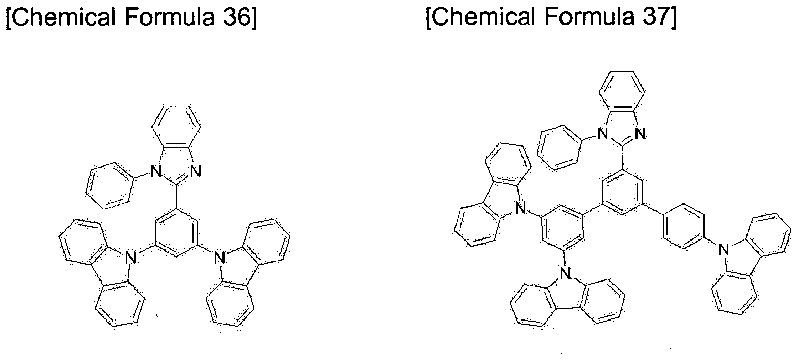

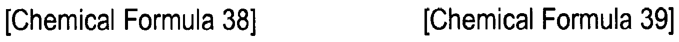

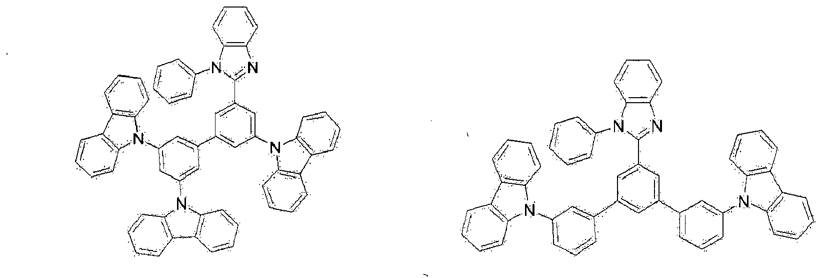

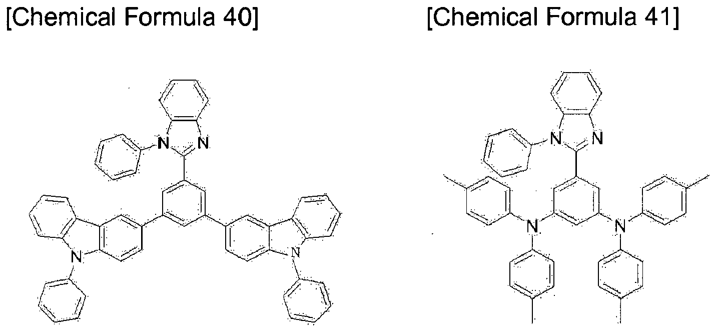

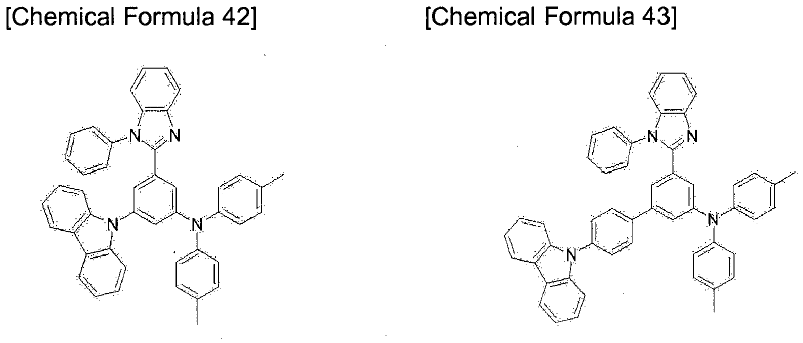

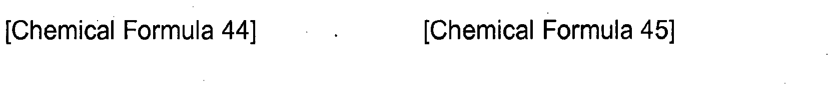

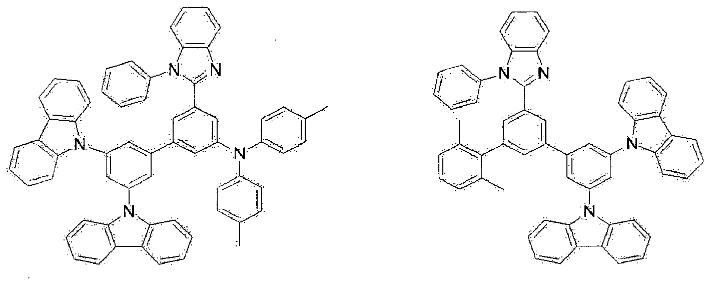

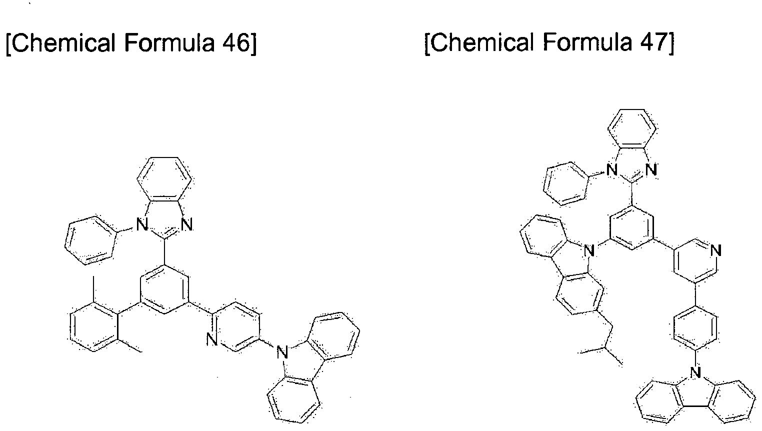

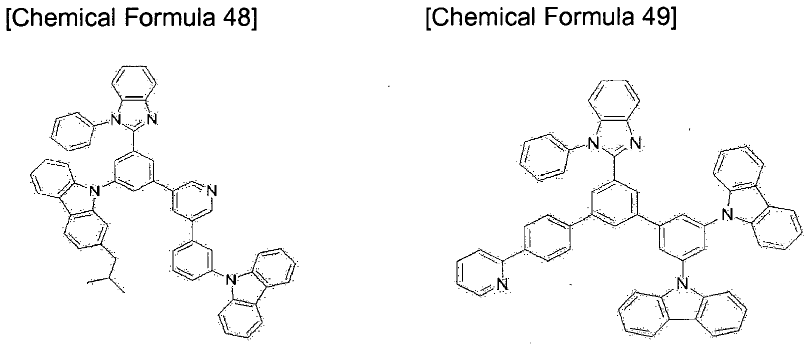

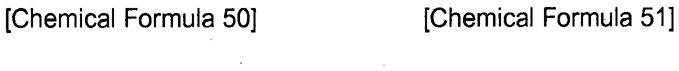

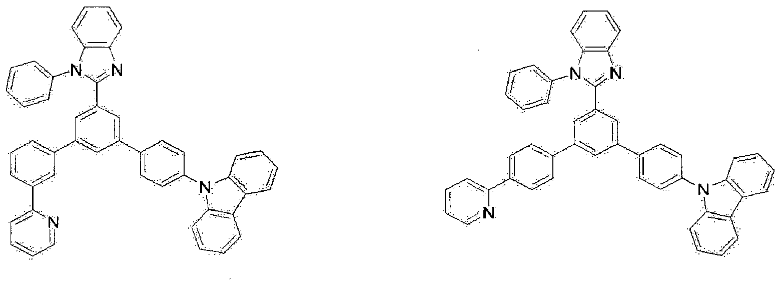

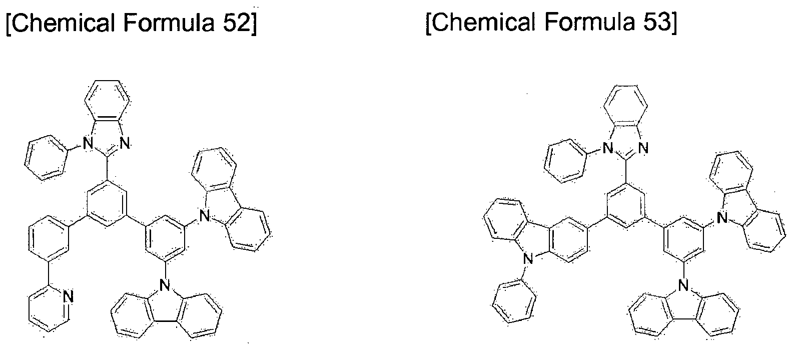

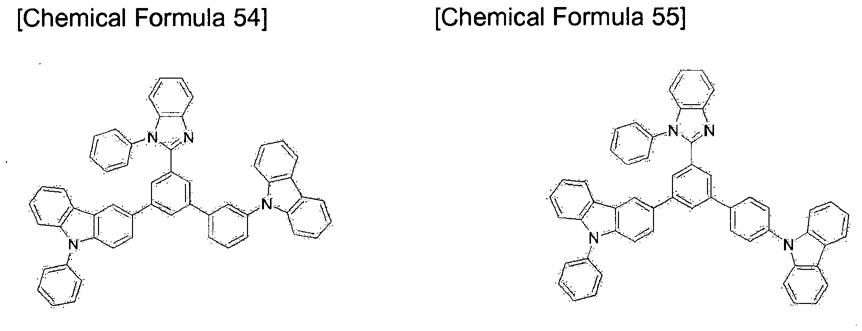

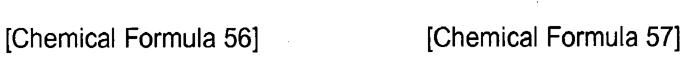

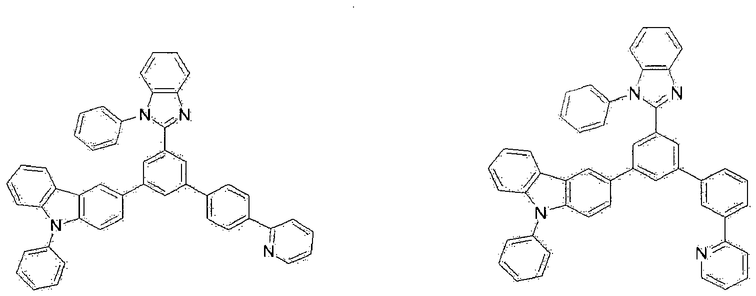

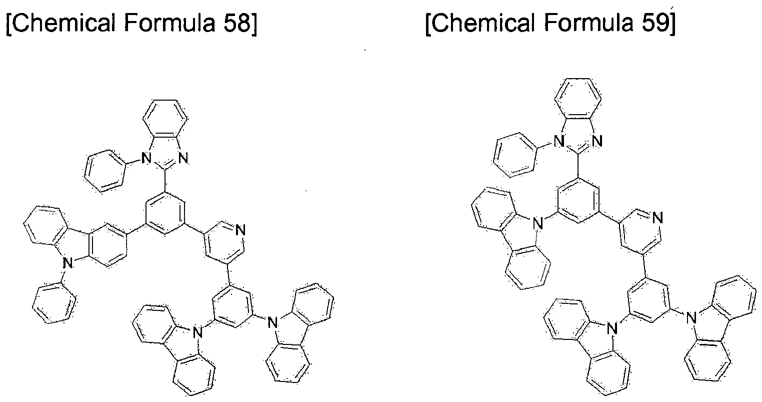

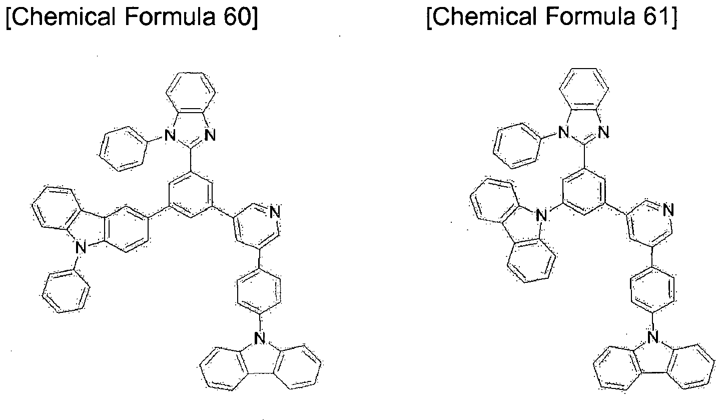

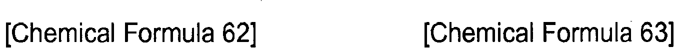

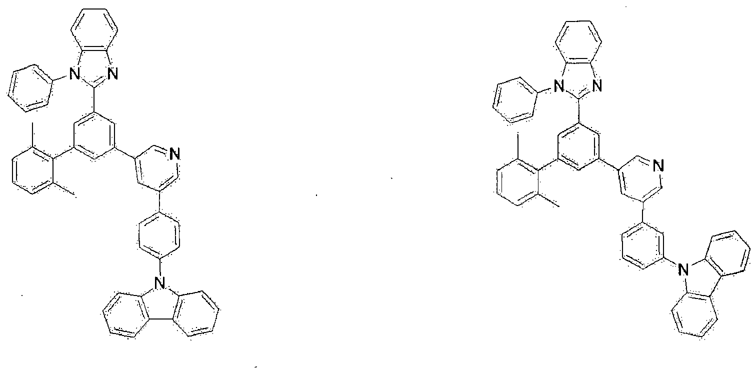

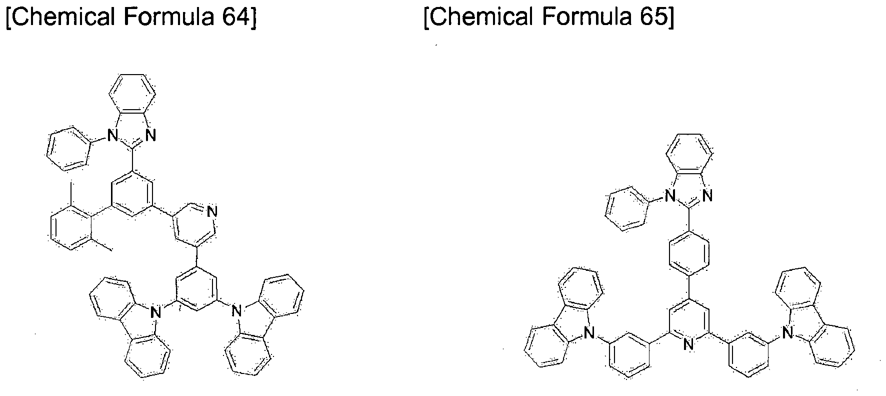

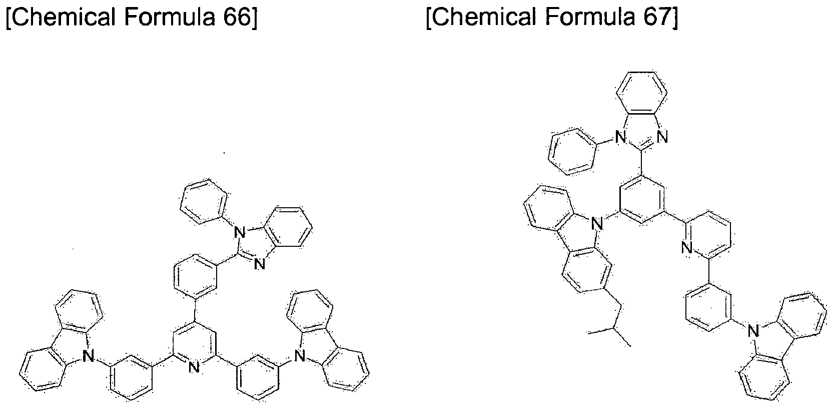

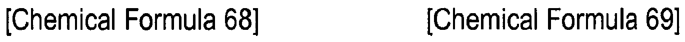

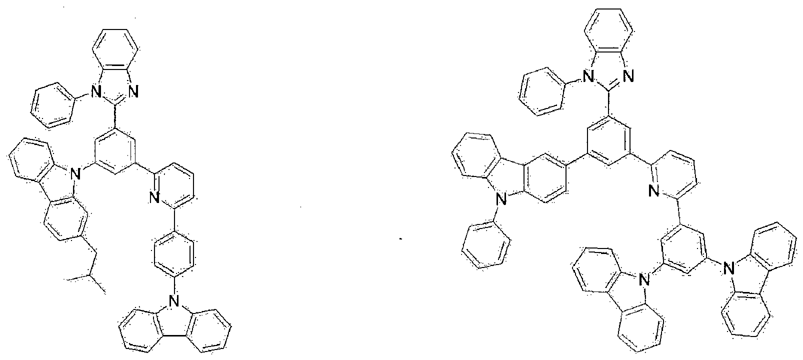

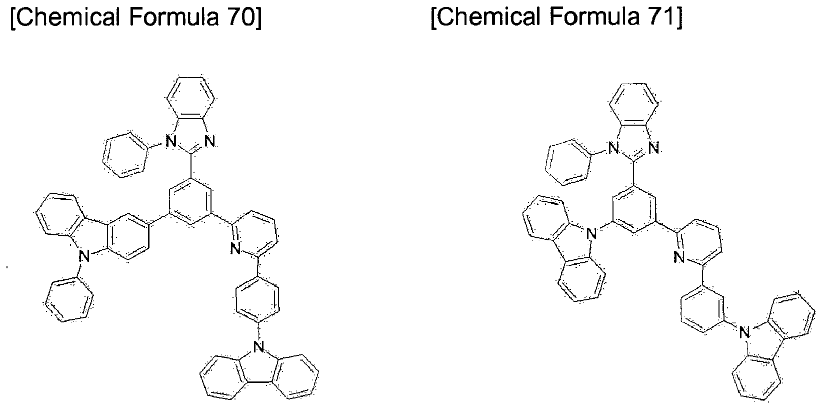

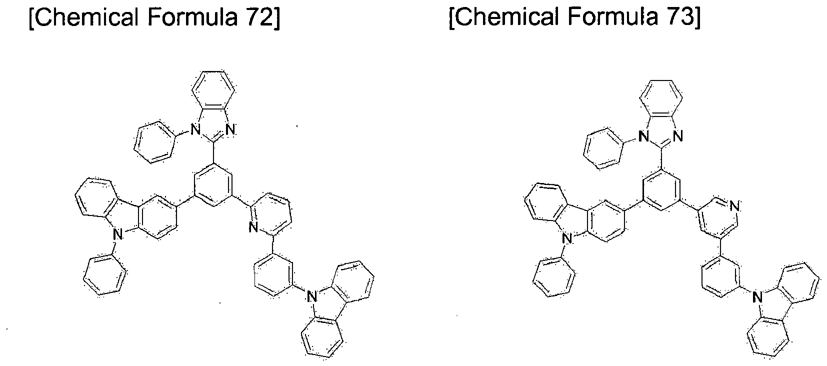

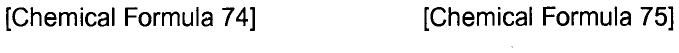

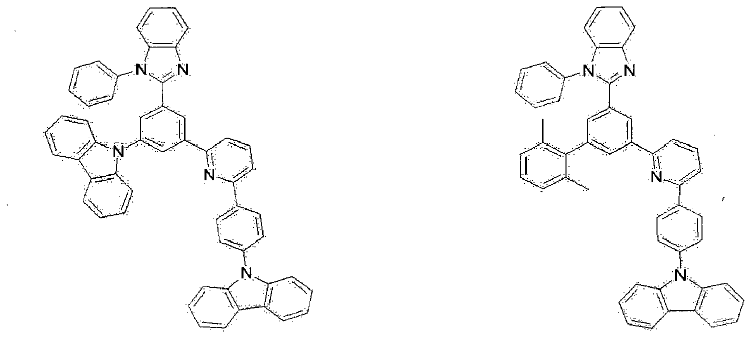

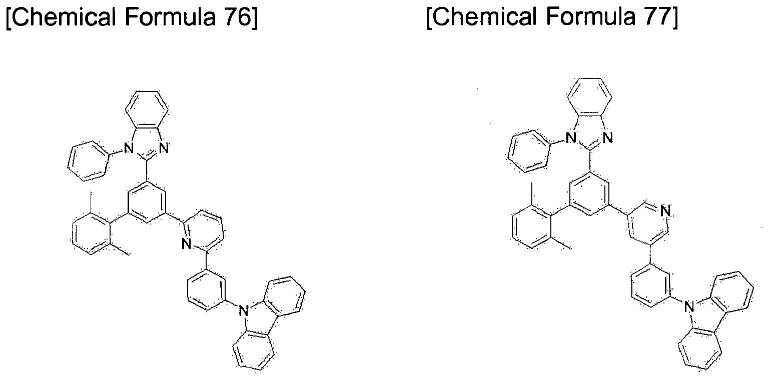

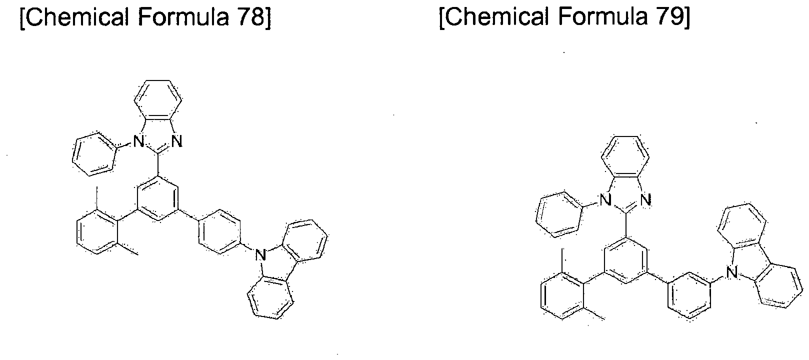

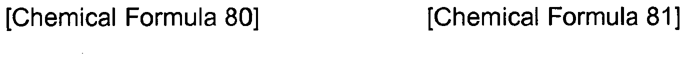

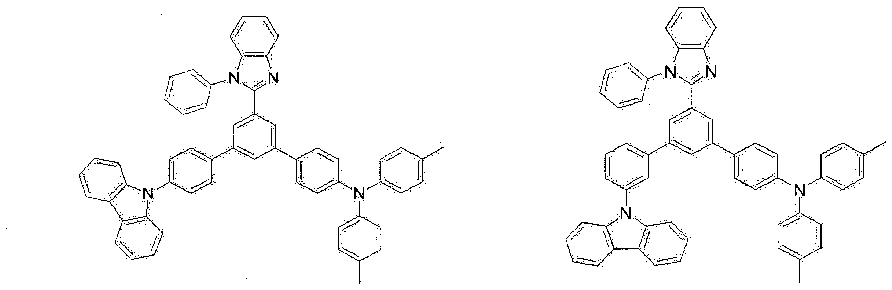

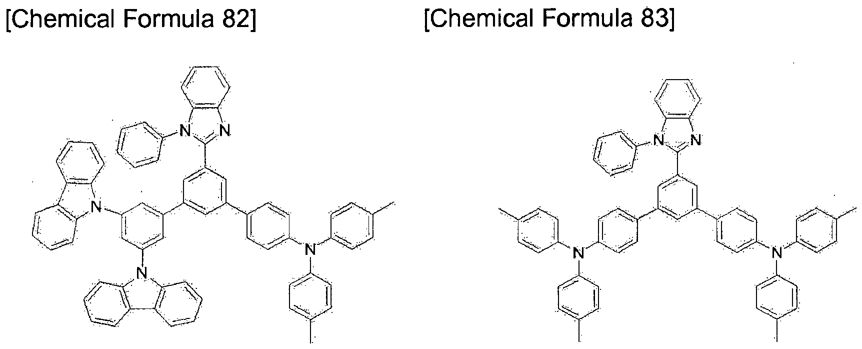

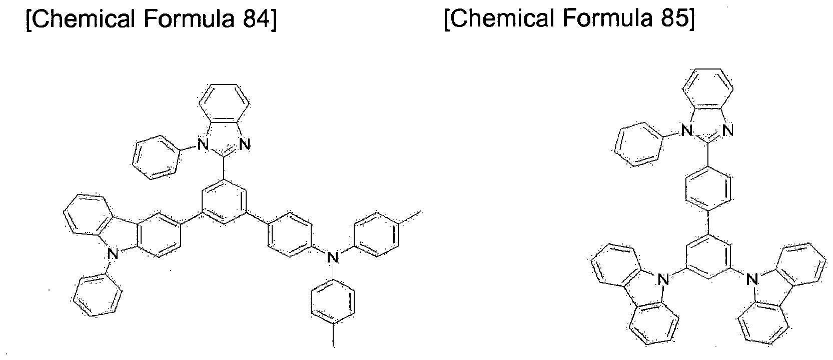

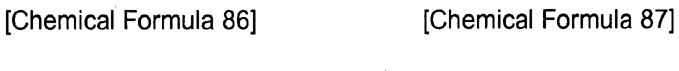

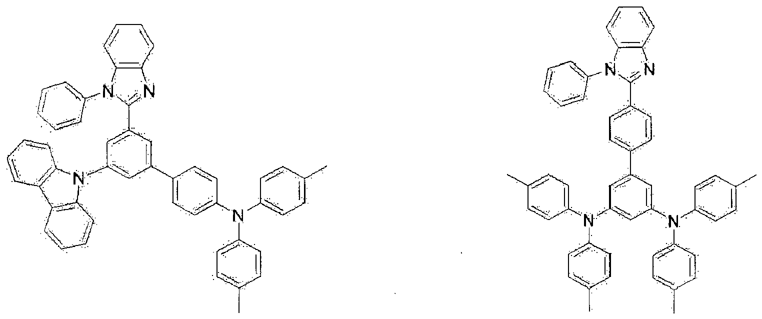

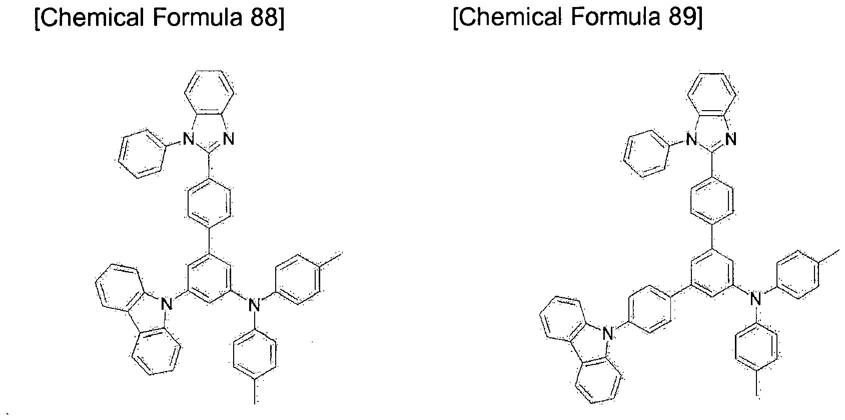

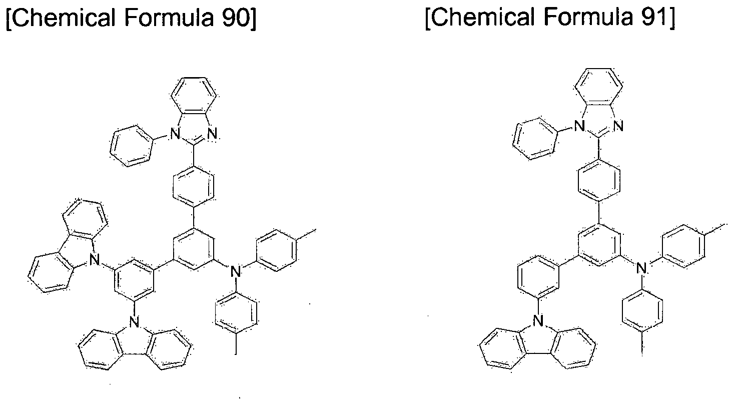

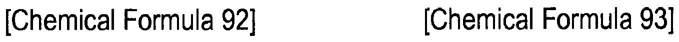

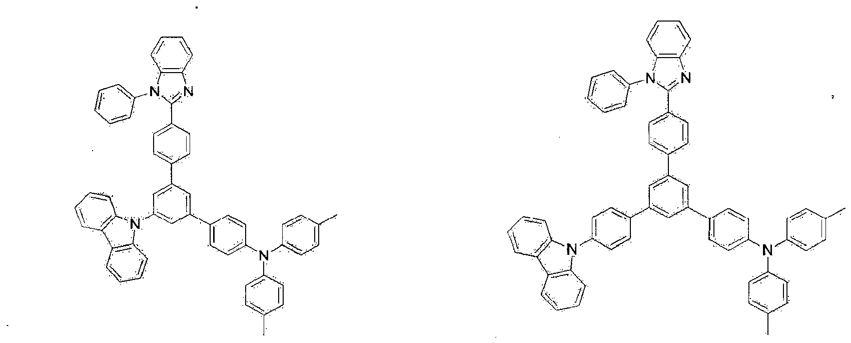

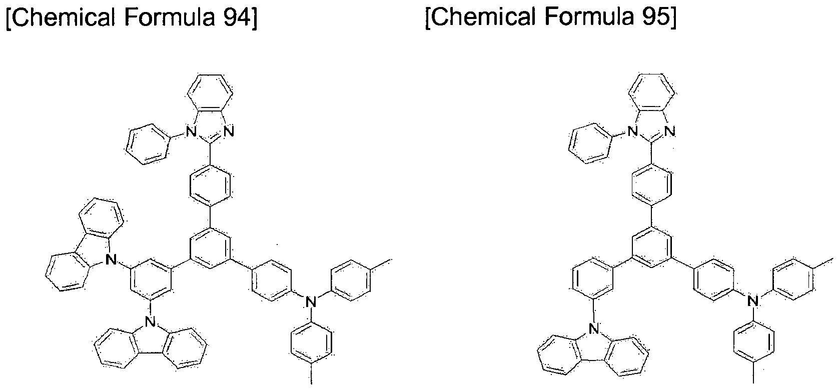

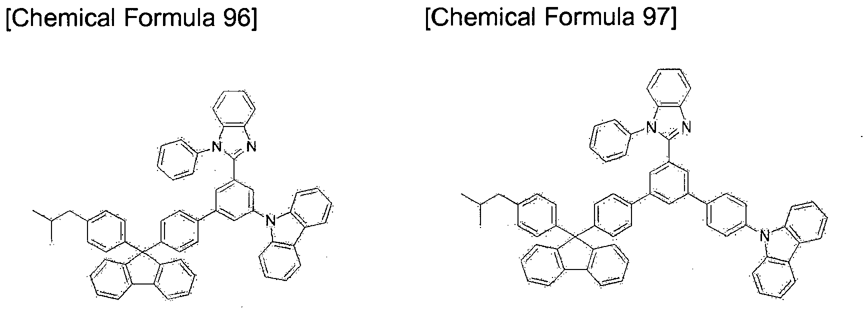

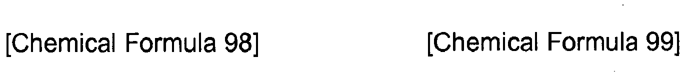

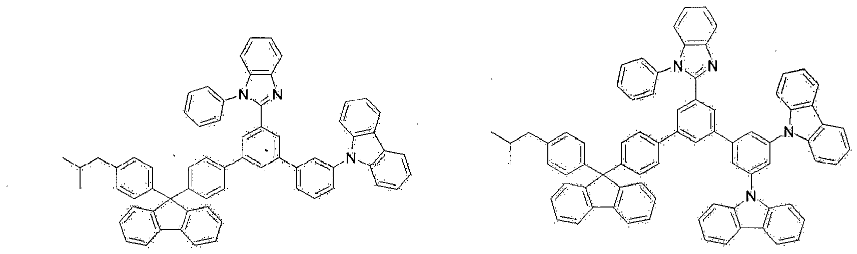

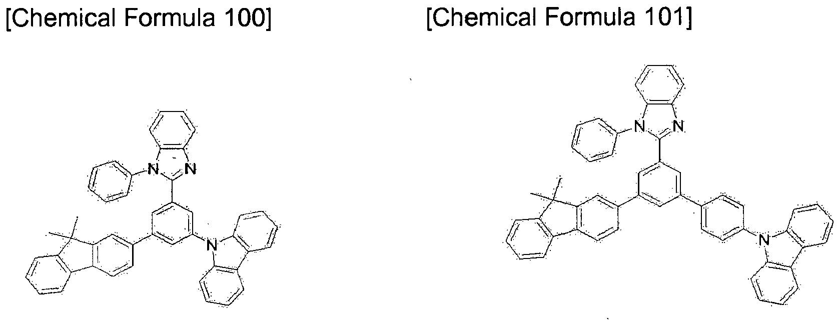

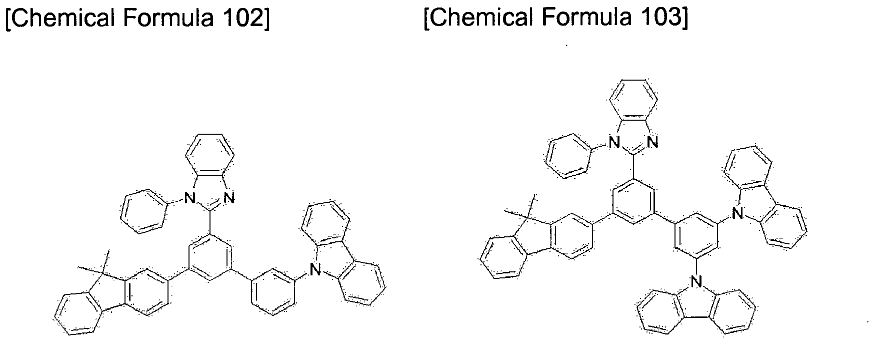

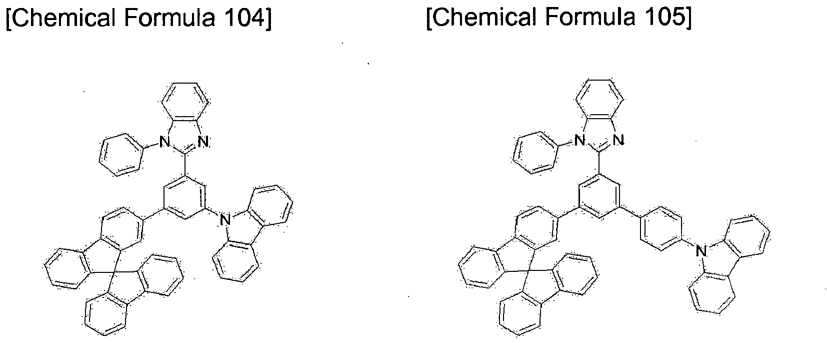

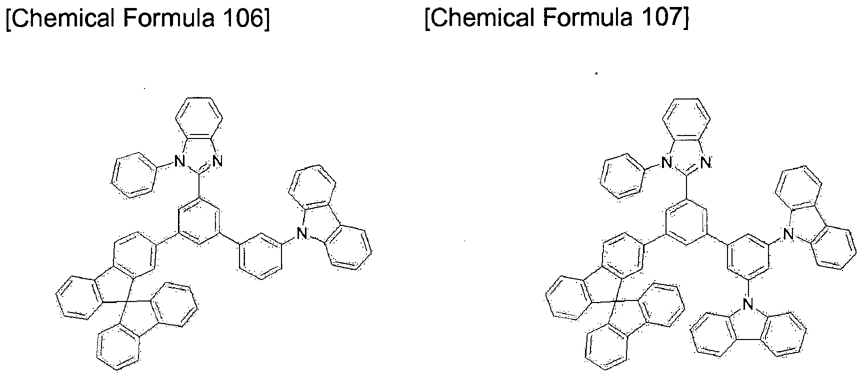

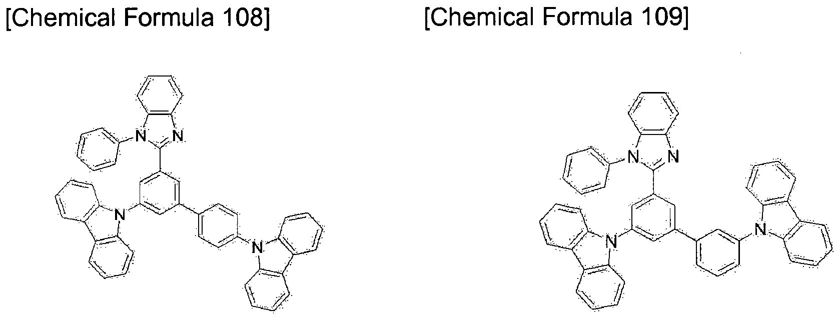

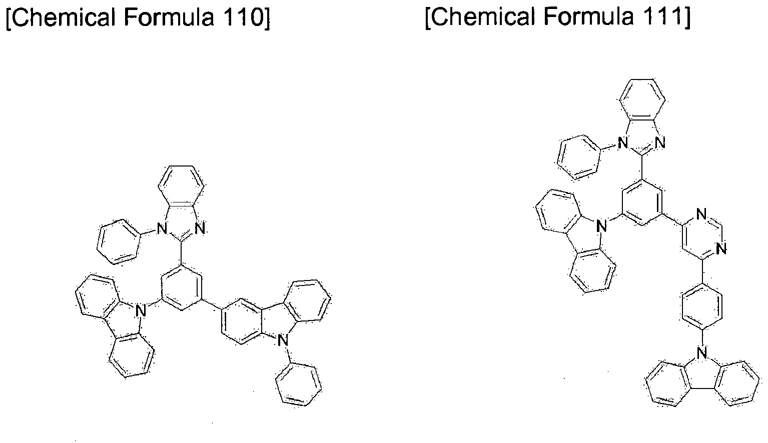

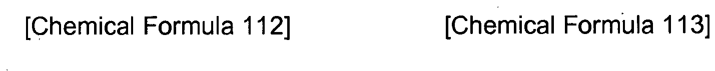

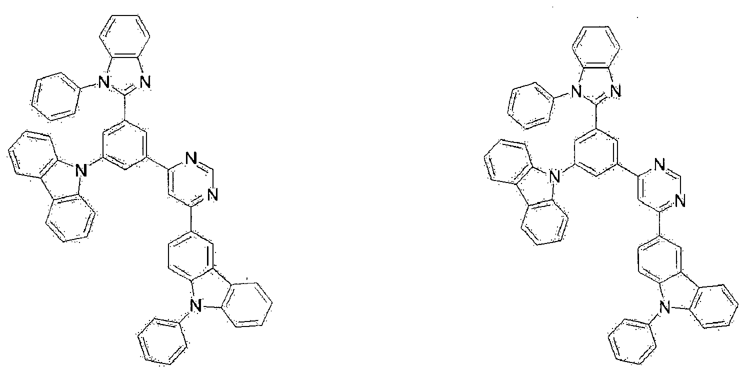

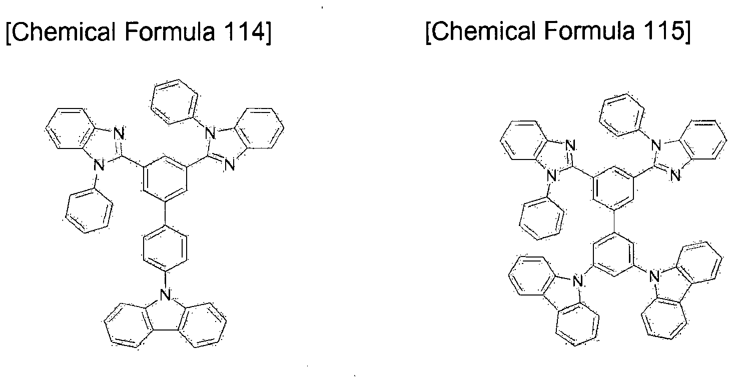

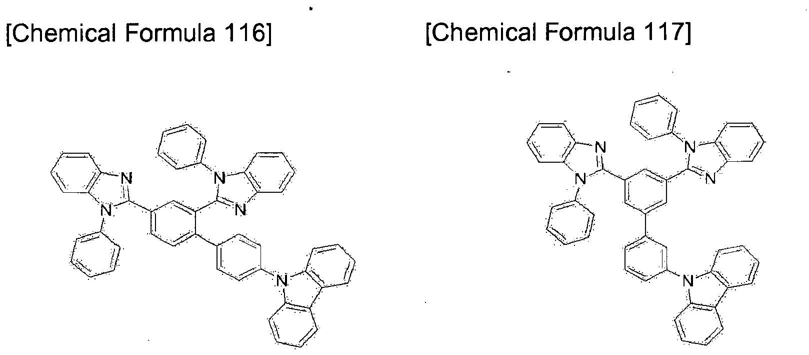

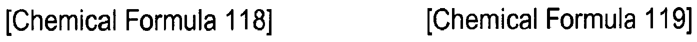

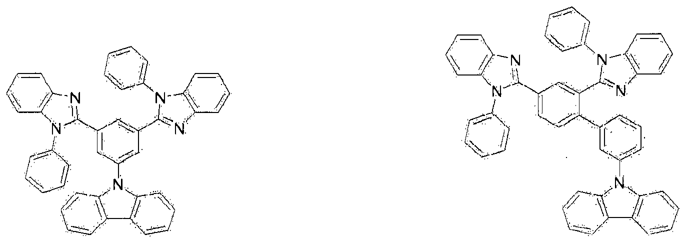

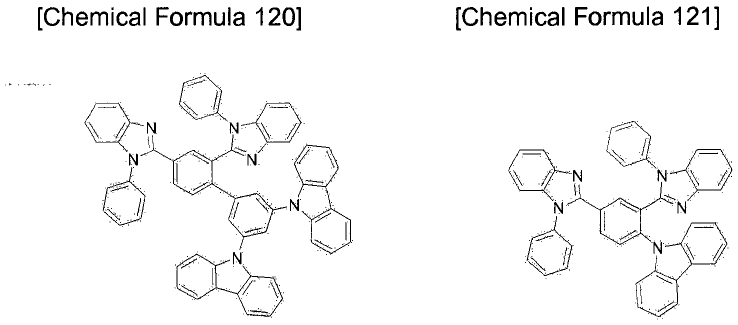

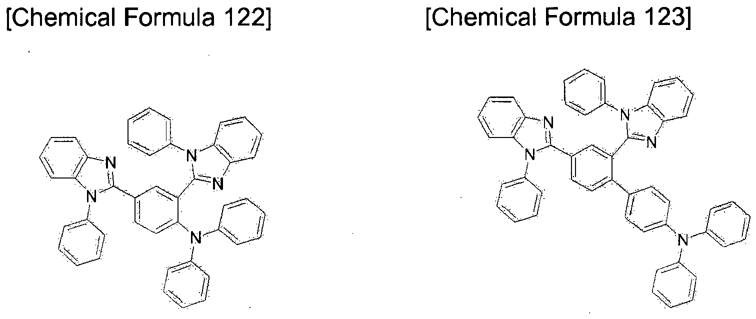

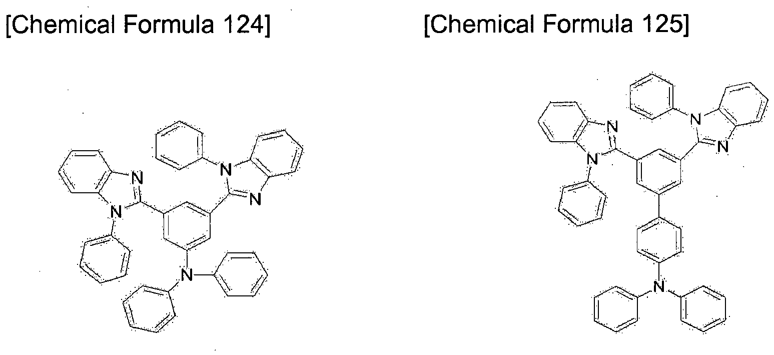

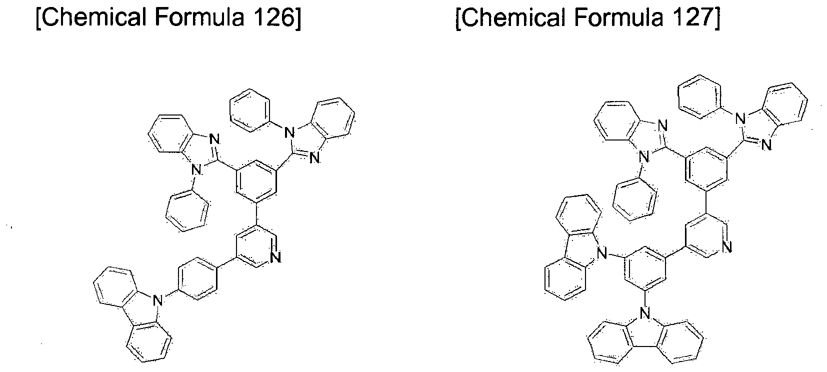

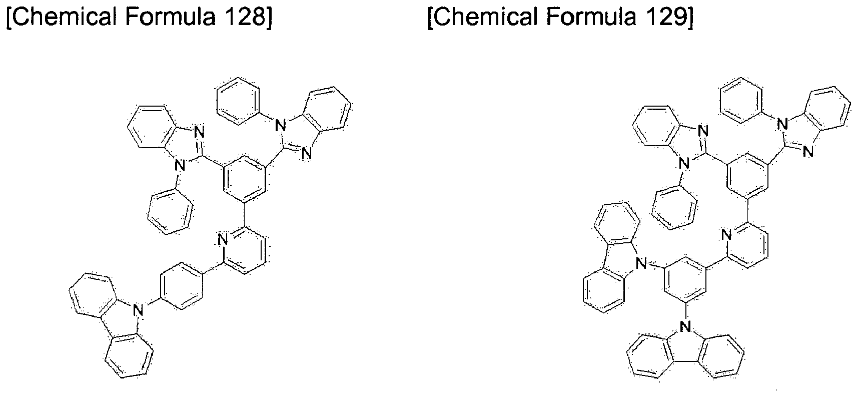

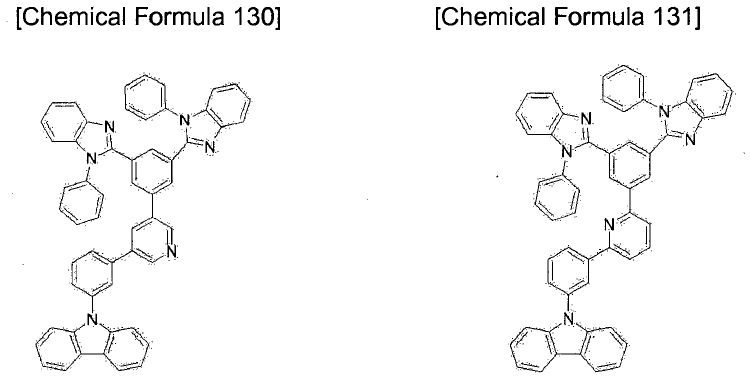

2. Use of the benzimidazole compound according to claim 1 for application to a charge transporting material or a host material for an organic photoelectric device.

3. An organic photoelectric device comprising an anode, a cathode, and at least one organic thin layer disposed between the anode and cathode, wherein at least one organic thin layer comprises the benzimidazole compound according to claim 1.

4. The organic photoelectric device of claim 3, wherein the organic thin layer is an emission layer.

5. The organic photoelectric device of claim 3, wherein the organic thin layer is selected from the group consisting of an electron injection layer (EIL), an electron transport layer (ETL), a hole blocking layer, and combinations thereof.

6. A display element comprising the organic photoelectric device according to claim 3.

## Patentansprüche

1. Benzimidazolverbindung der folgenden chemischen Formel 35 bis 131:

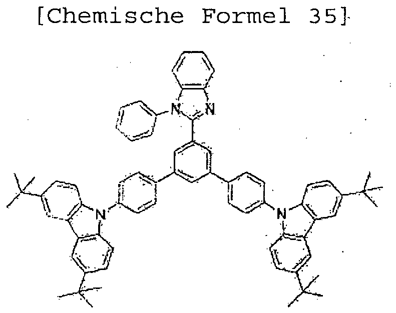

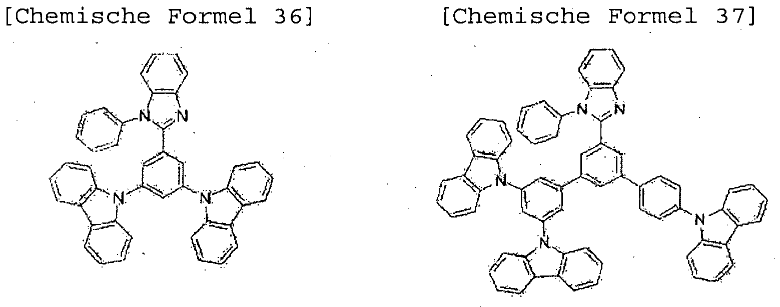

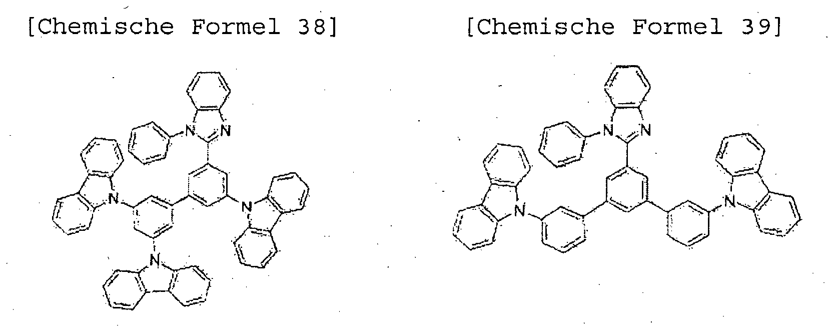

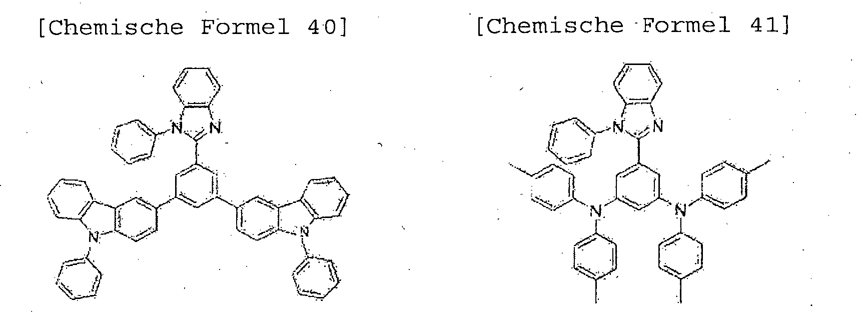

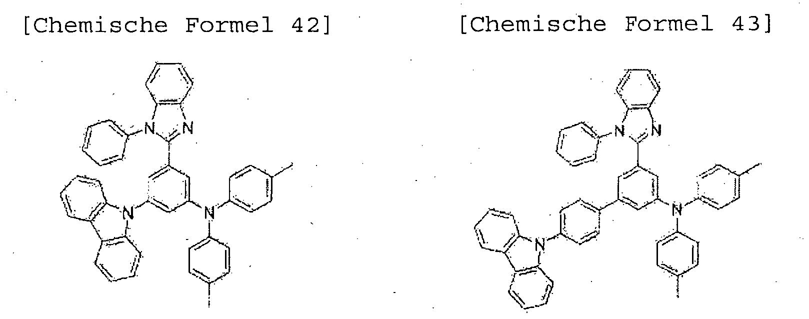

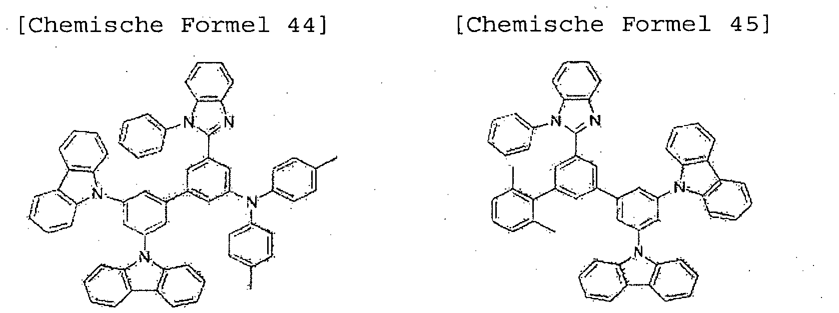

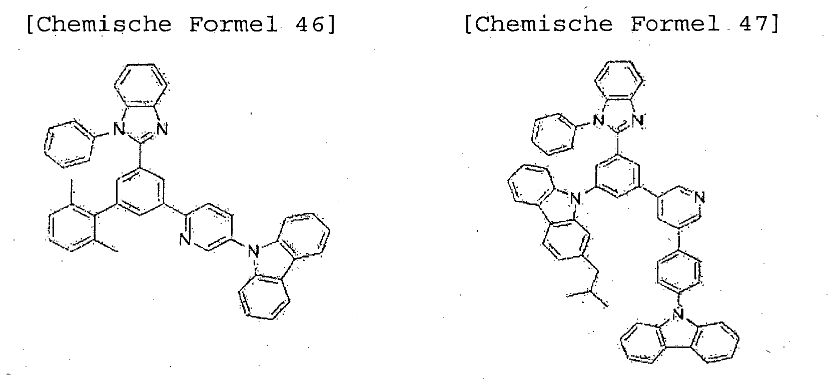

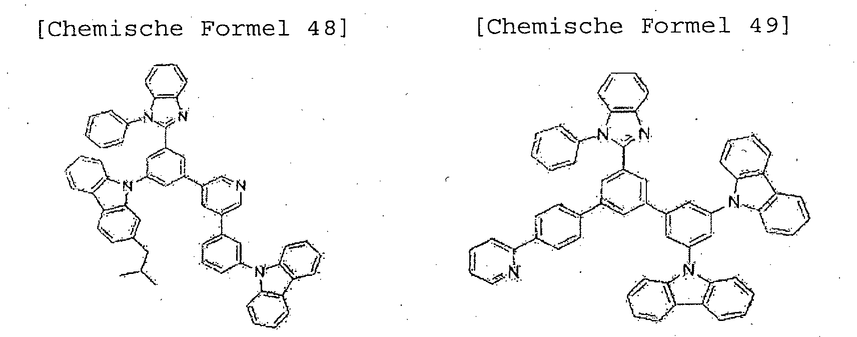

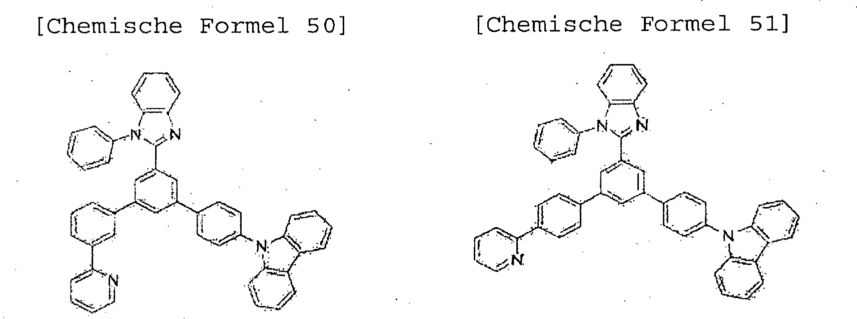

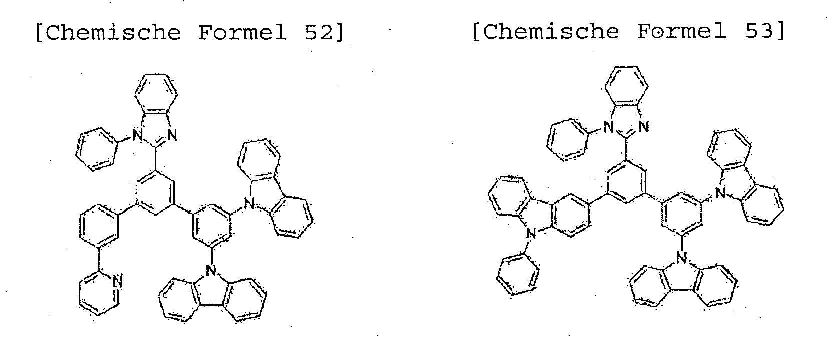

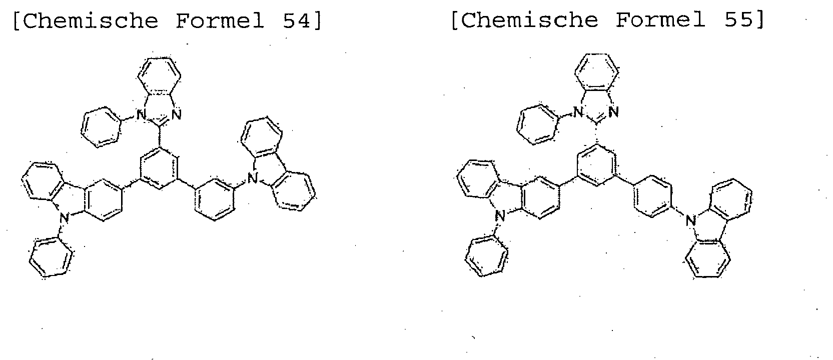

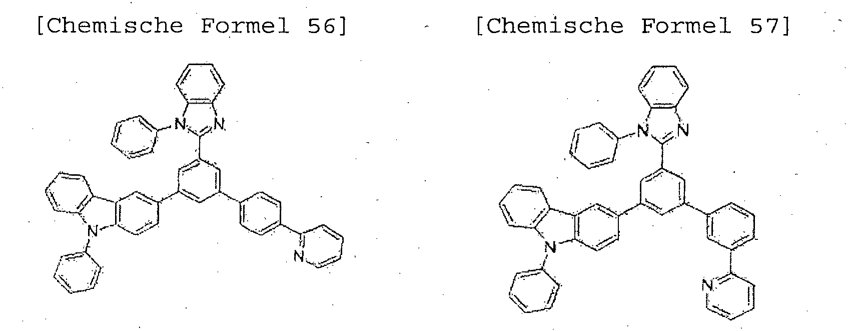

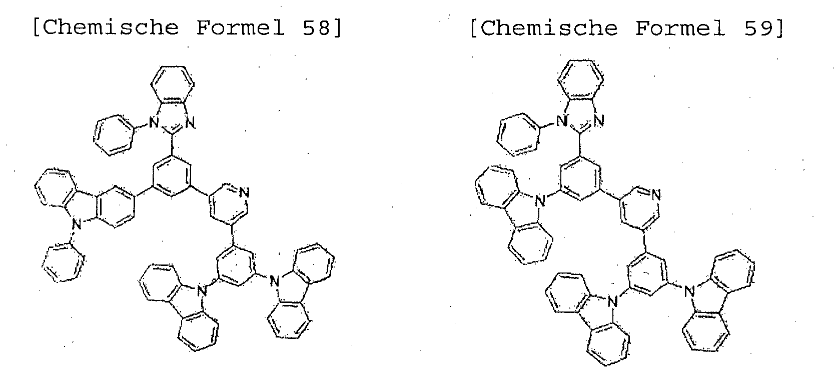

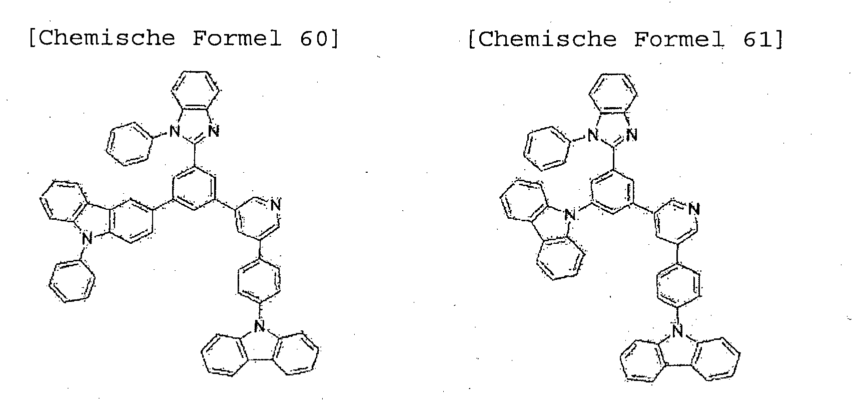

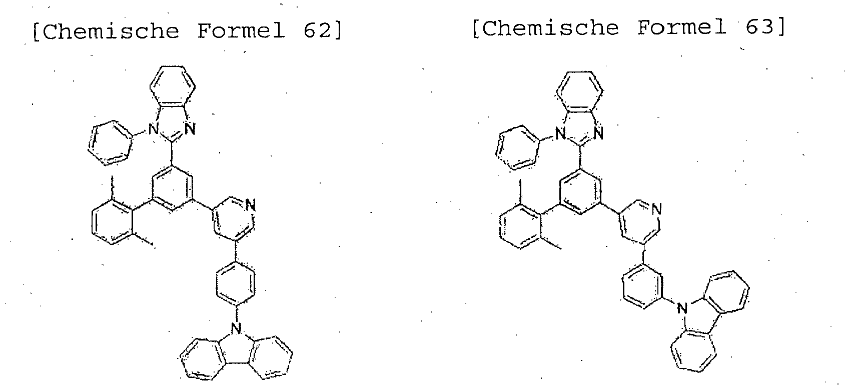

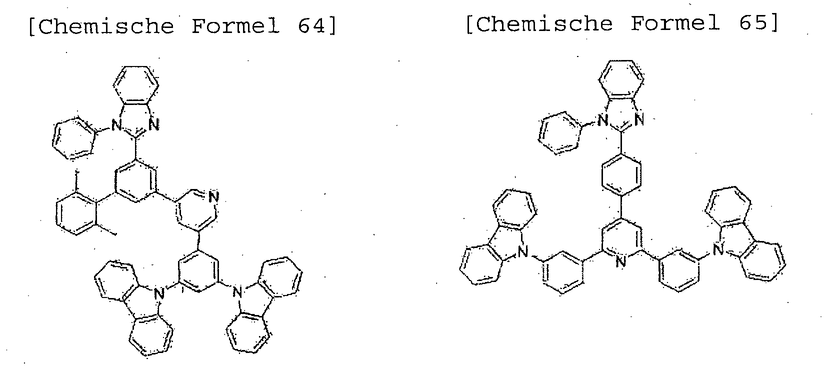

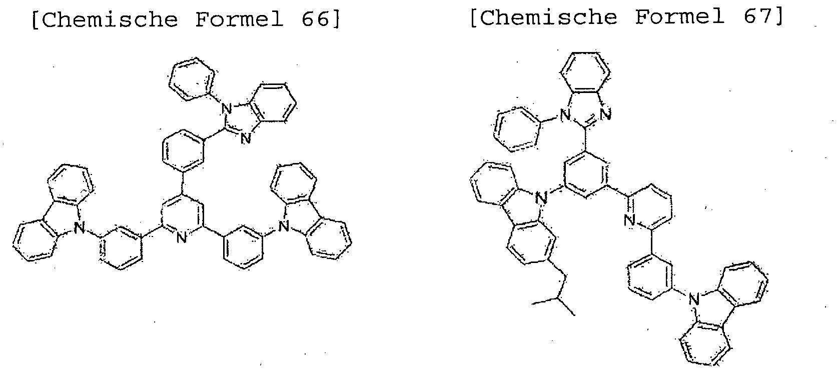

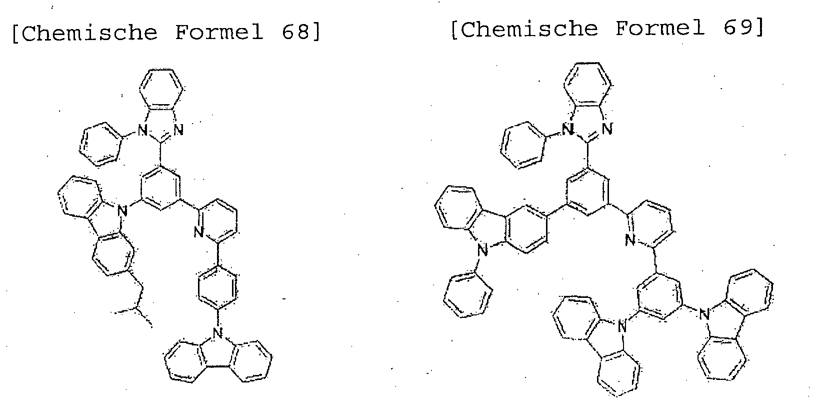

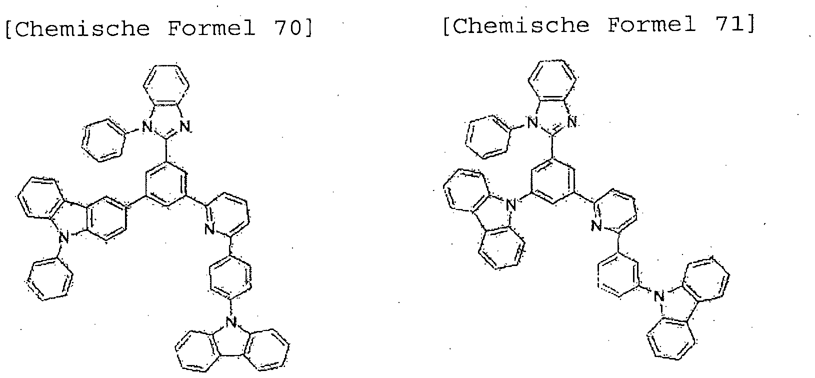

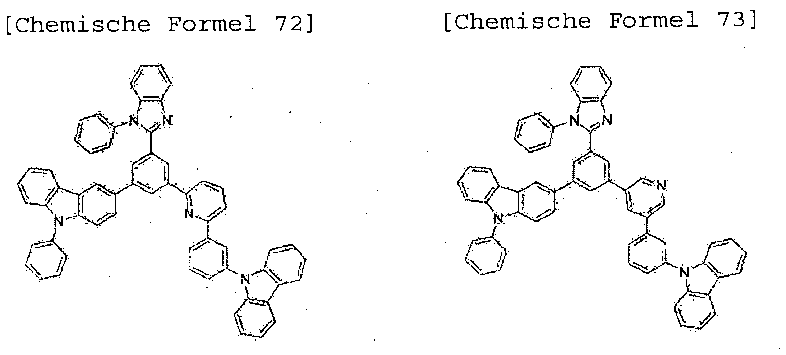

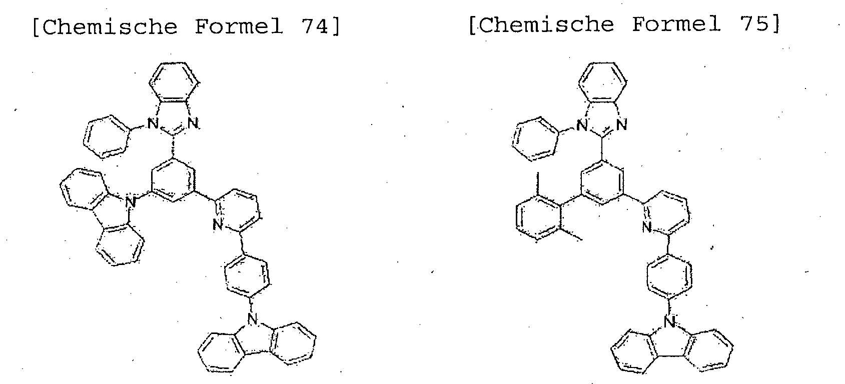

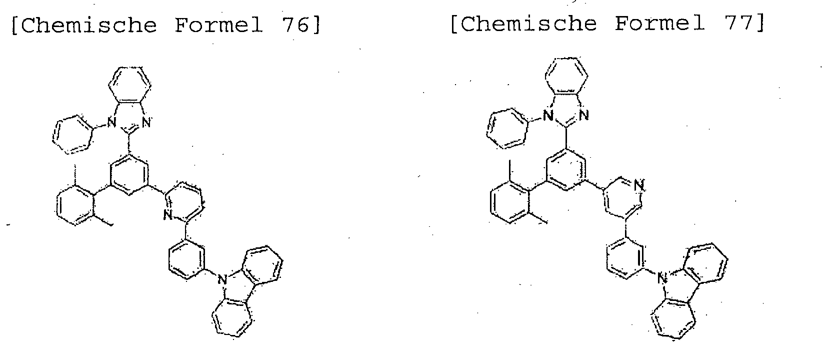

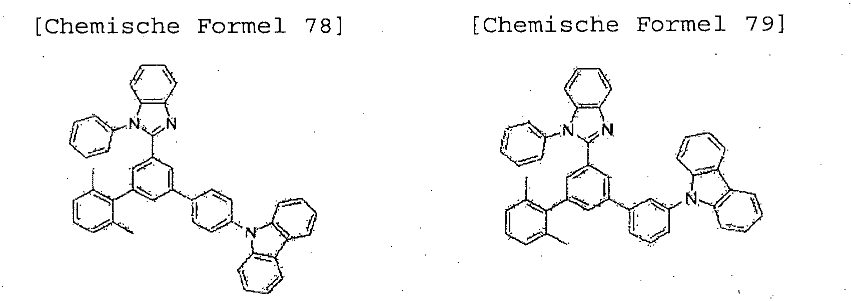

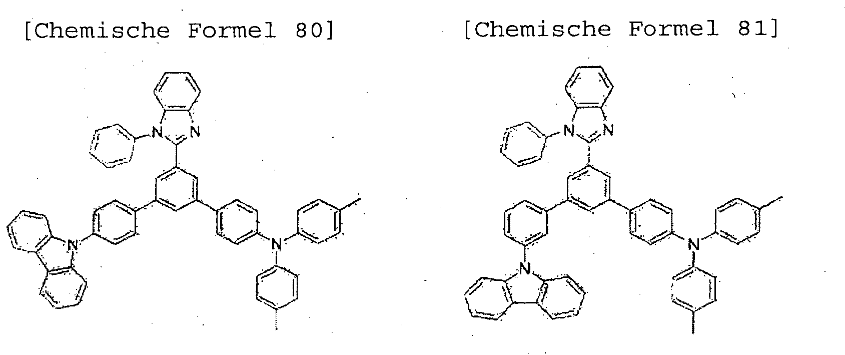

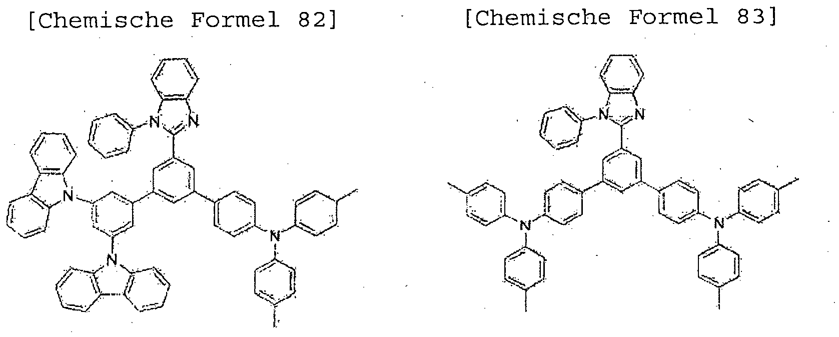

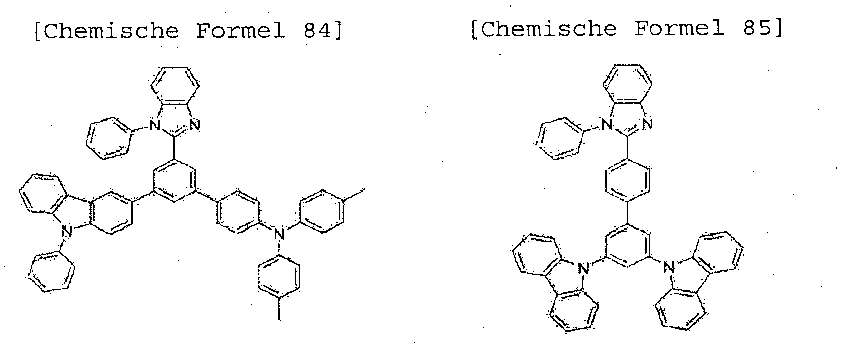

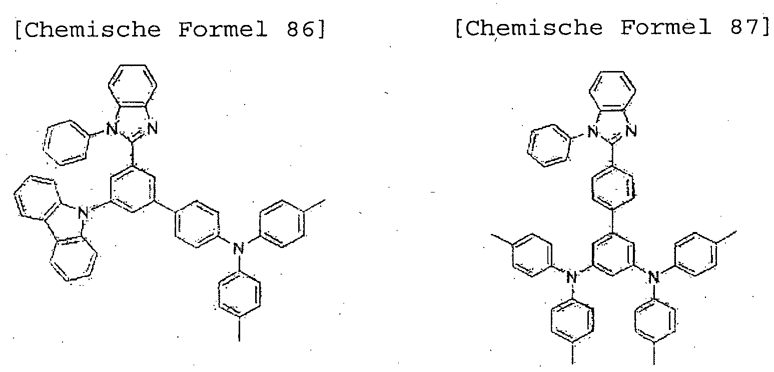

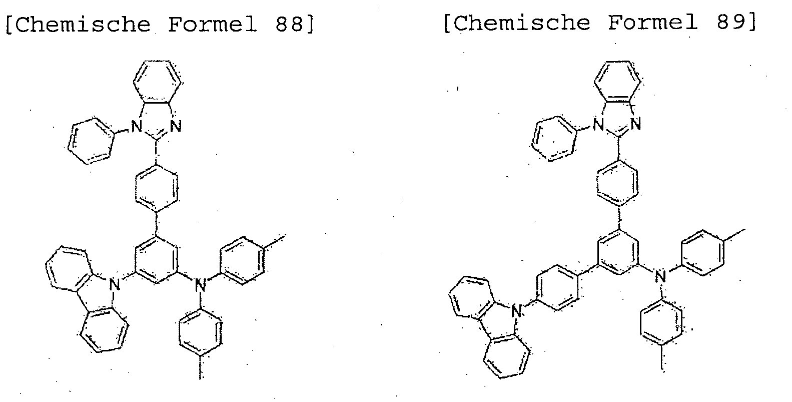

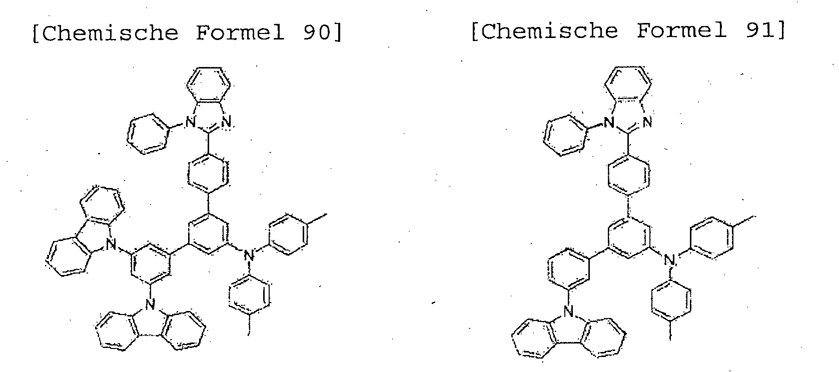

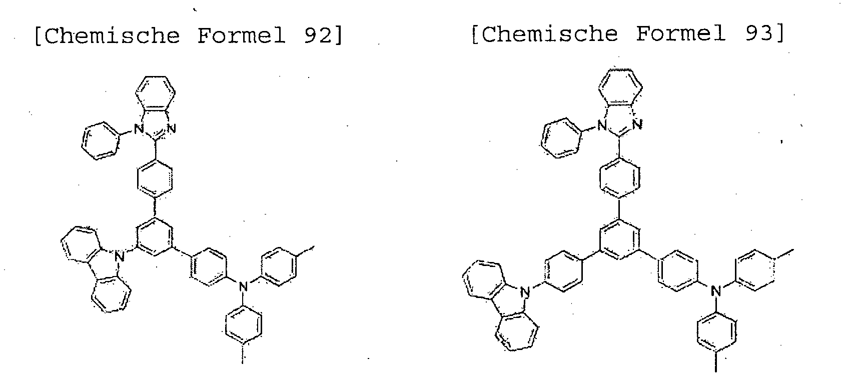

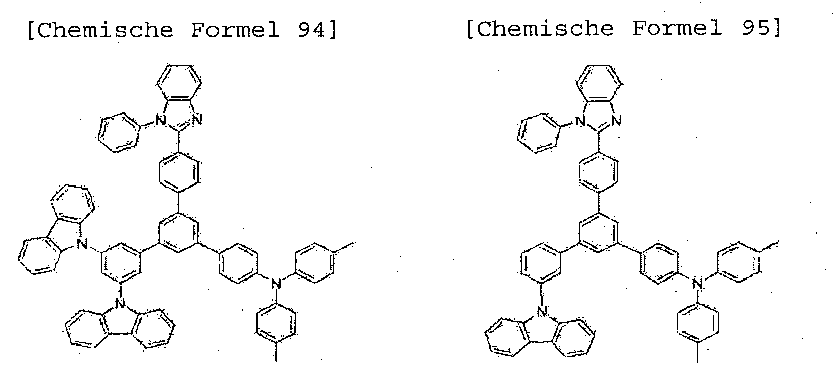

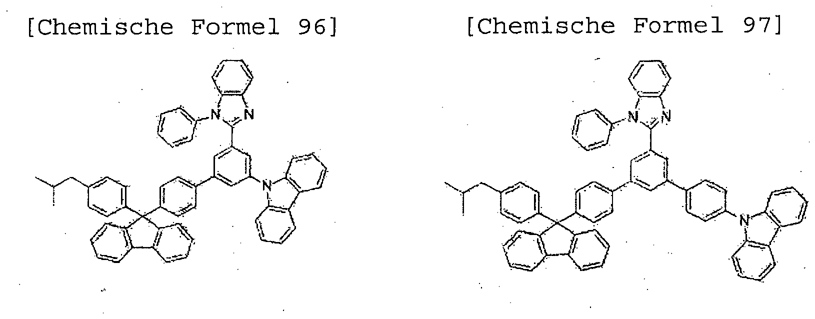

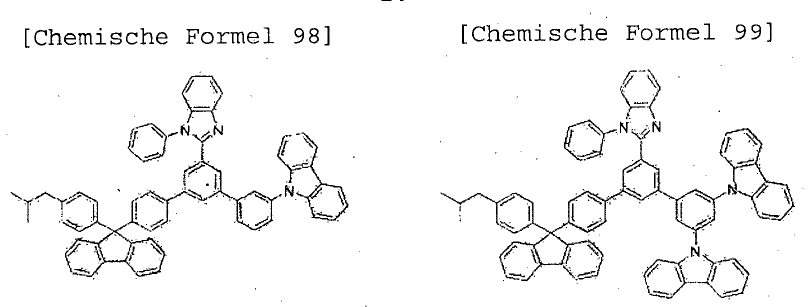

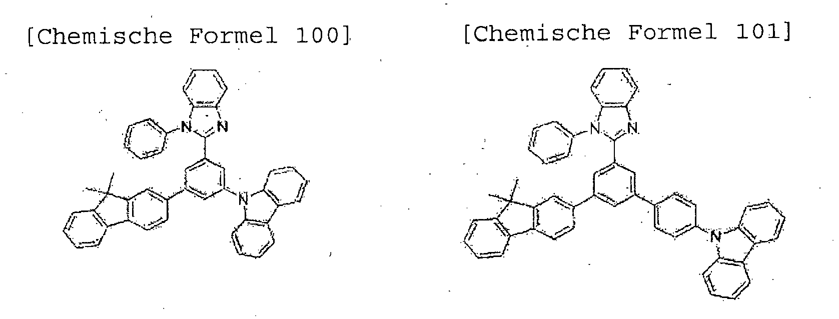

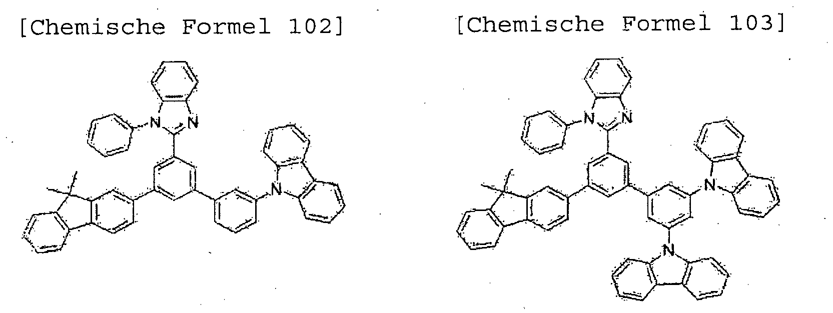

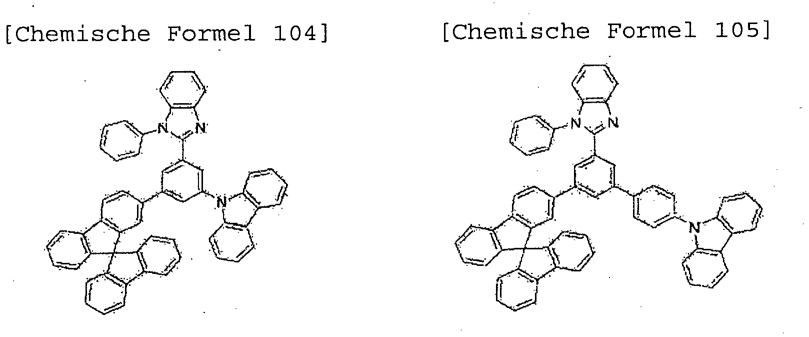

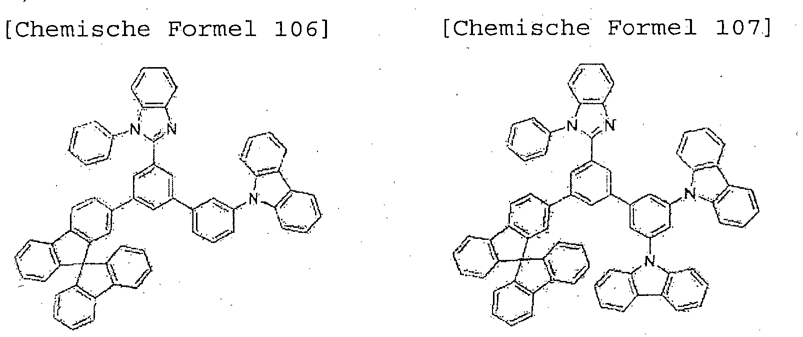

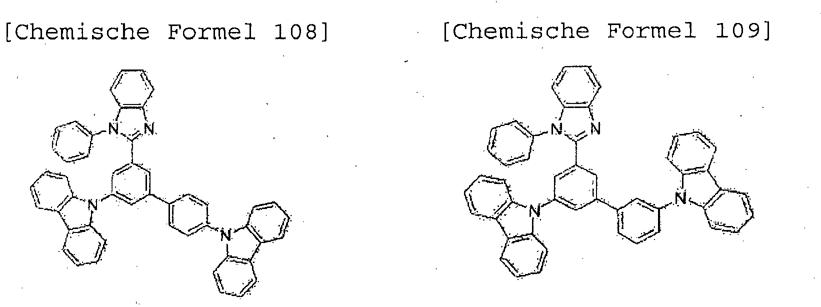

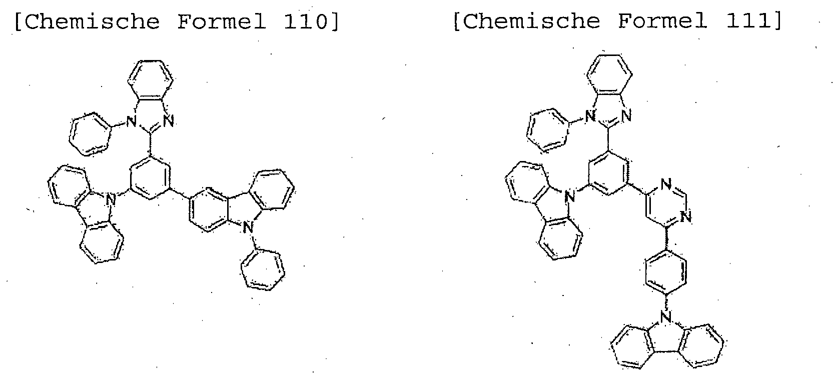

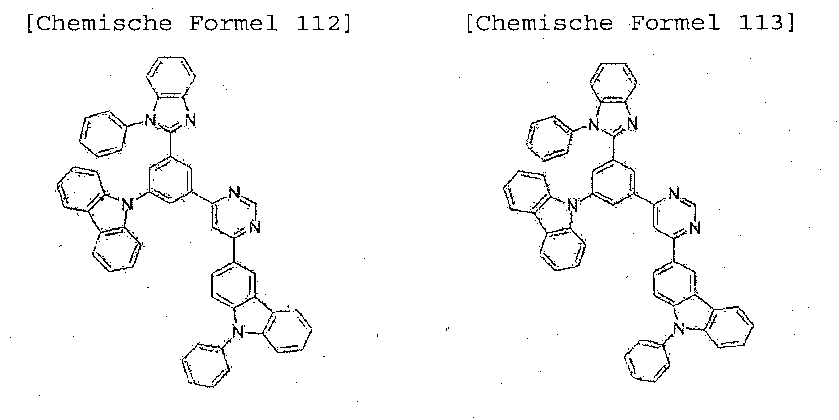

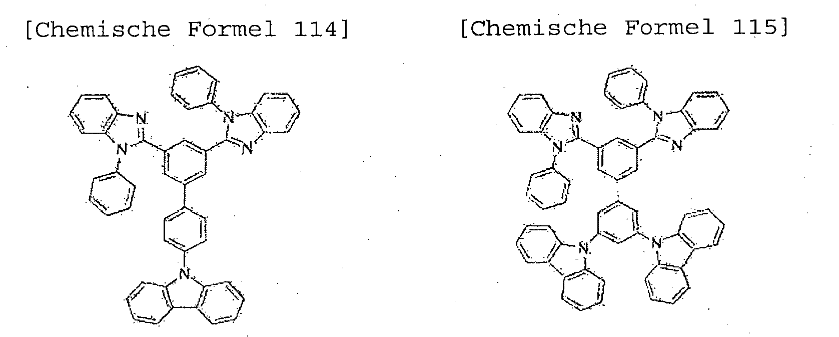

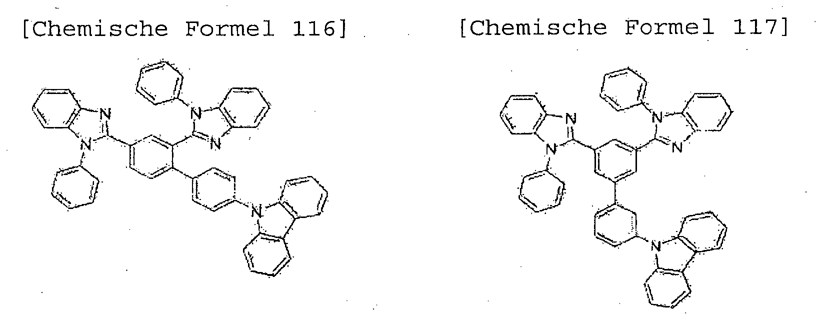

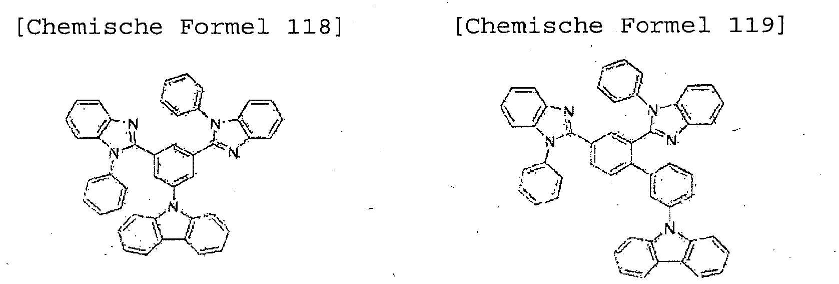

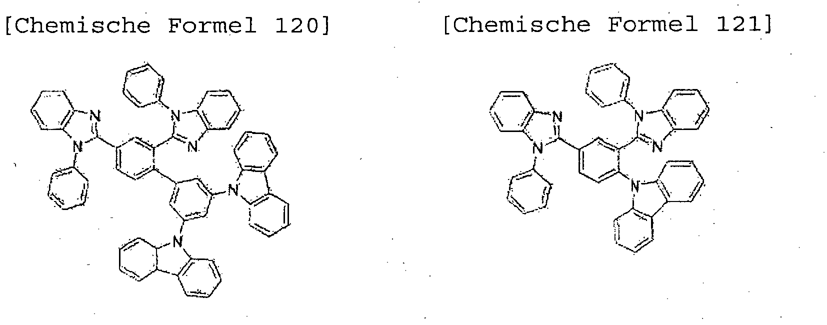

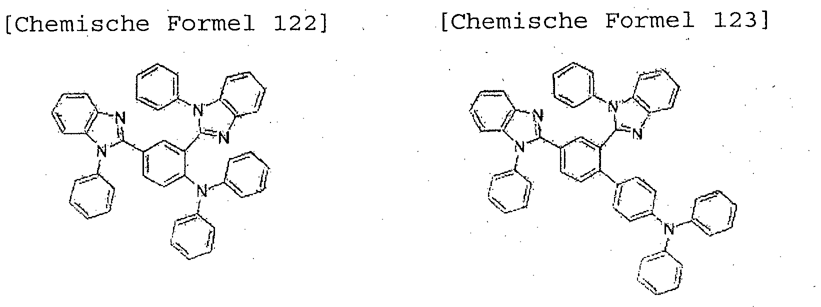

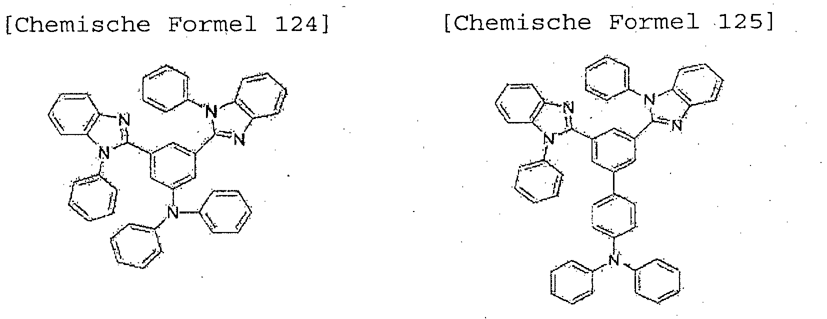

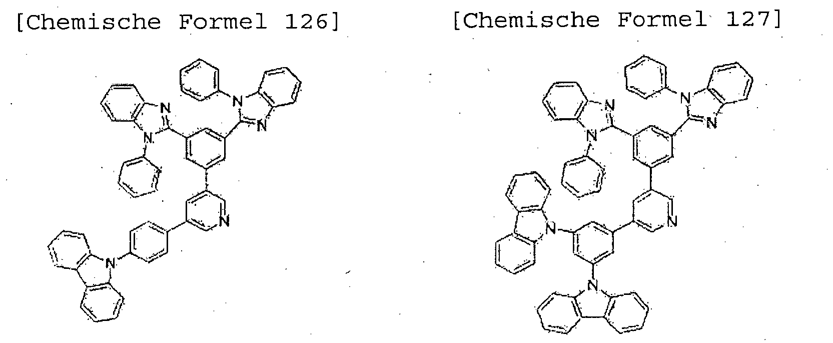

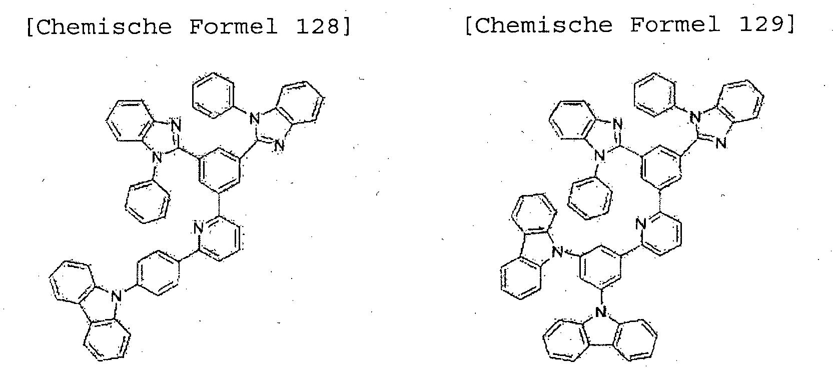

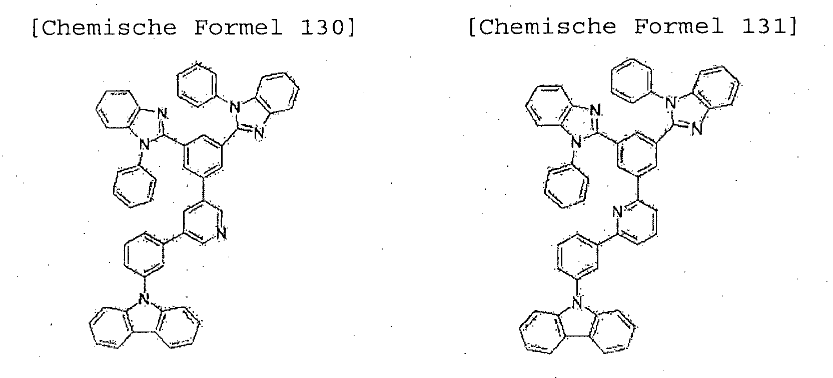

2. Verwendung der Benzimidazolverbindung nach Anspruch 1 zur Anwendung als Ladungstransportmaterial oder Wirtsmaterial für eine organische photoelektrische Vorrichtung.

3. Organische photoelektrische Vorrichtung, umfassend eine Anode, eine Kathode und mindestens eine organische dünne Schicht, die zwischen der Anode und Kathode angeordnet ist, wobei die mindestens eine organische dünne Schicht die Benzimidazolverbindung nach Anspruch 1 umfasst.

4. Organische photoelektrische Vorrichtung nach Anspruch 3, wobei es sich bei der organischen dünnen Schicht um eine Emissionsschicht handelt.

5. Organische photoelektrische Vorrichtung nach Anspruch 3, wobei die organische dünne Schicht aus der Gruppe bestehend aus einer Elektroneninjektionsschicht (Electron Injection Layer, EIL), einer Elektronentransportschicht (Electron Transport Layer, ETL), einer Lochblockierschicht und Kombinationen davon ausgewählt ist.

6. Anzeigeelement, umfassend die organische photoelektrische Vorrichtung nach Anspruch 3.

## Revendications

1. Composé de benzimidazole représenté par les formules chimiques 35 à 131 suivantes :

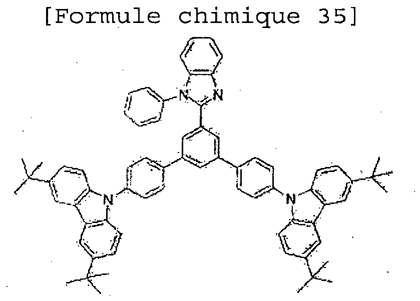

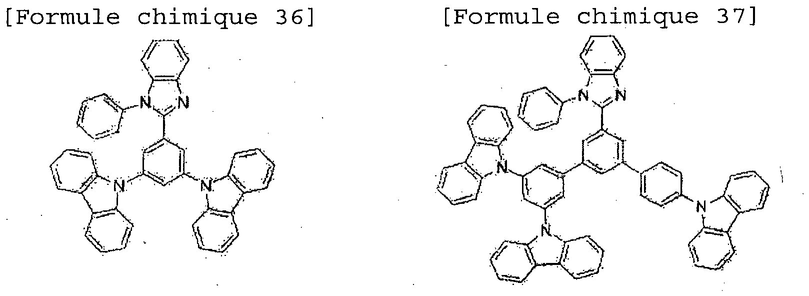

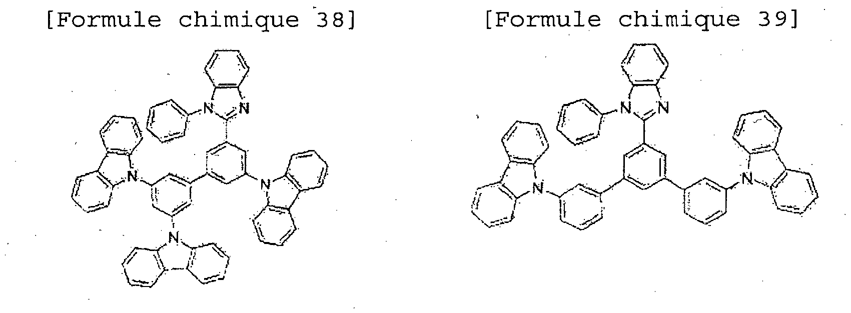

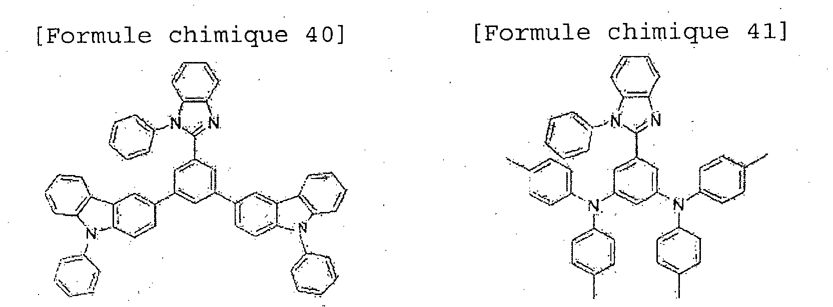

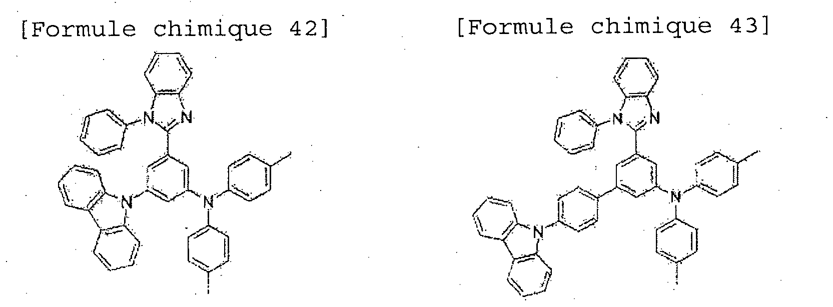

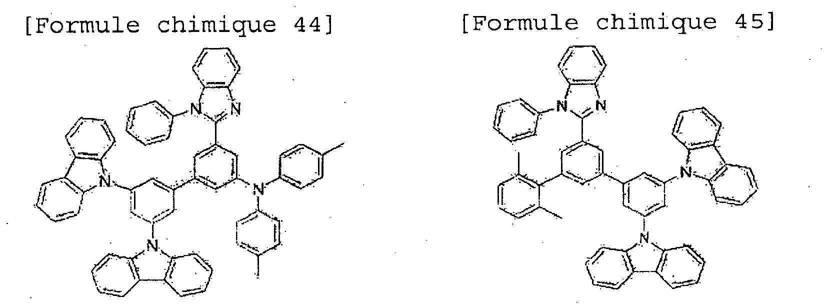

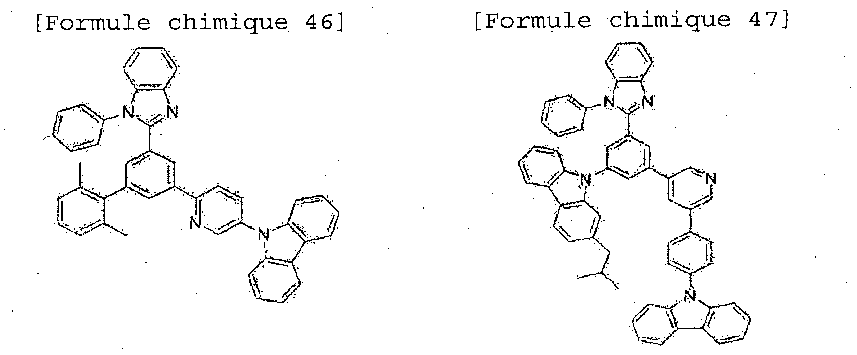

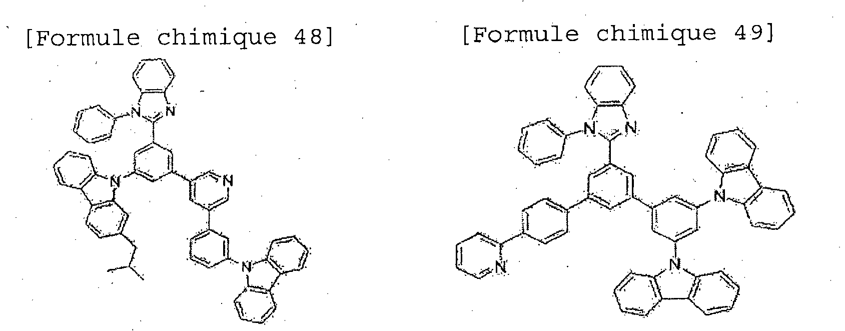

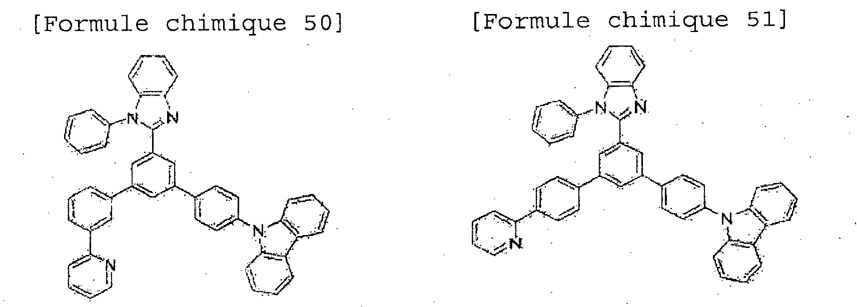

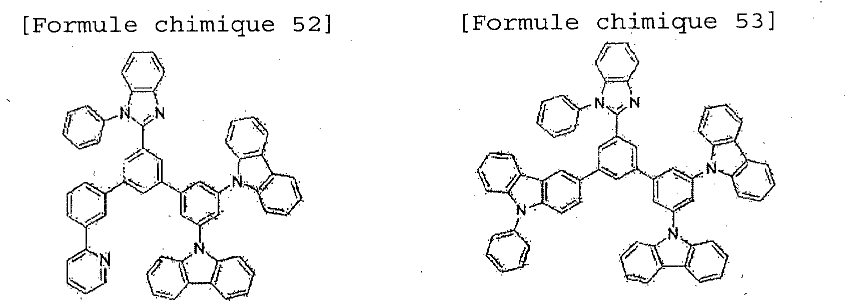

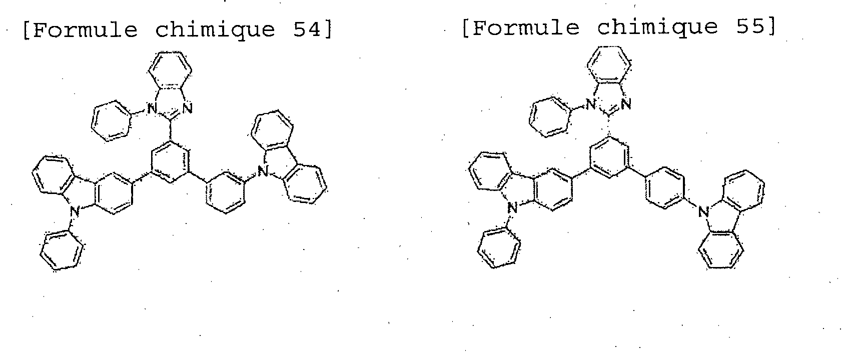

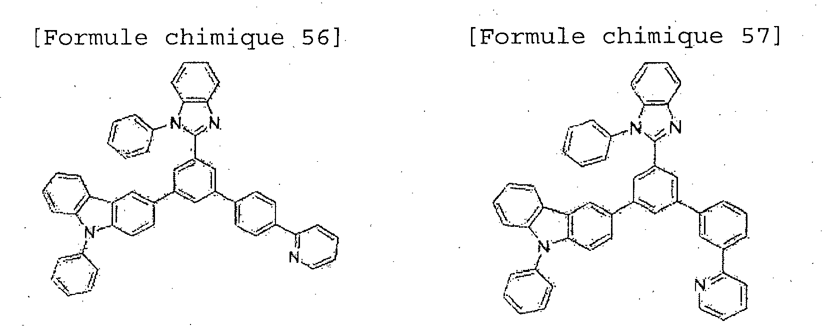

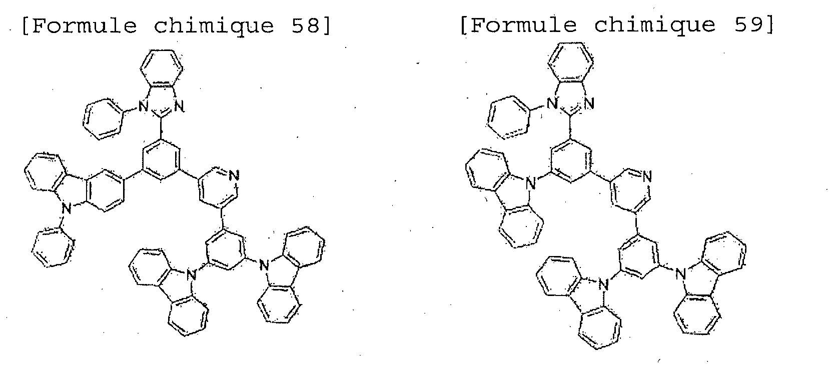

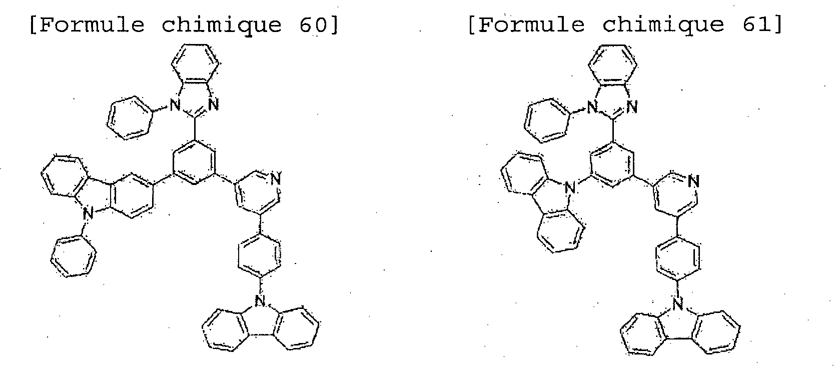

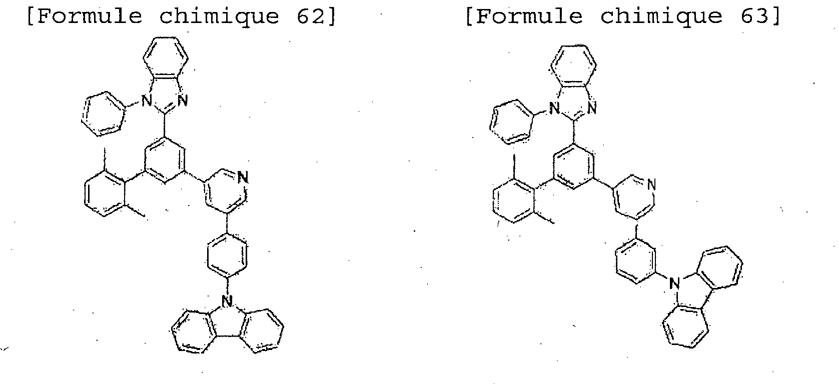

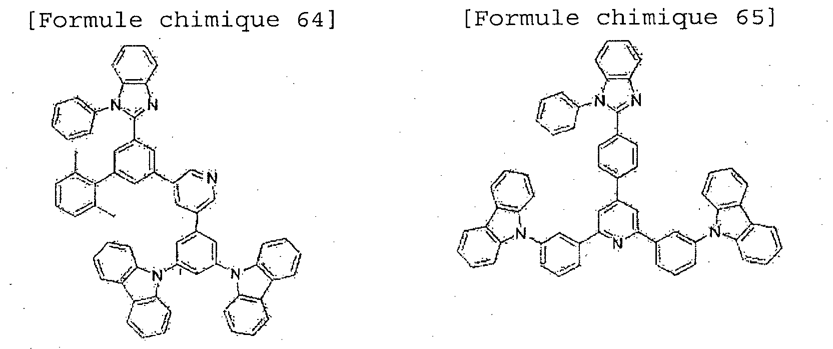

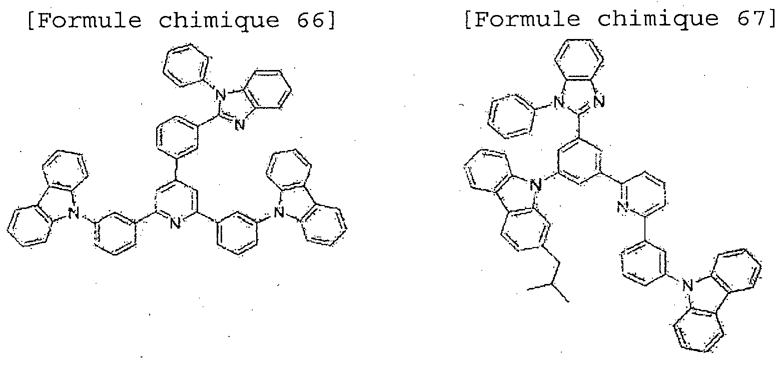

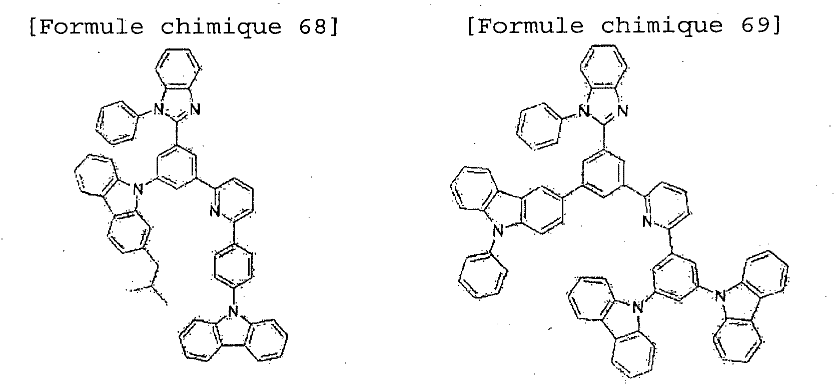

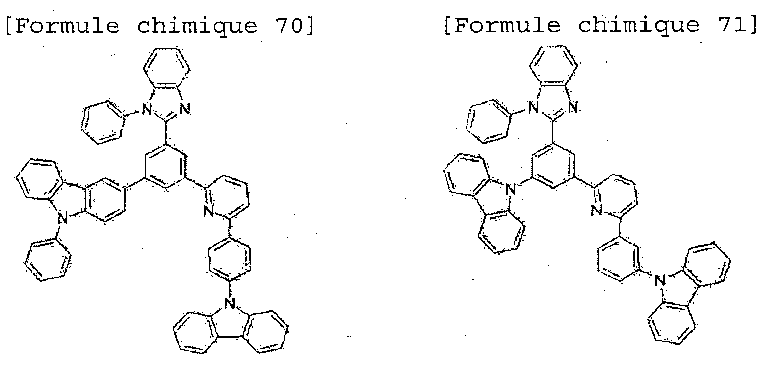

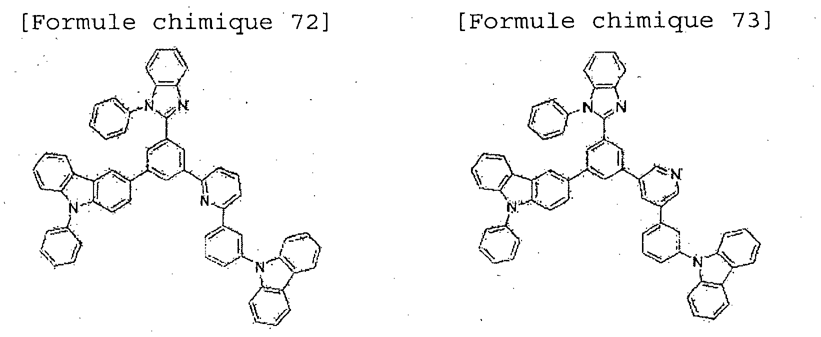

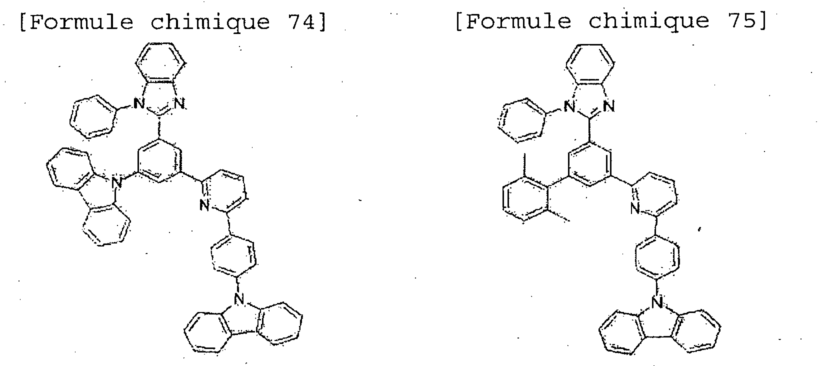

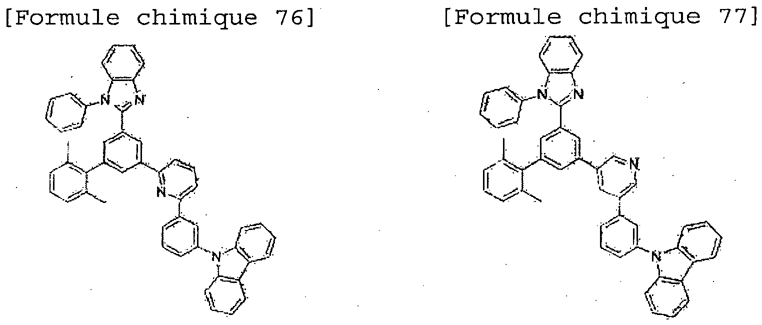

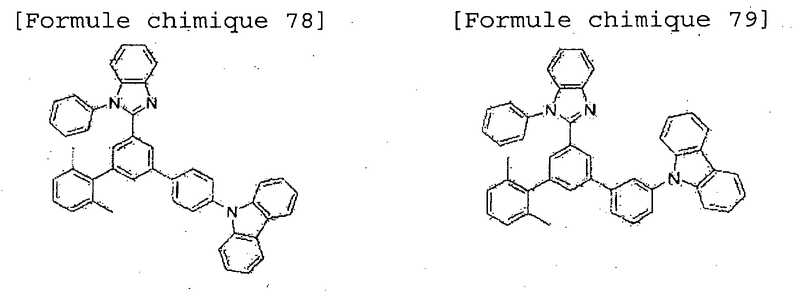

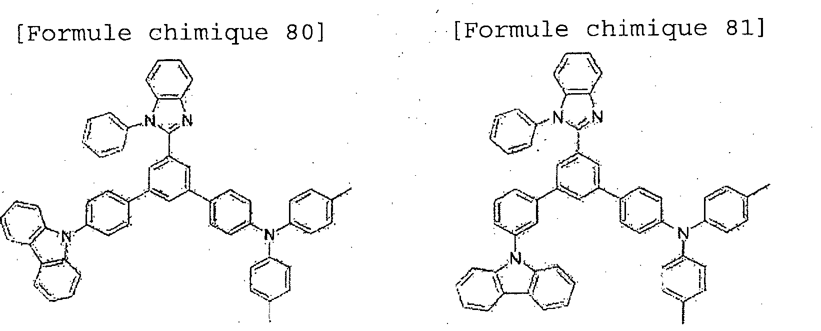

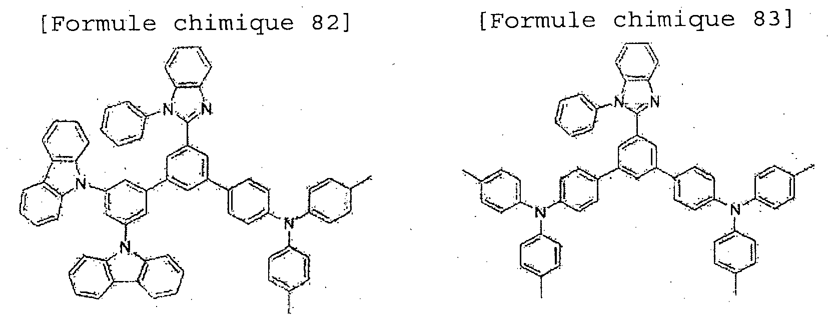

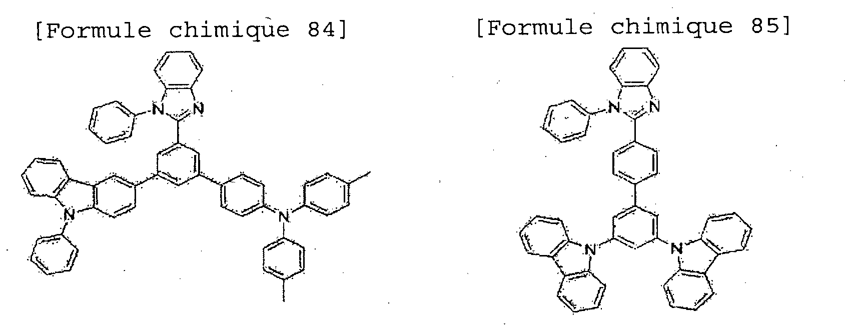

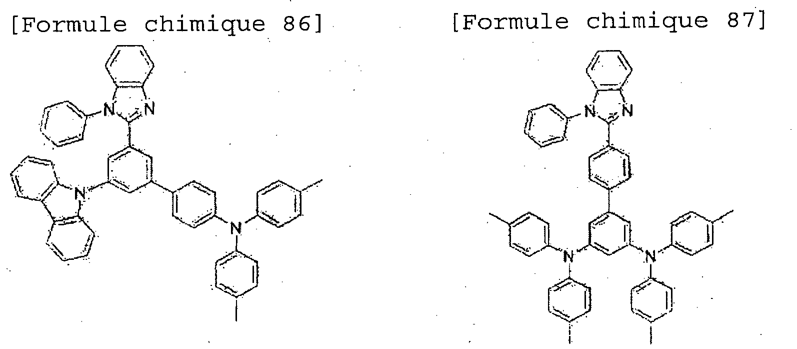

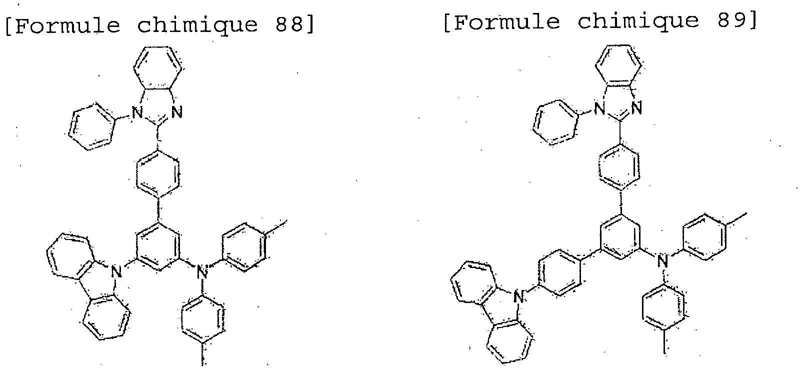

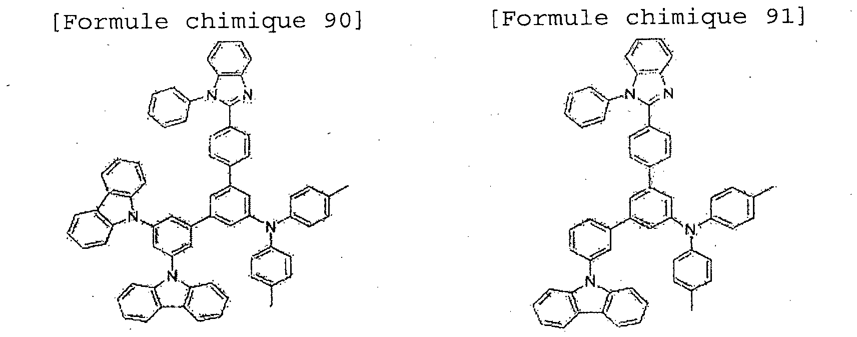

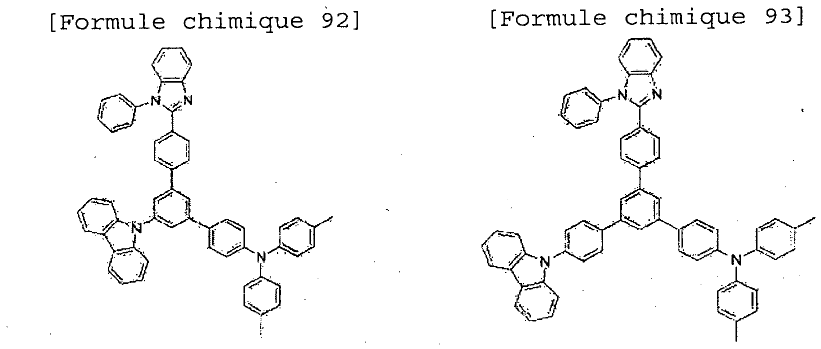

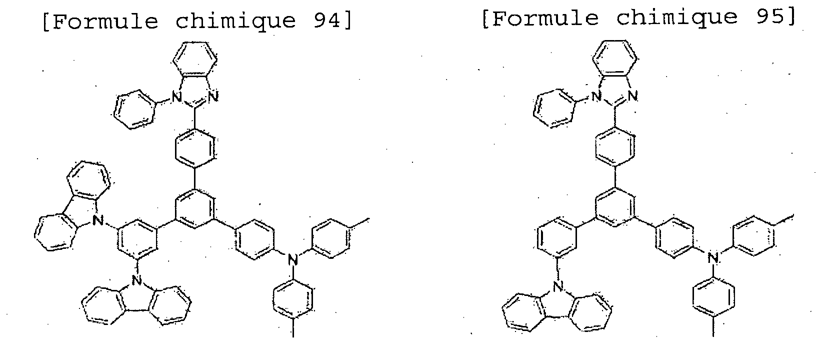

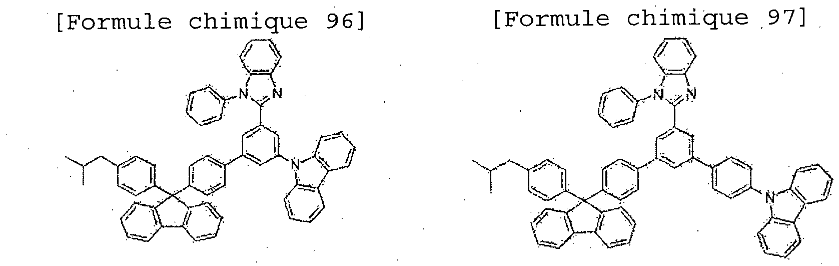

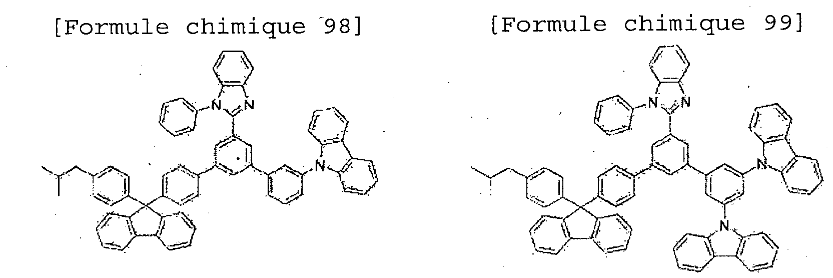

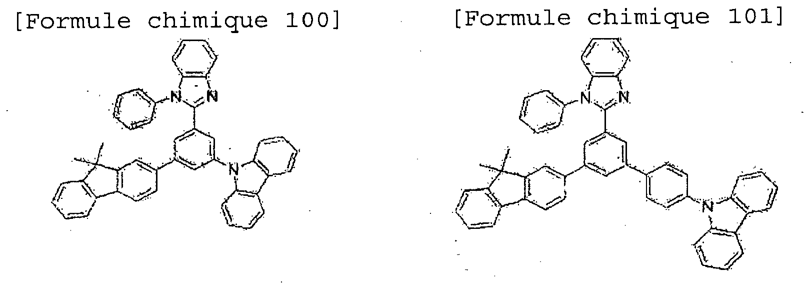

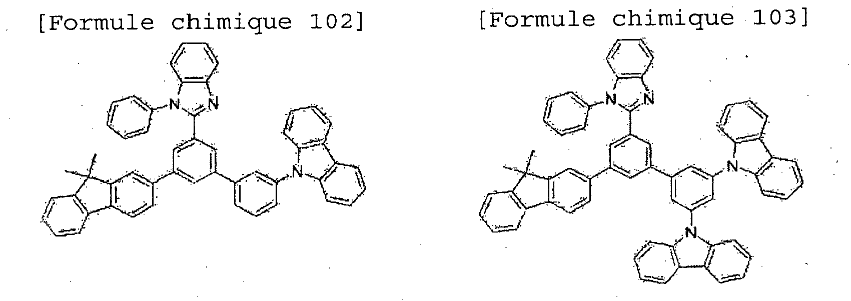

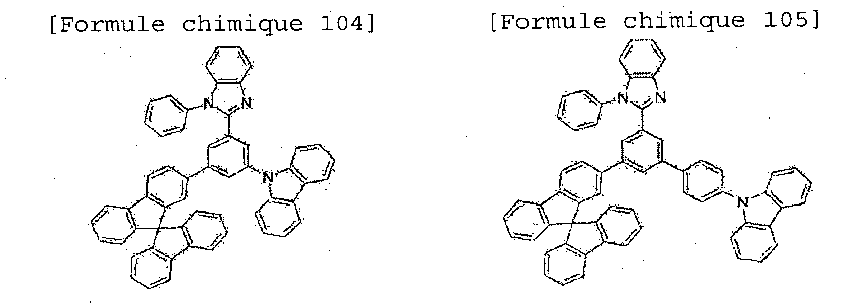

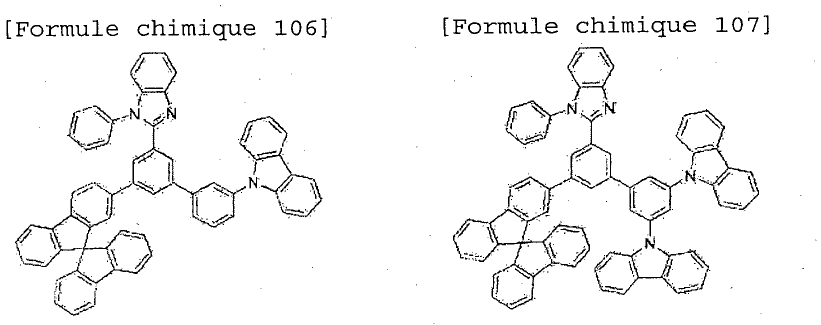

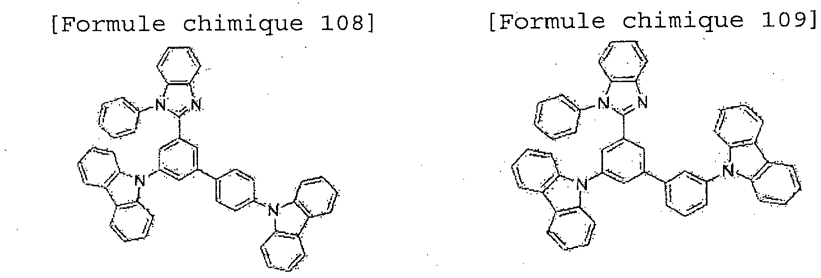

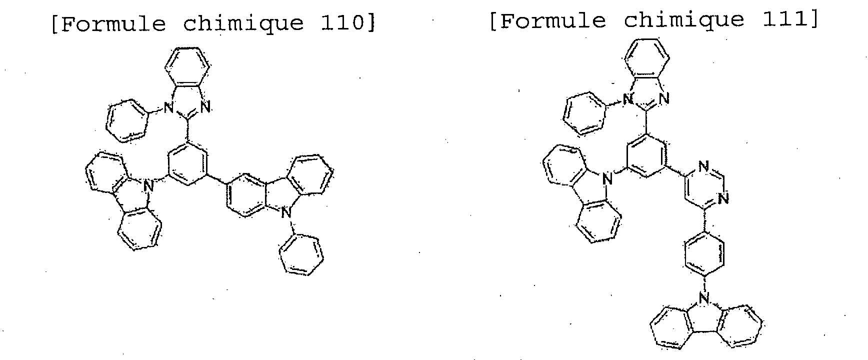

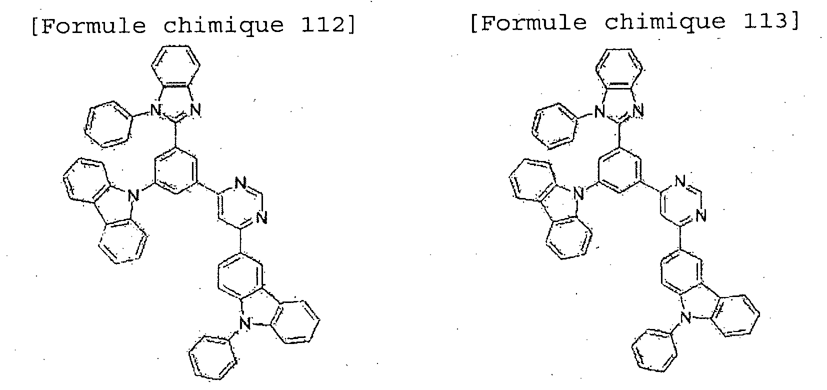

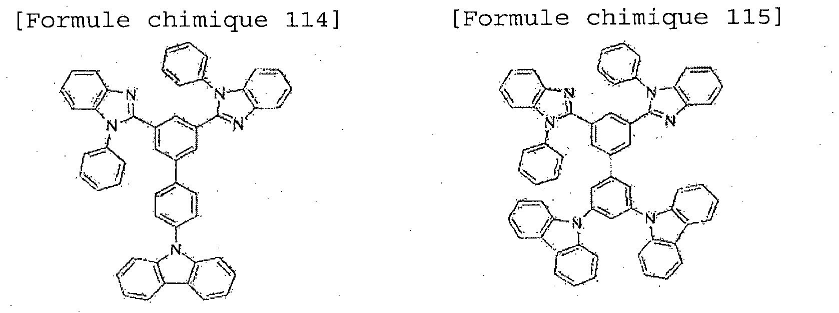

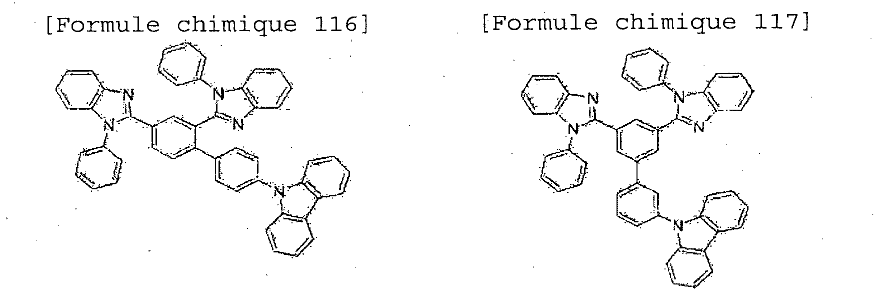

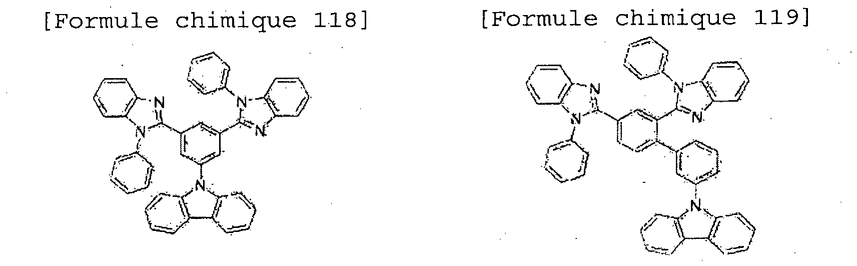

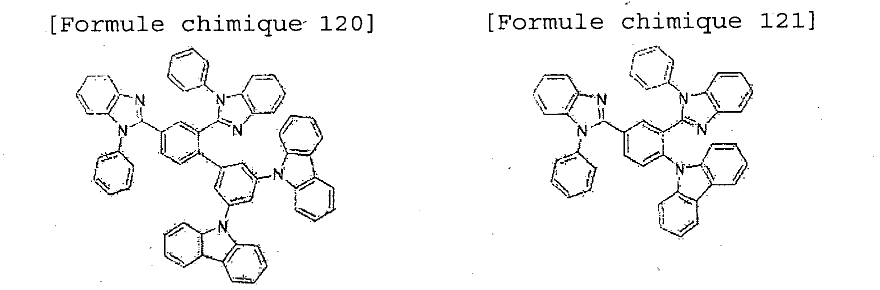

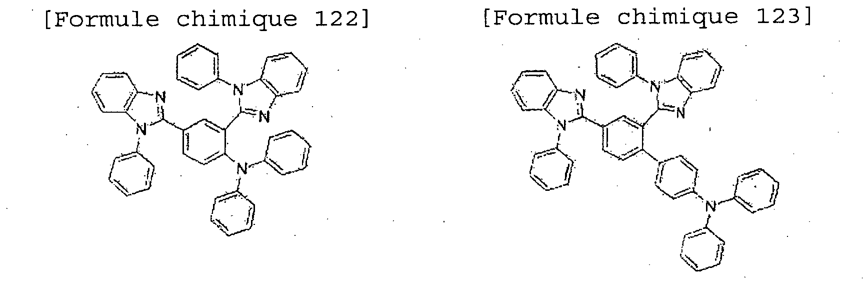

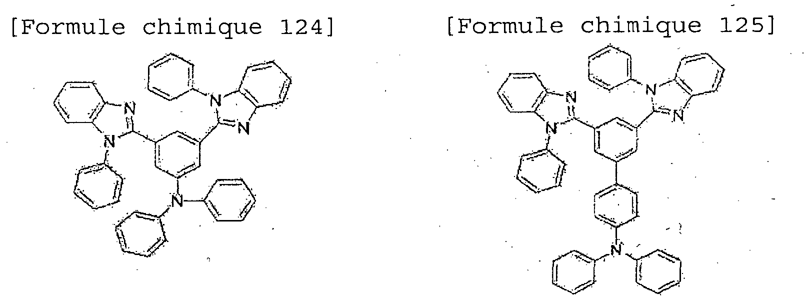

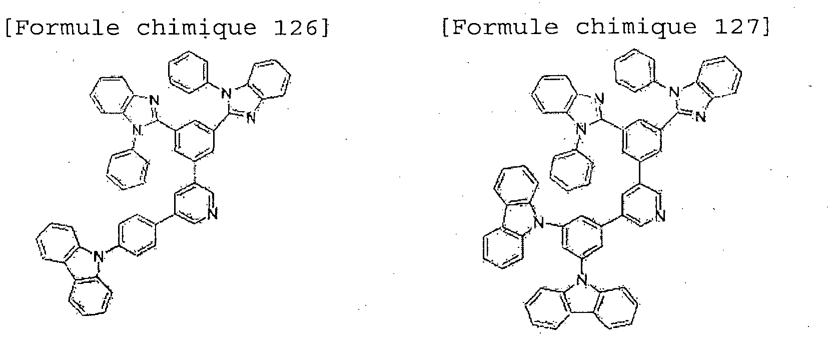

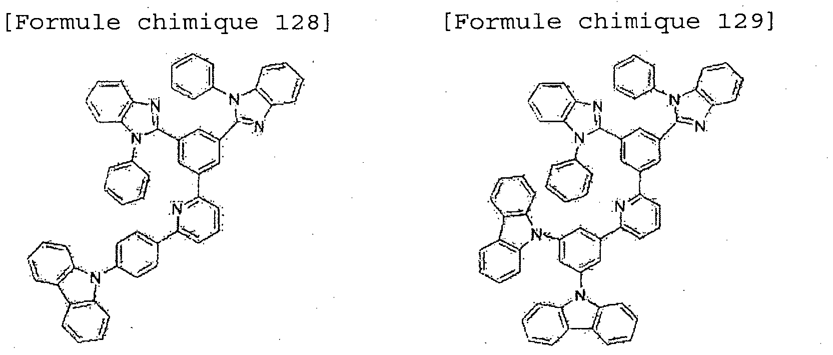

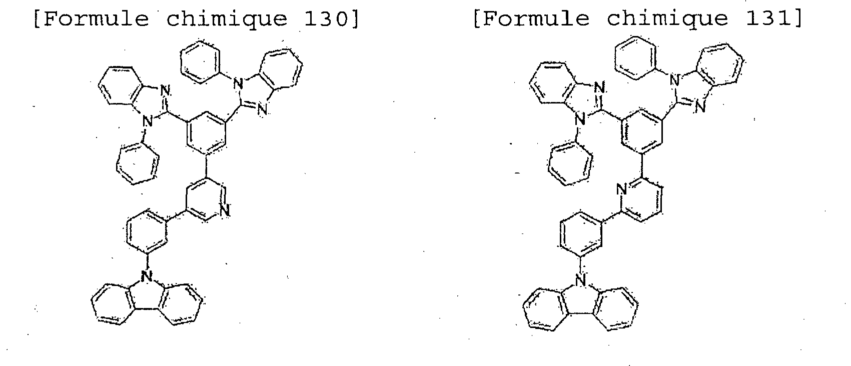

2. Utilisation du composé de benzimidazole selon la revendication 1 pour application à un matériau de transport de charges ou un matériau hôte pour un dispositif photoélectrique organique.

3. Dispositif photoélectrique organique comprenant une anode, une cathode, et au moins une couche mince organique disposée entre l'anode et la cathode, l'au moins une couche mince organique comprenant le composé de benzimidazole selon la revendication 1.

4. Dispositif photoélectrique organique selon la revendication 3, dans lequel la couche mince organique est une couche d'émission.

5. Dispositif photoélectrique organique selon la revendication 3, dans lequel la couche mince organique est choisie dans le groupe constitué par une couche d'injection d'électrons (EIL), une couche de transport d'électrons (ETL), une couche de blocage de trous, et leurs combinaisons.

6. Élément d'affichage comprenant le dispositif photoélectrique organique selon la revendication 3.
